(19) Europäisches Patentamt
European Patent Office
Office européen des brevets

(11) EP 0 920 522 B1

(12) EUROPEAN PATENT SPECIFICATION

(45) Date of publication and mention of the grant of the patent:
**29.10.2003 Bulletin 2003/44**

(21) Application number: **97937243.0**

(22) Date of filing: **14.08.1997**

(51) Int Cl.$^7$: **C12N 15/85**, A61K 48/00, C12N 5/10, C07K 16/32, C07K 16/30, C12Q 1/68, C12N 15/11

(86) International application number:
**PCT/US97/14306**

(87) International publication number:
**WO 98/006863 (19.02.1998 Gazette 1998/07)**

# (54) A VECTOR FOR POLYNUCLEOTIDE VACCINES

VEKTOR FÜR POLYNUKLEOTIDIMPFSTOFFE

VECTEUR DESTINE A DES VACCINS POLYNUCLEOTIDIQUES

(84) Designated Contracting States:
**AT BE CH DE DK ES FI FR GB GR IE IT LI LU MC NL PT SE**

(30) Priority: **14.08.1996 US 23931 P**

(43) Date of publication of application:
**09.06.1999 Bulletin 1999/23**

(73) Proprietor: **THE GOVERNMENT OF THE UNITED STATES OF AMERICA, as represented by THE SECRETARY, DEPARTMENT OF HEALTH AND HUMAN SERVICES**
**Rockville, MD 20852 (US)**

(72) Inventors:
- **NELSON, Edward, L.**
  **Eldersburg, MD 21784 (US)**
- **NELSON, Peter, J.**
  **D-80689 Munich (DE)**

(74) Representative: **Vossius, Volker, Dr. et al**
**Dr. Volker Vossius,**
**Patentanwaltskanzlei - Rechtsanwaltskanzlei,**
**Geibelstrasse 6**
**81679 München (DE)**

(56) References cited:
**WO-A-92/01055** **WO-A-92/01307**
**WO-A-95/07347** **WO-A-96/40987**

- **COLOMA, M. JOSEFINA ET AL: "Novel vectors for the expression of antibody molecules using variable regions generated by polymerase chain reaction" J. IMMUNOL. METHODS (1992), 152(1), 89-104 CODEN: JIMMBG;ISSN: 0022-1759, 1992, XP000289684**
- **PETER J. NELSON ET AL.: "Genomic organization and transcriptional regulation of the RANTES chemokine gene" JOURNAL OF IMMUNOLOGY, vol. 151, no. 5, 1 September 1993, BALTIMORE US, pages 2601-2612, XP002047102 cited in the application**

EP 0 920 522 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

### Field of the Invention

The present invention relates generally to polynucleotide vaccines and a novel vector useful for same. The present invention relates more specifically to a humanized polynucleotide vector vaccine which is useful in generating an immune response to a selected target antigen, in particular, to a tumor antigen.

### Background of the Invention

Cancer is a leading cause of morbidity and mortality. Conventional therapies such as surgery, radiation treatment and the like have resulted in a modest improvement of survival. There remains a need in the art for alternative treatments. With the advancing knowledge of immunology and tumor interaction with cells of the immune system, the upregulation of the immune system provides an alternative approach to treatment of cancers.

The process of oncogenesis involves multiple steps and predominantly involves intracellular protein products of either oncogenies or tumor suppressor genes (3-7). These intracellular proteins frequently have mutations or alterations leading to dysregulation or altered activity which can result in 'altered self' protein sequences and therefore, potential tumor-specific antigens (8,9). The immune response to any tumor requires recognition of such tumor associated antigens. These more subtle, 'altered self', antigenic differences are likely to be much less immunodominant than truly foreign antigens such as those from infectious disease pathogens or allograft tissues. The recent work defining antigen presentation has revealed that antibodies generally recognize antigens in the form of whole molecules, T cells typically recognize antigens in the form of small peptides combined with MHC molecules. This understanding of antigen presentation has aided the recent identification of a number of tumor-associated antigens which are recognized by T cells and derived from intracellular proteins (9). The characterization of oncogene mutations, dysregulated developmental or oncofetal genes, and mutations of tumor suppressor genes provides multiple potential unique target antigens for anti-tumor immune responses.

One form of cancer, breast cancer is the most common cancer affecting women in the United States of America, with 183,400 new cases annually (1). Breast cancer is also the second most frequent single case of female cancer mortality with 46,000 deaths annually (1). Although there has been significant progress over the past several decades in the surgical, radiotherapeutic, and medical treatment of this disease, none of these modalities alone or in combination provide curative therapy for the majority of patients with advanced (stage III or metastatic) breast cancer. Less than 40% of patients with advanced breast carcinoma will be alive and disease free, five years from diagnosis and initial treatment (2). Interestingly, patients with significant inflammatory infiltrates in the primary tumor, i.e. medullary carcinoma, have significantly improved survival (2) despite a higher degree of cellular anaplasia. The recent identification of tumor suppressor gene loss, oncogene mutations and oncogene dysregulation in breast cancers has opened new horizons for the treatment of the extraordinarily common cancer.

Numerous oncogene alterations have been documented in patients with breast adenocarcinoma including p53, HER2/neu (C-erbB-2), Rb, ras, PEM/MUC 1, BRCA1, BRCA2, Int-2, and hst. These particular oncogene alterations yield a panel of potential tumor associated targets for immunomodulatory therapies. Up to 80% of advanced breast adenocarcinomas will have p53 alterations (10-14) with most of the mutations occurring in the so called "hot spot", between codons 130 and 286, (15-20). The C-erbB-2 or HER-2/neu gene product shares homology with the epidermal growth factor receptor (21-25) and is expressed in up to 40% breast carcinomas (26). The tumor suppressor gene Rb has been noted to be altered or absent in 24% to 42% of breast cancer patients (27-29) and this percentage appears to be increased in metastatic lesions over primary tumors. The role of ras alterations appears to be more complex in breast cancer than for most other tumors (30-33). H-ras is the member of this family of proteins which is most frequently altered in breast cancers although, either K ras and/or H ras are overexpressed in excess of 70% of breast cancers. Polymorphic Epithelial Mucin (PEM) or MUC-1 is a tumor-associated antigen which is now known to represent an underglycosylated and dysregulated glycoprotein (34-36) and is diffusely overexpressed on 94% of breast cancers (37). BRCA1 has recently been cloned (38) and mutations have been characterized in patients with ovarian and breast cancer (39-43). Int-2 and Hst are oncogene sequences encoding proteins which are members of the fibroblast growth factor (FGF) supergene family (44). Amplification of Int-2 and Hst is significantly correlated with invasive breast carcinoma (45, 46) and estrogen receptor positivity (47). Breast carcinomas have other alterations including overexpression of non-mutated ubiquitous cellular proteins such at c-myc, Bcl-1, Bcl-2, EGFR, PRADI (cyclin Dl), IGF1R, bFGF, PDGF-B, TGF-b and others (48, 49). When the novel or mutated regulatory elements responsible for the overexpression of these individual genes are identified and characterized they could prove to be appropriate target tumor antigens. Clearly, there is a diverse and growing set of potential tumor antigens as a result of the multiple molecular events inherent in the development of breast adenocarcinoma.

Evidence has recently accumulated suggesting a potential for immunotherapeutic approaches in the treatment of breast cancer. Patients with breast cancer can have a cellular response to autologous tumor associated antigens (50). Breast cancer patients can also have antibodies to mutated p53 proteins (51). Elution of antigens from immune complexes present in sera of patients with breast carcinoma reveal multiple oncogenic antigens (52). T-lymphocytes reactive to mutated p53, HER2/neu, and ras peptides have been described (9, 53, 54-58). PEM/MUC-1 has been shown to be recognized by both antibodies and cytotoxic T lymphocyte (CTL) lines (34, 52, 59-61). Some CTL recognize the target antigen in a non-MHC restricted fashion (52, 58-61) however, in a murine model classical HLA restricted CTL recognizing the non-repeating core sequences have been elicited (62, 63). These findings, in addition to the association of improved survival with inflammatory infiltration of primary breast tumors, suggest that patients can mount an immune response to the malignant cells of breast tumors.

Oncogene and tumor suppressor genes can have mutations within confined domains, such as p53 and ras (15-20, 64, 65), while others have mutations which inactivate the protein product and/or can have total loss of the normal alleles, such as RB-1 and BRCA1 (5-7, 66-70). Obviously, allelic deletions provide no protein product but, mutated inactive gene products can be the source of peptides recognized as tumor associated antigens. Significantly, humoral and CTL immune responses to polypeptides encompassing the mutated sequences for both p53 and ras have been identified (50-52, 55, 71, 72, 73). These genetic alterations from germline can be highly specific and unique for any one individual tumor, even those with identical phenotypic features, and may yield unique individual tumor antigens (74, 53, 17, 51). Some oncogenies products such as HER2/neu (C-erbB-2), PEM/MUC-1, Int-2, Hst-1, and TRE17 (75-80) do not display polymorphism due to mutations but rather, are overexpressed in malignant tissues and essentially not expressed in normal adult tissues. These proteins and glycoproteins which are preferentially expressed in/on neoplastic mammary cells more closely fit the classical model of tumor antigens. When immune responses have been demonstrated to these preferentially expressed proteins and responses are frequently limited to regions of dissimilarity with the homologous or normal proteins expressed in adult tissues (9, 32, 56, 58, 60). Therefore, regions of dissimilarity, like mutated sequences, are potential antigenic targets for immunomodulation of the anti-tumor immune response.

The ability to precisely define and characterize tumor associated antigens has facilitated the design and evaluation of immunomodulatory therapies. Effective immunotherapies have been largely limited to non-Hodgkin's lymphoma and melanoma. In the case of non-Hodgkin's lymphomas, the idiotypic immunoglobulin molecule has been a target of several different strategies (81-84). The use of anti-idiotypic antibodies resulted in some clinical responses but, modulation of the antigen was also seen (85, 86). Use of the intact idiotypic immunoglobulin molecule as a polypeptide vaccine has elicited both cellular and humoral immune responses which correlate with improved clinical courses (87-89). This suggests that even within a single tumor-associated protein a broader immune response, potentially to multiple epitopes within the polypeptide, will result in improved clinical efficacy. Several groups have conducted trials using allogeneic cells or allogeneic cellular preparations in an attempt to address the issue of antigenic heterogeneity within tumors but, with notable exceptions these strategies have not contributed significantly to improved survival in any tumor type (9, 90, 91). Although the evaluation of clinical efficacy rests on improved survival, immunological parameters may be surrogate end points which facilitate the design and evaluation of new strategies. In the case of these "polyvalent" preparations, the ability to adequately evaluate immunologic parameters is severely hindered by the lack of defined antigens. To date, there has not been an immunomodulatory trial with multiple, defined, autologous, tumor associated antigens.

Intratumoral heterogeneity dictates that antigen specific immunomodulatory maneuvers be individualized or face severe limitations on the patient population for which they are applicable. Single antigen based immunomodulatory strategies do not account for intratumoral heterogeneity and thus, are at risk for tumor escape from immune surveillance even if effective augmentation of the anti-tumor immune response is accomplished. Based on our current understanding of the biology of neoplasms, including the multitude of cellular processes which must be deranged for transformation and tumorigenesis, each patient's tumor offers multiple potential targets for antigen-specific, immunomodulatory therapies. A more effective and efficacious strategy should take advantage of this multiplicity of potential, highly specific, tumor antigens. However, there is no current immunomodulatory strategy which allows for this individualized 'polyclonal' approach.

We now appreciate that, as with the immune response in allograft rejection or viral infections, an effective anti-tumor immune response must involve a cytolytic cellular response. Evidence is accumulating that the MHC class I pathway of antigen presentation and cytotoxic T lymphocytes (CTL) are important for effective in vivo anti-tumor immune responses (62, 89, 92-96). It has been shown that even in vaccine studies using exogenous protein antigen, modest increases in the cytotoxic T lymphocyte compartment have been demonstrated and correlated with freedom from progression (89). Furthermore, the preeminent role of the TH1 phenotype of T helper cells in both humans and animal models supports the critical role of multiple arms of the cellular immune system for an effective anti-tumor immune response (97-101) and provides further evidence for the critical role of CTL anti-tumor immune responses.

The recently described polynucleotide vaccine strategy has been demonstrated to yield humoral and cellular (both helper and cytotoxic T lymphocyte) immune responses to numerous encoded antigens (102, 103, 104-131). This is in contrast to the overwhelming humoral response to exogenous protein or cellular vaccine preparations. This strategy uses covalent closed circular (CCC) plasmid DNA, 'naked DNA', to express the target antigen (117). These plasmids are non-replicating but, are capable of extended stable expression of the target sequences in skeletal muscle and potentially in professional antigen presenting cells such as members of the macrophage lineage or dendritic cells (117). The plasmids are non-replicating but, capable of stable mRNA expression within skeletal muscle and reticuloendothelial cells. Thus, antigens encoded by these mRNA sequences are processed as endogenous proteins and presented to the immune system in a manner analogous to presentation of virus encoded protein antigens. It is now recognized that most potential tumor antigens are recognized as peptides derived from intracellular proteins, some of which are dysregulated or mutated oncogene products (103, 132a, 133a, 134a, 104-112, 118, 119). Polynucleotide vaccine strategy provides an avenue for eliciting immune responses to such intracellular oncogene or dysregulated products. Multiple studies have demonstrated the expression of target antigens following intramuscular injection and uptake of 'naked DNA' (132a, 133a, 134a, 103, 1-20). The uptake of DNA is increased if preceded by 24 hours with an intramuscular injection of a myo-toxic agent in the same vicinity (133a, 134a, 1, 7, 10, 15).

The existing vectors use an antibiotic resistance gene as the selection agent. These genes generate a foreign protein, raise the possibility of an anti-vector immune response, and have the theoretical risk of transferring antibiotic resistance to normal host flora. These vectors use the CMV promoter, b actin promoter, or other retroviral LTR elements to drive transcription of the target sequence. These promoter elements have ubiquitous activity but, are large in the case of CMV and b actin, and in the case of the LTR have the theoretical risk of integration and potential oncogenic event. All of the current vectors are designed with a single target antigen in place with little or no flexibility for changing target sequences. The functional size limitation of all plasmid vectors limits the size of insertable elements. By utilizing large promoter elements and the complete ColE1 or MB1 origins of replication the size of the vector is increased and limitations are placed on the size and number of insertable elements. Thus, none of the existing vectors are optimally designed for evaluation of an anti-tumor polynucleotide vaccine strategy.

The majority of the antigens evaluated to date are generally monomorphic and thus, can be readily incorporated into established plasmid vectors. However, putative tumor antigens, i.e., altered self proteins, in any given tumor are not heterogeneous and in some cases distinctly unique. For example, mutations in p53, although limited to a contiguous region of approximately 600bp, are very heterogeneous from tumor to tumor (15-20). Other intracellular tumor antigens will likely demonstrate similar heterogeneity in the range of mutations. Therefore, the present invention is a vector which accommodates both monomorphic and polymorphic target antigens via PCR technology. This vector is useful in generating patient-specific, target-specific multiple antigen, anti-target polynucleotide vaccines.

**Summary of the Invention**

An object of the invention is a humanized, polynucleotide vector.

An object of the invention is a kit comprising a humanized, polynucleotide vector alone, or in combination with at least one nucleic acid sequence encoding one or more target antigens or antigenic epitopes thereof.

Another object of the invention is a humanized polynucleotide vector vaccine useful in eliciting an immune response against one or more target antigens or antigenic epitope thereof.

Yet another object of the invention is a pharmaceutical composition comprising the humanized polynucleotide vector vaccine and a pharmaceutically acceptable carrier.

A further object of the invention is a kit comprising a humanized polynucleotide vector vaccine alone or in combination with an expression enhancing agent.

Another object of the invention is the use of a sequence acceptance site which accepts cDNA target products from rt-PCR cloning.

One aspect of the invention is a method of preparing a humanized polynucleotide vector.

Another aspect of the invention is a method of preparing a humanized polynucleotide vector vaccine.

Yet another aspect of the invention is an in vitro or ex vivo method for expressing at least one target antigen or antigenic epitope thereof in cells comprising introducing a humanized polynucleotide vector into cells under conditions for' expression of the target antigen or antigenic epitope thereof.

Another object of the invention is a host cell expressing at least one target antigen or antigenic epitope thereof provided by a polynucleotide vector.

A further aspect of the invention is the use of a polynucleotide vector vaccine for the preparation of a pharmaceutical composition for stimulating a specific immune response to at least one target antigen or antigenic epitope thereof in a mammal.

Yet another aspect of the invention are target antigen specific cytotoxic lymphocytes and target antigen specific helper T lymphocytes elicited by administration of a polynucleotide vector vaccine.

These and other objects, features and many of the attendant advantages of the invention will be better understood upon a reading of the detailed description of the invention and drawings.

**Brief Description of the Drawings**

Figure 1 is a genetic map of the polynucleotide vector, pITL.
Figure 2 depicts the insertion site in the polynucleotide vector, pITL, for cDNA encoding target antigen.
Figures 3A through 3C show the amino acid sequence homology between human epidermal growth factor receptor (EGFR) and human Her 2/neu. The line underscores the target sequence of human Her 2/neu. The bold letters indicate the transmembrane domain.
Figures 4A through 4C show the amino acid sequence of human Her 2/neu and Rat Her 2/neu. The lines overlie the target sequence for both human and rat Her 2/neu. The bold letters indicate the transmembrane domain.
Figure 5 shows the restriction enzyme sites of the polynucleotide vector pITL.

**Detailed Description Of The Invention**

The present invention is a "humanized" vector which has the necessary elements to express mRNA for a target antigen. The resultant translated polypeptides are available for processing into presentable antigens to the immune system. The vector accommodates monomorphic and polymorphic nucleic acid sequences of a target antigen or antigens. The vector of the present invention is useful for constructing polynucleotide vector vaccines or "naked DNA" vaccines containing a nucleic acid sequence encoding one or more target antigens.
The polynucleotide vaccines vector of the present invention has the following characteristics:

(1) is selectable for growth and production of a polynucleotide vaccine product,
(2) is capable of eukaryotic expression of one or more target antigens or antigenic epitopes thereof,
(3) is functional in selected tissue and selected cells of the inflammatory immune system,
(4) has minimal extraneous non-human DNA sequences to minimize potential toxicity, and
(5) is capable of accepting target nucleic acid sequences from a number of different transcripts,
(6) lacks an antibiotic resistance encoding nucleic acid sequence.

A polynucleotide vaccine of the invention offers multiple advantages over other vaccine strategies for immunomodulation. Polynucleotide vaccine strategies appear to elicit brisk CTL responses in all models in which this aspect of the immune response has been evaluated as seen in Table 1. The primary advantage of the polynucleotide vaccine of the invention is that the target antigen(s) is expressed as an intracellular polypeptide or peptide and, as such, is precessed as a self polypeptide or peptide and appropriately presented on antigen presenting cells.

TABLE 1

| ANTIGEN | MODEL SPECIES | IMMUNE RESPONSES | | | TH1/TH2 | FUNCTIONAL EFFECT |
|---|---|---|---|---|---|---|
| | | HUMORAL | PROLIFERATIVE | CTL | | |
| Rabies | Mouse | + | | + | THI | Protective |
| Plasmodium | Mouse | + | | + | | Protective |
| Influenza, Nucleoprotein | Mouse | + | | + | | Protective |
| | Mouse | | | + | | |
| Influenza, Hemagglutinin | Mouse | + | | | | Protective |
| | Mouse | + | | | | Protective |
| | Chicken | + | | | | Protective |
| | Chicken | + | | | | |
| Bovine Herpes Virus - 1 | Mouse | + | | + | | |
| | Bovine | + | | | | Decreased Shedding |
| HBV, Envelope | Mouse | + | | | | Protective & Ig Class Switch |
| HBV, Surface A | Rabbit | + | | | | |
| HCV, Core Protein | Mouse | + | + | + | | |
| HCV, Nucleocapsid | Mouse | + | | | | |
| HSV, Glycoprotein D2 | Guinea Pig | + | | | | Protective |
| HIV-1, GP160 | Mouse | + | + | | | Reduced Syncytia |
| | Mouse | + | | + | | |
| HIV-1, GP160 | Primate | + | | | | Reduced Syncytia |
| HIV-1, GP120 | Primate | + | | | | |

TABLE 1, CONT.

| | | | | | | |
|---|---|---|---|---|---|---|
| SIV | Primate | + | | + | | Protective |
| BBTA GAL | Primate | + | | | | Expression |
| Luciferase | Primate | + | | | | Expression |
| Apolipoprotein E | Rat | | | | | Expression |
| CEA | Mouse | + | + | | | |
| p53 | Mouse | + | | + | | Protective |
| Factor IX | Mouse | + | | + | | |
| h Growth Hormone | Mouse | | | | | Expression |
| Dystrophin | Mouse | + | | | | Expression |

Other added advantages of the polynucleotide vector as a vaccine is that preparation of plasmid DNA is much less labor and time intensive than cellular or protein vaccine preparations. Polynucleotide vaccine preparations are readily purified, sterilized, and DNA preparations are known to have a long shelf life when stored as a precipitate. Technical expertise in standard sterile tissue handling procedures, standard molecular techniques, and established PCR technology is all that is required for generation of the polynucleotide vaccine preparation. Administration is by standard medical procedures performed routinely. Safety concerns are minimal as no retroviral or oncogenic viral elements are used in the present invention. Finally, with the vector of the present invention, the only protein to be transcribed and translated is the target antigen thus, unlike the pox virus vectors or existing polynucleotide vaccine vectors there is no acquired immunity to the vector of the present invention.

The polynucleotide vector of the present invention has minimal exogenous, non-human DNA and has been maximally "humanized". A 'humanized' vector is one in which the majority of the functional elements are derived from or synthesized based on a human element or a mammalian homolog of the human element. The intent of humanization is to achieve sustained expression of a target antigen(s) with no or minimal risk of methylation and genetic downregulation which would prevent or inhibit translation of the target antigen(s). Therefore, the vector does not contain sequences which may increase inactivation by methylation or changes in tertiary structure. Thus, the polynucleotide vector selectively elicits immune responses to the target sequence(s) with little or no immune response to the other components of the polynucleotide vector.

The humanized polynucleotide vector comprises a human derived promoter or mammalian homolog thereof which is functional in a mammalian target tissue and mammalian target cells and a sequence acceptance site which accepts cDNA target products from rtPCR cloning.

There are minimal non-human components in the polynucleotide vector. These components of the polynucleotide vector which are non-human derived components are necessary for production of the vector. These non-human components are the origin of replication which allows replication and growth of the vector in bacterial or yeast host cells and a nucleic acid sequence which allows for selection of recombinant plasmids in the bacterial or yeast host cells.

In one embodiment, the plasmid comprises an origin for plasmid replication and growth in bacterial cells, a nucleic acid sequence which allows for selection of recombinant plasmids which is operably linked to a human-derived promoter which is functional in a mammalian target tissue and mammalian target cells, a cloning site for insertion of sequences encoding target antigen(s), a stuffer sequence, a human derived 3' splice sequence, and a human derived poly A sequence.

The origin of replication may be non-human and may be derived from several sources including bacteria and yeast and the like. Such origins of replication include, but are not limited to PMB1, pUB110, pBC16, pSAQS01, pX012, pE194, pC194, pS174pSA2100, pSE3, pAM330, pCG1, pCG4, pHM1519, pSa151, pLS103, pTA1060, pBs81/6, pSC101, R1, RK2, RSF1010, ARS1, R6K, Sa, R300B, Rep, RepA, pR01614, OriT, OriV, OriW, OriC, OriF, OriP, OriT, OriT, CdE1, pEW27, pE194, fd, F1, F, NR1, p15A, a colE1 origin, functional portions thereof, and the like which are available from plasmids deposited with the American Type Culture Collection (ATCC), Rockville, MD. In a preferred embodiment, the origin of replication is a colE1 origin or functional portion thereof. In one embodiment, the origin of replication for use in constructing the polynucleotide vector is a minimal colE1 origin isolated from the vector pBR327 (ATCC) (Oka, A. et al Molec. Gen. Genet. Vol. 172, 151-159, '1979) comprising the sequence:

GGCCGCGTTG CTGGCGTTTT TCCATAGGCT CCGCCCCCCT GACGAGCATC
ACAAAAATCG ACGCTCAAGT CAGAGGTGGC GAAACCCGAC AGGACTATAA
AGATACCAGG CGTTTCCCCC TGGAAGCTCC CTCGTGCGCT CTCCTGTTCC
GACCCTGCCG CTTACCGGAT ACCTCTCCGC CTTTCTCCCT TCGGGAAGCG
TGGCGCTTTC TCAATGCTCA CGCTGTAGGT ATCTCAGTTC GGTGTAGGTC
GTTCGCTCCA AGCTGGGCTG TGTGCACGAA CCCCCCGTTC AGCCCGACCG
CTGCGCCTTA TCCGGTAACT ATCGTCTTGA GTCCAACCCG GTAAGACACG
ACTTATCGCC ACTGGCAGCA GCCACTGGTA ACAGGATTAG CAGAGCGAGG
TATGTAGGCG GTGCTACAGA GTTCTTGAAG TGGTGGCCTA ACTACGGCTA
CAC (SEQ. ID. NO. 1), and analogs thereof.

The minimal colE1 origin is a minimal size yet functional and provides plasmid replication and growth within permissive strains of E. coli. The colE1 in antiparallel as it reads in the polynucleotide vector and comprises the sequence:

GTGTAGCCGT AGTTAGGCCA CCACTTCAAG AACTCTGTAG CACCGCCTAC

ATACCTCGCT CTGCTAATCC TGTTACCAGT GGCTGCTGCC AGTGGCGATA
AGTCGTGTCT TACCGGGTTG GACTCAAGAC GATAGTTACC GGATAAGGCG
CAGCGGTCGG GCTGAACGGG GGGTTCGTGC ACACAGCCCA GCTTGGAGCG
AACGACCTAC ACCGAACTGA GATACCTACA CCGTGAGCAT TGAGAAAGCG
CCACGCTTCC CGAAGGGAGA AAGGCGGACA GGTATCCGGT AAGCGGCAGG
GTCGGAACAG GAGAGCGCAC GAGGGAGCTT CCAGGGGGAA ACGCCTGGTA
TCTTTATAGT CCTGTCGGGT TTCGCCACCT CTGACTTGAG CGTCGATTTT
TGTGATGCTC GTCAGGGGGG CGGAGCCTAT GGAAAAACGC CAGCAACGCG
GCC (SEQ. ID. NO. 2), and analogs thereof.

In a preferred embodiment, the colE1 origin of replication in the polynucleotide vector comprises the sequence:

CTCGGGCCGCGTTGCTGGCGTTTTTCCATAGGCTCCGCCCCCCTGACGAG
CATCACAAAAATCGACGCTCAAGTCAGAGGTGGCGAAACCCGACAGGAC
TATAAAGATACCAGGCGTTTCCCCCTGGAAGCTCCCTCGTGCGCTCTCCT
GTTCCGACCCTGCCGCTTACCGGATACCTGTCCGCCTTTCTCCCTTCGGGA
AGCGTGGCGCTTTCTCAATGCTCACGCTGTAGGTATCTCAGTTCGGTGTA
GGTCGTTCGCTCCAAGCTGGGCTGTGTGCACGAACCCCCCGTTCAGCCCG
ACCGCTGCGCCTTATCCGGTAACTATCGTCTTGAGTCCAACCCGGTAAGA
CACGACTTATCGCCACTGGCAGCAGCCACTGGTAACAGGATTAGCAGAGC
GAGGTATGTAGGCGGTGCTACAGAGTTCTTGAAGTGGTGGCCTAACTACG
GCTACACTAGAAGGACAGTATTTGGTATCTGCGCTCTGCTGAAGCCAGTT
ACCTTCGGAAAAAGAGTTGGTAGCTCTTGATCCGGCAAACAAACCACCGC
TGGTAGCGGTGGTTTTTTTGTTTGCAAGCAGCAGATTACGCGCAGAAAAA
AAGGATCT (SEQ. ID. NO. 26)

This fragment of the colE1 replicon was isolated by digesting pBR327 with Bst YI and Ava I. After isolation of the fragment by agarose gel electrophoresis the ends were polished to blunt with klenow fragment (large fragment) of DNA polymerase I. Then this fragment was ligated into pBluescript SK at the Sma I site and orientation was determined such that the orientation of the replicon was anti-parallel to the origin of replication of the pBluescript. The SupF fragment was isolated and ligated as described herein, orientation was again determined such that the SupF was read in an anti-parallel direction to the RANTES promoter and the putative target sequence in the completed vector.

A sequence which will provide a mechanism for selection and growth of recombinant plasmids in bacteria or yeast is provided in the vector construct. However, the polynucleotide vectors does not contain foreign antibiotic resistance genes. The sequences may be non-human derived sequences. The polynucleotide vector uses sequences such as suppressor tRNA genes including but not limited to as SupE, SupP, SupD, SupU, SupF, SupZ, glyT, glyU, SerP, $psu_1^+$, $psu_2^+$-C34, $psu_3^+$am, $psu_2^+$OC (Eggertson, G. et al, Am. Soc. Microbiology, vol. 52:354-374, 1988), and synthetic supF complementation tRNA gene, derivatives thereof (Brown, El. et al. Methods in Enzymology Vol. 68: 109-51, 1979) and the like to provide a mechanism for selection and growth.

The synthetic supF complementation tRNA gene (143) provides the mechanism for selection and growth of recombinant plasmids in a manner analogous to that used in eukaryotic expression cloning using the 'Seed' vector (pCDM8 or derivatives). This selection is dependent upon the presence of the 60kb p3 helper plasmid which contains inactive tetracycline and ampicillin resistance genes due to amber stop codon mutations which are complimented by the supF tRNA. The p3 helper plasmid is derived from pLM-2 as described in Mindich, L. et al. J. Bacteriology vol. 126, pp. 177-182, 1976. The supF tRNA is not functional in eukaryotic cells.

In one embodiment, a Sup F gene sequence for use in the polynucleotide vector comprises:

GAATTCTTTC GGACTTTTGA AAGTGATGGT GGTGGCCGAA GGATTCGAAC
CTTCGAAGTC GATGACGGCA GATTTAGAGT CTGCTCCCTT TGGCCGCTCG
GGAACCCCAC CACGGGTAAT GCTTTTACTG GCCTGCTCCC TTATCGGGAA
GCGGGGCGCA TCATATCAAA TGACGCGCCG CTGTAAAGTG TTACGTTGAG
AAAGAATTC (SEQ. ID. NO. 3)

, and analogs thereof.

The synthetic supF complementation tRNA gene may be isolated from the vector, pVX, which is present in the bacterial strain 39083 available at the American Type Culture Collection. The Sup F sequence as it reads in the polynucleotide vector is antiparallel and comprises:

```
1  GAATTCTTTC TCAACGTAAC ACTTTACAGC GGCGCGTCAT TTGATATGAT
   GCGCCCCGCT TCCCGATAAG GGAGCAGGCC AGTAAAAGCA TTACCCGTGG
   TGGGGTTCCC GAGCGGCCAA AGGGAGCAGA CTCTAAATCT GCCGTCATCG
   ACTTCGAAGG TTCGAATCCT TCCCCCACCA CCATCACTTT CAAAAGTCCG
   AAAGAATTC   (SEQ. ID. NO. 4)
```

, and analogs thereof.

Promoters for use in the vector are human promoters or functional portions thereof, promoters derived from or synthesized based on a human promoter, and mammalian homologs of human promoters, or portions thereof. The promoters for use in the polynucleotide vector of the present invention do not encompass viral promoters or viral derived promoters. The human promoters are selected on the basis of the tissue and cells to be targeted and should provide optimal expression of the target antigen(s) or antigenic epitopes thereof in the selected mammalian target tissue and mammalian cells. Embodiments of promoters which may be used in constructing the polynucleotide vector of the present invention include but are not limited to the human derived RANTES promoter (Nelson, P.J. et al. J. Immunol. Vol. 151:2601-33, 1993; Ortiz; B.D. et al. Molecular Cell. Biol. pp 202-210, 1996 (Jan); Nelson, P.J. Immunol Vol. 157, No. 3, 1996 (August 1)), truncated RANTES promoters and derivatives thereof. Truncated RANTES promoters include but are not limited to a 249 base pair fragment, a 440 base pair fragment and a 900 base pair fragment. In a preferred embodiment, the truncated RANTES promoter is a base pair fragment of approximately 440 base pairs. A preferred truncated RANTES promoter is a fragment that spans the region approximately between the NCO restriction endonuclease site through the KpnI site of the RANTES promoter as described in GenBank Accession No. S64885. The human derived promoter is selected based on the mammalian target tissue and mammalian target cell in which expression of the target antigen(s) is desired. The target tissue is one containing antigen presenting cells or easily accessible to antigen presenting cells. Such tissue include but is not limited to muscle, lymph nodes, epithelium, subepithelium, skin, and the like. In one embodiment, the promoter is active in muscle or skin. Promoters may express one or more target antigen(s) or antigenic epitope thereof in muscles cells or professional antigen presenting cells. Preferred are promoters which express target antigen(s) in professional antigen presenting cells such as monocytes, macrophages, dendritic cells, Langerhans cells and the like.

The 3' splice sequence and the poly A sequences in the polynucleotide vector are a mammalian sequence, or a synthetic sequence based on a mammalian sequence, preferably a human sequence or mammalian homolog thereof. Possible sources of these elements include but are not limited to bovine growth hormone, human growth hormone gene, and the like. In a preferred embodiment of the polynucleotide vectors the 3' splice (intron) and the poly A sequences are synthetic sequences based on sequences from the human growth hormone gene (DeNoto, F.M. et al. Nuc. Acids. Res. Vol. 9(15):371930, 1981). Oligonucleotides may be synthesized based on the published sequence or modified to condense the third intron and poly A tail sequences along with the appropriate splice functions. Poly A-signal sequences which may be used in constructing the polynucleotide vector include but are not limited to:

AATAAA (SEQ. ID. NO. 5)

ATTAAA (SEQ. ID. NO. 6)

AGTAAA (SEQ. ID. NO. 7)

AAGAAC (SEQ. ID. NO. 8)

AATACA (SEQ. ID. NO. 9)

and the like.

In one embodiment, a combined 3' splice sequence and poly A tail sequence for incorporation into the polynucleotide vector construct includes but is not limited to:

```
5' GCCTTAAGGG CCATATGGTG AGTGGATCCC TTGACCCCAG GCGGGGATGG
3' GGAATTCCC GGTATACCAC TCACCTACGG AACTGGGGTC CGCCCCTACC

   GGAGACCTG TAGTCAGAGC CCCCGGGCAG CACAGGCCAA TGCCCGTCCT
   CCCTCTGGAC ATCAGTCTCG GGGGCCCGTC GTGTCCGGTT ACGGGCAGGA

   TCCCCTGCAG GATGAGTAGT GAGTGCCTCT CCTGGCCCTG GAAGTTGCCA
   AGGGGACGTC CTACTCATCA CTCACGGAGA GGACCGGGAC CTTCAACGGT

   CTCCAGTGCC CACCAGCCTT GTCCTAATAA AATTAAGTTG CATCATTTTG
   GAGGTCACGG GTGGTCGGAA CAGGATTATT TTAATTCAAC GTAGTAAAAC

   TCTGACTAGG TGTCCTCTAT AATATTAT 3' (SEQ. ID. NO. 10)

   AGACTGATCC ACAGGAGATA TTATAATA 5' (SEQ. ID. NO. 11)
```

Operably located downstream of the promoter and upstream of the splice and poly A sequences, is the cloning site and stuffer site. The sequence acceptance site is functional in mammalian cells, preferably human cells. The sequence acceptance site is synthetically constructed, however, the sequences are derived from sequences that function in human cells. The sequence acceptance site is designed to directionally accept sequence-specific products from rtPCR-based cloning strategies via unique sequences within the interrupted palindrome recognition sequence for the Bgl I restriction endonuclease, which is incorporated into the PCR primers. The 5' cloning site of the vector was designed in such a way as to provide an integral Kozak consensus sequence (145) and an in-frame initiation codon. Oligonucleotides may be synthesized for the sequence acceptance site so as to provide an initiation codon, Kozak consensus sequence and downstream termination codon. Kozak consensus sequence which may be incorporated into the polynucleotide vector includes but is not limited to:

GCCCGCC (either A or G) CCAUGG and the like, as are known in the art (Kozak, M. J. Cell Biol. vol. 108, pp 229-241, 1989).

The polynucleotide vector may be constructed to allow for insertion of a internal ribosomal entry site (IRES). Such a site allows for translation of a second open reading frame from a polycistronic mRNA molecule. Such IRES elements are described by Sachs et al., Cell June 13, 1997 Vol. 89: 831-838; Chen, C-Y et al. Science Vol. 268, pp. 415-417, 1995; Tahara, H. et al. J Immunol pp. 6467-6474, 1995; Joshi, C. et al. Nucleic Acids Research Vol. 23, No. 4, pp. 541-549, 1995; Ting, J. et al. DNA Vol. 7, No. 4, pp 275-286, 1988; and Aharon, T. et al. Molecular Cellular Biology, Mar 1993, pp 1971-1980. A polynucleotide vector comprising an IRES allows for translation of more than one target antigen or epitope from the polycistronic mRNA. A polynucleotide vector comprising an IRES allows expression of a target antigen and a cytokine or chemokine from a polycistronic mRNA.

A stuffer site is provided in the vector as a space occupying, non-coding fragment of DNA. A stuffer site may be synthetically synthesized. In one embodiment the stuffer site has the nucleic acid sequence:

```
CCTCGGTACCTGCCATGGCGCGGATTCTTTATCACTGATAAGTTGGTGGA
CATATTATGTTTATCAGTGATAAAGTGTCAAGCATGACAAAGTTGCAGCC
GAATACAGTGATCCGTGCCGGCCCTGGACTGTTGAACGAGGTCGGCGTAG
ACGGTCTGACGACACGCAAACTGGCGGAACGGTTGGGGGTGCAGCAGCC
GGCGCTTTACTGGCACTTCAGGAACAAGCGGGCGCCTTAAGGGCCATATG
CCG (SEQ. ID. NO. 12)
```

, and variants thereof.

This stuffer region sequence is based on the stuffer region in vector pCDM8 (In Vitrogen). The sequence of the stuffer is not critical as long as it is one which does not interfere with cloning of the rt-PCR target sequence and does not contain a BglI site. The stuffer site is not present in the final polynucleotide vector vaccine, as it is excised.

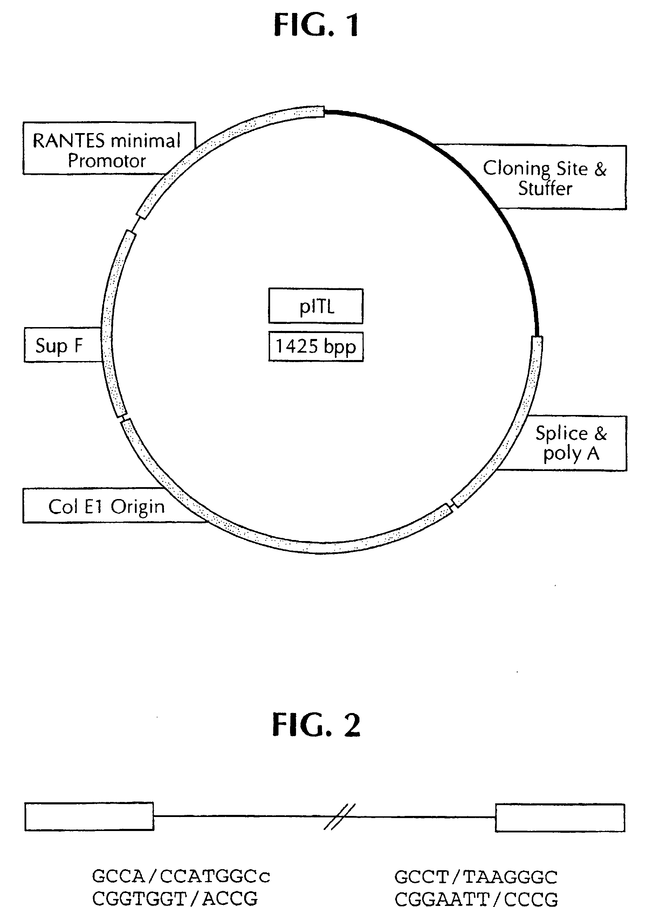

In one embodiment of the present invention the polynucleotide vector comprises pITL as schematically depicted in Figure 1. cDNA encoding one or more target antigen(s) or antigenic epitopes thereof may be ligated into the cloning site or sequence acceptance site depicted in Figure 1. The sequence acceptance site is designed to directionally accept sequence specific products from rtPCR based cloning strategies via unique sites within an interrupted palindrome recognition sequence for a restriction endonuclease which is incorporated into the PCR primer. In one embodiment, the pallidrome recognition sequence is for the Bgl I restriction endonuclease. In a preferred embodiment the sequence acceptance site is as depicted in Figure 2 in which the 5' acceptance site reads on the (+) strand as the sequence GCCA/CCATGGCc wherein the GCC sequence is recognized by Bgl I and ATG is the start codon. GCc keeps the sequence in-frame and encodes the amino acid serine. The 3' acceptance site reads on the (+) strand as GCCTTAAGGGC.

Any exogenous gene may be inserted into the polynucleotide vector and expressed in a host cell or host tissue. Of interest are exogenous genes which are associated with diseases or pathological conditions in humans. Of particular interest are the antigens or antigenic epitopes thereof which are useful in stimulating an immune response in a mammal, preferably a human for the purpose of prevention or treatment of a disease or pathological condition.

The polynucleotide vaccine vector is useful as a single target antigen vaccine and as a multi-target antigen vaccine. Of interest are target antigens derived from intracellular organisms and the host intracellular compartment. The target antigen includes but is not limited to tumor antigen, bacterial, viral, parasitic antigen and the like. Such antigens include but are not limited to antigens or antigenic epitopes derived from rabies virus, plasmodium, Herpes Virus, HIV 1, HIV 2, influenza virus, HBV, HCV, SIV, Cytomegalovirus, Mycobacterium, Measles virus, papillomavirus, and the like. The polynucleotide vector is particularly well suited for expression of tumor associated genetic derangements, which encompass aberrant transcription regulatory controls on oncogene mutation, a dysregulated developmental or oncofetal gene, a mutated tumor suppressor gene, dysregulated cellular enzymes coding sequences such as metaloproteases or combinations thereof. Tumor antigen or antigenic epitopes thereof which may be expressed by the polynucleotide vector include but are not limited to p53, RB, ras, int-2, Hst, Trel7, BRCA-1, BRCA-2, MUC-1, HER-2/neu, PEM/MUC-1, and the like. The polynucleotide vector is also well suited for insertion of one or more target antigens or antigenic epitopes thereof derived for an individual's tumor. The nucleic acid sequences encoding one or more target antigens is ligated into the cloning site of the polynucleotide vector thereby forming the polynucleotide vector vaccine.

The nucleic acid sequence encoding a cytokine and/or chemokine may also be incorporated into the polynucleotide vector vaccine for enhancement of an immune response. Such cytokines include but are not limited to interleukin 2, interleukin 4, interleukin 7, granulocyte-monocyte colony stimulating, factor (GM-CSF), tumor necrosis factor (TNF), interferon, interleukin 12, interleukin 3, interleukin 15, interleukin 8, interleukin 18 and the like. Nucleic acid sequences encoding chemokines which may incorporate into the cloning site of the polynucleotide vector vaccine include those that recruit lymphocytes and antigen presenting cells into a target tissue. These include but are not limited to RANTES, MCP, MIP-$\alpha$, MIP-1$\beta$, defensins and the like.

In one embodiment, the polynucleotide vector vaccine comprises a gene encoding a target antigen or epitope thereof and a gene encoding GM-CSF or functional portion thereof. The polynucleotide vector allows expression of both the target antigen or epitope thereof and GM-CSF in the target cell or target tissue resulting in an enhanced immune response to the target antigen or epitope thereof.

The present invention encompasses methods of making a humanized polynucleotide vector which comprises operably linking an origin of replication with a nucleotide sequence which provides a mechanism for selection, which in turn is operably linked to a human derived promoter or mammalian homolog thereof which is functional in a mammalian target tissue and mammalian target cells. The promoter is operably linked with a cloning site containing a sequence acceptance site which directionally accepts target sequence specific products from rtPCR cloning. The 5' sequence acceptance site comprises an initiation codon and Kozak consensus sequence. The 3' sequence acceptance site comprises termination codons (Figure 2). The method also provides a stuffer region, two separate in1 frame termination codons, a human derived 3' splice (intron) or mammalian homolog thereof and a human derived poly A sequence.

The present invention also provides a method of preparing a polynucleotide vector vaccine in which one or more nucleic acid sequences encoding one or more target antigens or antigenic epitopes thereof are incorporated. The method of making the substantially humanized polynucleotide vector vaccine provides a product able to express stable mRNA for one or more target sequences.

For vaccination against a tumor in a patient, the construction of the polynucleotide vector vaccine is tailored to the individual's tumor antigen or antigens. Most potential tumor antigens are recognized as peptides derived from intracellular proteins, some of which are dysregulated or mutated oncogene products (74, 8, 9, 30, 53, 71, 132b, 133b, 134b, 135b, 136, 138) as opposed to unique, intact cell surface molecules. Therapies such as monoclonal antibodies or peptide vaccine strategies are generally directed against a single tumor associated antigen and as such are confronted by the fundamental issue of intratumoral antigenic heterogeneity and are limited in their scope of applicability due to both intertumoral antigenic heterogeneity and the nature of most tumor-associated antigens. The multiple and diverse set of molecular events involved in neoplastic transformation, in the evolution to a malignant/metastatic phenotype, and in resistance to therapy provides a foundation for devising novel therapeutic strategies as each molecular event has the potential to generate a tumor specific antigen (139-141). Current molecular techniques and the recently described polynucleotide vaccine strategy (102, 132a, 133a, 134a, 135a, 103) allow a novel antigen-specific, anti-tumor, immunomodulatory therapeutic strategy. Polynucleotide vaccine strategies have been shown in animal models to elicit a broad antigen-specific immune response including humoral, proliferative and cytolytic T cell responses. The present invention addresses the issues of tumor heterogeneity and takes advantage of this phenomena to generate a highly specific yet broad anti-tumor immune response directed against multiple defined tumor-specific antigens.

Using RT-PCR technology, it is possible to isolate target cDNA sequences encoding antigenic polypeptides from individual tumors and to use this nucleic acid template in the polynucleotide vector to induce an immune response to the uniquely mutated or dysregulated intracellular tumor associated proteins in individuals immunized with the vaccine.

mRNA isolated from a patient's tumor is subjected to rtPCR by standard techniques. PCR primers are used to amplify, clone into the vector of the present invention and subsequently evaluate, by thermocycle sequencing, the resultant cDNA for mutations. If mutated sequences are identified and present in at least about 20% of clones analyzed, the sequences are used as a component of the polynucleotide vaccine of the present invention. For the oncofetal proteins, such as int-2 and Hst, demonstration of the presence of mRNA is sufficient for constructs with the oncofetal sequences to form part of the polynucleotide vaccine. Tumors which express MUC-1 or HER-2/neu antigens by immunohistochemistry have monomorphic constructs for these two tumor antigens which are ligated into the polynucleotide vector. Thus, each individual patient may receive a unique set of plasmid constructs depending upon the repertoire of mutated oncogenies, mutated tumor suppressor genes, expressed oncofetal genes, and disregulated monomorphic antigens. Examples of such include but are not limited to nucleic acid sequences for c-erb- β-2/HER2/neu, PEM/MUC-1, Int-2, Hst, BRCA-1, BRCA-2, truncated EGFRvIII, MUC-1,CEA, p53, ras, RK, Myc, Myb, OB-1, OB-2, BCR/ABL, GIP, GSP, RET, ROS, FIS, SRC, TRC, WT], DCC, NF1, FAP, MEN-1, ERB-B1 (Cell vol. 64:pp235-326, Jan. 25, 1991) and the like, which are inserted into the polynucleotide vector.

In one embodiment, the polynucleotide vector vaccine of the present invention include but are not limited to: pITL-HER2/neu, pITL-PEM/MUC-1, pITL-Int-2, pITL-Hst, pITL-BRCA-1, pITL-BRCA-2, pITL-p53, pITL-ras, pITL-RB, pITL-TRE17 and the like.

The polynucleotide vector vaccine of the invention may be formulated into a pharmaceutical composition comprising the vector vaccine and a pharmaceutically acceptable carrier such as physiological saline and the like, as known in the formulation art. The pharmaceutical composition may comprise one or more different polynucleotide vectors depending on the needs of the patient to be treated. The pharmaceutical composition may also comprise an agent which enhances the uptake and expression of the polynucleotide vaccine into the target tissue and target cells. These agents include but are not limited to mycotoxic agents. Such mycotoxic agents include but are not limited to dextrose, bupivacaine HCl (1-butyl-N-[2,6-dimethylphenyl]-2-piperidine carboxamide) (Sigma, St. Louis, MO), and the like.

The present invention also encompasses the use of the polynucleotide vector vaccine for the preparation of a pharmaceutical composition for stimulating an immune response, cellular and humoral, against a target antigen or antigenic epitope with little or no toxicity or autoimmune reactions. In the case where the target antigen is a tumor antigen or antigens, the immune response thus generated should result in generation of cytotoxic T lymphocytes which are capable of inhibiting or killing tumor cells or premalignant cells expressing the tumor antigen(s).

In said use at least one polynucleotide vector vaccine comprising a nucleotide sequence of the target antigen or multiple antigens is administered to a patient in a dose of at least about 1 μg per component, preferably at doses of about 1 μg to about 1 mg per DNA per construct, although higher or lower doses may be used. The dose is administered intramuscularly (IM) or by cutaneous ballistic impregnation in a volume of about 0.1 ml to a volume of about 1 ml. Preferably the volume is about 0.5 ml per injection. In one embodiment, the dose of vaccine is administered at three week intervals for a total of three doses.

The intramuscular injection of the polynucleotide vaccine may be preceded by IM injection of an expression enhancing agent approximately 24 hours before vaccine administration. The enhancing agent may be bupivacaine-HCl, dextrose, and the like. In the case of dextrose, a concentration of about 10 to about 50% is administered, preferably a concentration of about 50%. Preferably the enhancing agent is bupivacaine-HCl and functional equivalents. Bupivacaine-HCl is administered in a concentration of about 0.25% to about 1.0%, preferably a concentration of about 0.5% in a volume of 0.5 ml.

The pharmaceutical composition with at least one polynucleotide vector vaccine comprising a nucleotide sequence of the target antigen or multiple antigens may be administered using gene gun techniques. Then, the polynucleotide vector vaccine is adsorbed on to particles and administered by bombardment of the particles into the target tissue such as skin. (Yan, N.S., et al. 1990, Proc. Natl. Acad. Sci. USA 87; 9568; Williams, R.S. et al. 1991, Proc. Natl. Acad. Sci. USA 88:2726; Fynan, E.R.G. et al. Proc. Natl. Acad. Sci. USA 90:11478; Eisenbraun, M.D. et al. DNA and Cell Bio. 1993 12:791).

Patients may also receive conventional cancer therapy prior to the polynucleotide vaccine use. Treatments that induce or result in immunodepression are discontinued a minimum of 4 weeks prior to immunization with the polynucleotide vaccine. In the case of viral, bacterial, yeast, parasitic infections, appropriate antiviral, antibiotics, antifungals and the like may be administered prior to, concurrent with, or after administration of the polynucleotide vaccine.

Patients are monitored at periodic intervals to access the efficacy of the treatment. Tumor burden of the individual is monitored. The immune response to each component of each indivudal's vaccines preparation is evaluated. This is accomplished by evaluating the proliferative and cytolytic response to autologous cells transfected with single individual components (plasmid constructs). Tumor specific CTLp frequency is assessed for each patient. Anti-tumor humoral responses are assayed by staining of autologous tumor cells and analysis by fluorescent cell scanning (FACS) or the whole cell ELISA technique. Immune responses induced by polynucleotide vector vaccines containing nucleic acid sequences encoding virus, bacterial, yeast, parasite target antigens may be measured using standard immunoassays as are known in the art.

Polynucleotide vaccine therapy is applicable to any cancer in which a genetic defect is identified. Such cancers include but are not limited to breast cancer, prostate cancer, lung cancer, liver cancer, melanoma, colorectal cancer, pancreatic cancer, thymoma, sarcoma, non-Hodgkins lymphoma, Hodgkins lymphoma, leukemia, ovarian cancer and the like. Of particular interest is breast adenocarcinoma.

**EXAMPLE 1**

**Construction Of The Vector**

A plasmid, pITL was designes to contain minimal exogenous DNA and to be maximally "humanized", with elements of the plasmid except the origin of replication and selection element (supF) being derived from human genetic elements (Figure 1).

Standard molecular techniques were used for the following manipulations. From piVX a 207 base sequence (SupF) is excised using ECO RI and gel purified. This is ligated into the ECO RI site of the cloning vector pBluescript II KS (Stratagene, La Jolla, CA). The minimal colEl origin of replication (142) is utilized for plasmid replication and growth within permissive strains of *E. coli.* It was excised from pBR327 (ATCC #37516) using Sau 96 and Bfa I enzymes as a double digest and the 453 base pair fragment gel purified. This fragment is 'polished', that is the overhangs form these digests are filled in with DNA polymerase I (Klenow) large fragment. This blunt end, 'polished' fragment is ligated into the Sma I site of pBluescript II KS in which the SupF gene has previously been ligated into the ECORI site. The resultant product of these 2 ligations consists of pBluescript KS with the SupF gene and a second Coil origin of replication, both located within the multiple cloning site.

The splice poly A sequence was synthesized and inserted into the vector pGEM 7f+ between the Hind II and Xho I sites.

The unique directional cloning site or sequence acceptance site is upstream of two separate in frame termination codons, a 3-prime splice (intron), and poly-A sequences (the latter two synthetic sequences based on the human growth hormone gene) (144). These synthetic sequences were directionally inserted into pGEM-7F at the Xho and HindIII sites (Genosys, The Woodlands, TX). This vector is digested with Bam HI and HIND III. Likewise, the product from the minimal colE1, SupF, pBluescriptII KS is digested with these same enzymes. These two resultant purified, double digests are ligated together. The product of this ligation contains pGEM7F+ with the synthetic splice poly A sequence, minimal col E1 origin of replication and SupF gene all with the multiple cloning site.

The stuffer region is isolated from pCDM8 by using the following PCR primers to amplify an approximately 200 base pair portion of the stuffer (the exact PCR product will be 253 bases but, incorporates sequence modifications as detailed below). The upper primer (positive strand or 5') is CCTCGGTACCTGCCACCATGGCGCGGATTCTTTAT (SEQ. ID. NO. 13) and spans the 2217 to 2252 bases of pCDM8 but, diverges at 2235 to accommodate linking restriction sites in this case the 5' Bgl I cloning site (Figure 2) and a Kpn I site at the upstream, 5' end. This is important later for promoter ligation. The lower primer (negative strand or 3 ' ) is CGGCATATGGCCTTAAGGCGCCCGCTTGTTCCTGAAGT (SEQ. ID. NO. 14) and spans the 2394 to 2433 bases of pCDM8 but, diverges at 2454 to accommodate linking restriction sites in this case the 3' Bgl I cloning site (see Figure 2) and a Nde I site at the upstream, 5' end. This allows isolation of a fragment without a Bgl I site or any other conflicting sites. This fragment is digested with Nde I and gel purified. The pGEM product from above is digested with Nde I (site engineered into synthetic sequence) and Stu I which results in a blunt end on the 5' end. The fragment isolated above is ligated into this digested construct, i.e., Nde I end to Nde I end and blunt to blunt.

The following is the splice poly A signal sequence cloned into the Xho and Hind III sites of pGEM-7Zf reading from Xho site to Hind III site. Bold is intron 3, underline is poly A signal sequence, double underline is cloning site and potential site for internal ribosomal entry sites (IRES), with the remainder from the fifth exon but, following the stop codon:

gCCTTAAgggCCATATggTgAgTggATgCCTTgACCCCAggCggggA
TgggggAgACCTgTAgTCAgAgCCCCCgggCAgCACAggCCAATg
CCCgTCCTTCCCCCTgCAggATgAgTAgTgAgTgCCTCTCCTggCCC
TggAAgTTgCCACTCCAgTgCCCACCAgCCTTgTCCTAATAAAA
TTAAgTTgCATCATTTTgTCTgACTAggTgTCCTCTATAATATTA
T (SEQ. ID. NO. 15)

All triplets in italics are stop codons. After processing of punitive mRNA, excision of intron 3, the sequence will contain duplicate stop codons in two reading frames. The 3rd reading frame can contain a stop codon by mutagenizing the double underlined sequence resulting in deletion of last "g" of the double underlined sequence.

The RANTES promoter construct has a Kpn I site at its 3' end and depending upon the size of the truncation, various restriction sites at the 5' end. From pGL-RANTES the 249 base fragment is excised using Kpn I and Sac I enzymes. The product of the above paragraph is likewise digested with Kpn I and Sac I. The two digests, 249 bases and 1176 bases respectively, are gel purified an ligated together resulting in the intact vector pITL-1.

## EXAMPLE 2

### Construction Of Minimal Promoter Elements For Expression In Skeletal Muscle, Monocytes And Dendritic Cells

The choice of promoter sequence included in the vectors is critical as the promoter must be functional in the mammalian target tissues preferably human tissues and drive the expression of the mRNA at an appropriate level. The human RANTES promoter is one promoter useful in the present invention because it is smaller than the CMV or b-actin promoters, is functionally between the two in terms of activity, and is functional in the desired tissues (146). This promoter is highly conserved between human, rat and mouse (146, 147). This promoter has been well characterized and contains myoD like elements functional in skeletal muscle cells along with elements known to be active in cells of the reticuloendothelial system. The minimal promoter elements necessary for expression in lymphocytes has been determined (146, 147). Constructs with a series of 5' truncations of the RANTES promoter upstream of the luciferase reporter are constructed in order to evaluate the minimal promoter elements required for transcriptional activity in cells of interest. Such truncated RANTES promoters include a 249,440 and 990 base pair fragments.

Primary cultures of skeletal muscle cells were purchased (Clonetics, San Diego, CA) and cultured according to manufacturers directions. These cells can be transiently transfected with the control plasmid pGreen Lantern (Gibco, BRL, Gaithersburg, MD) using the cationic lipid mixtures Lipofectin and Lipofectamine (Gibco, BRL, Gaithersburg, MD) with only slight modifications from the manufacturer's suggested protocol. These modifications include trypsinizing cells and replanting 20 minutes before transfection and exposing the cells to the lower concentrations of cationic lipids. The previously described pGL-RANTES promoter constructs, pGL-basic and pGL-control vectors are transfected similarly and luciferase activity is assayed after 72 hours using the Luciferase Assay System (Promega, Madison, WI). To control for transfection efficiency pCMV-βGal (Clontech, San Francisco, CA) is cotransfected with the above plasmids and assayed using Galactolight Plus (Tropix Inc., Bedford, MA). Luminescence is determined for both assays on an existing LKB scintillation counter.

Monocytes are isolated by elutriation from normal donor leukapharesis packs. The elutriation protocols provides an 90-95% pure monocyte population as evidenced by FACS analysis for CD1-a, CD3, CD14, CD16, CD19, CD45RO, CD56, CD80, HLA-DR and HLA-DQ. The remaining 5% of cells are granulocytes/basophils. FACS analysis of MHC class II and CD25 expression reveals that these cells are not activated after the isolation procedure. Routinely $5 \times 10^8$ monocytes are isolated from a given leukopharesis pack. The same series of constructs and assay conditions are evaluated using these cellular preparation. The transfection procedure are modified for suspended cells, but utilize cationic lipid mixtures are above.

The recently reported ability to isolate dendritic cells (DCs), defined by phenotype and functional assays, from peripheral blood mononuclear cells (148) makes it possible to assay for promoter activity in this cellular population. Following elutriation, monocytes are placed into culture with IL-4 and GM-CSF as reported by Sallusto and Lanzavecchia (148). After 48 hours a representative sample is evaluated by FACS for DC phenotype, i.e., CD3-, CD19-, CD14-, CD1a-c+, CD80+, HLA-DR+, and HLA-DQ+. Upon demonstration of DC phenotype, cationic lipid transient transfections are carried out as above with identical assay procedures followed.

Upon conclusion of these experiments the appropriate promoter fragment is determined based on the activities in the above three cellular preparations. Preferred, is a promoter which drives optimal expression in muscle cells, or antigen presenting cells of the target antigen(s). The promoter fragment is ligated into the pITL vector. This constitutes the base vector. The vector is transfected using the standard heat shock method into frozen competent DH10 β/p3 *E. coli* bacteria (Gibco BRL, Gaithersburg, MD), grown under ampicillin and tetracycline selection as previously described. The cloning site of the base vector is the location of all subsequent cloning of reporter gene sequences or target antigen sequences.

The computer generated nucleic acid sequence which approximates the sequence of one base vector, pITL, is as follows:

| | | | | |
|---|---|---|---|---|
| TGCCATGGCG | CGGATTCTTT | ATCACTGATA | AGTTGGTGGA | CATATTATGT |
| TTATCAGTGA | TAAAGTGTCA | AGCATGACAA | AGTTGCAGCC | GAATACAGTG |
| ATCCGTGCCG | GCCCTGGACT | GTTGAACGAG | GTCGGCGTAG | ACGGTCTGAC |
| GACACGCAAA | CTGGCGGAAC | GGTTGGGGGT | GCAGCAGCCG | GCGCTTTACT |
| GGCACTTCAG | GAACAAGCGG | GCGCCTTAAG | GGCCATATGG | TGAGTGGATG |
| CCTTGACCCC | AGGCGGGGAT | GGGGGAGACC | TGTAGTCAGA | GCCCCCGGGC |
| AGCACAGGCC | AATGCCCGTC | CTTCCCCTGC | AGTGAGTAGT | GACTGCCCGG |
| GTGGGATCCC | TGTGACCCCT | CCCCAGTGCC | TCTCCTGGCC | CTGGAAGTTG |
| CCACTCCAGT | GCCCACCAGC | CTTGTCCTAA | TAAAATTAAG | TTGCATCATT |
| TTGTCTGACT | AGGTGTCCTC | TATAATATTA | Taagcttgat | atcgAATTCT |
| TTCTCAACGT | AACACTTTAC | AGCGGCGCGT | CATTTGATAT | GATGCGCCCC |

GCTTCCCGAT AAGGGAGCAG GCCAGTAAAA GCATTACCCG TGGTGGGGTT
CCCGAGCGGC CAAAGGGAGC AGACTCTAAA TCTGCCGTCA TCGACTTCGA
AGGTTCGAAT CCTTCCCCCA CCACCATCAC TTTCAAAAGT CCGAAAGAAT
Tcctgcagcc cGTGTAGCCG TAGTTAGGCC ACCACTTCAA GAACTCTGTA
GCACCGCCTA CATACCTCGC TCTGCTAATC CTGTTACCAG TGGCTGCTGC
CAGTGGCGAT AAGTCGTGTC TTACCGGGTT GGACTCAAGA CGATAGTTAC
CGGATAAGGC GCAGCGGTCG GGCTGAACGG GGGGTTCGTG CACACAGCCC
AGCTTGGAGC GAACGACCTA CACCGAACTG AGATACCTAC AGCGTGAGCA
TTGAGAAAGC GCCACGCTTC CCGAAGGGAG AAAGGCGGAC AGGTATCCGG
TAAGCGGCAG GGTCGGAACA GGAGAGCGCA CGAGGGAGCT TCCAGGGGGA
AACGCCTGGT ATCTTTATAG TCCTGTCGGG TTTCGCCACC TCTGACTTGA
GCGTCGATTT TTGTGATGCT CGTCAGGGGG GCGGAGCCTA TGGAAAAACG
CCAGCAACGC GGCCgggga tccggaGAGC TCACTCTAGA TGAGAGAGCA
GTGAGGGAGA GACAGAGACT CGAATTTCCG GAGCTATTTC AGTTTTCTTT
TCCGTTTTGT GCAATTTCAC TTATGATACC GGCCAATGCT TGGTTGCTAT
TTTGGAAACT CCCCTTAGGG GATGCCCCTC AACTGGCCCT ATAAAGGGCC
AGCCTGAGCT GCAGAGGATT CCTGCAGAGG ATCAAGACAG CACGTGGACC
TCGCACAGCC TCTCCCACAG GTACC 1425 base pairs (SEQ. ID. NO. 16),

and variants thereof.

The minimal ColE1 origin of replication comprises base pairs 712 through 1164 of pITL, the SupF sequence comprises base pairs 495 through 701, the minimal RANTES promoter comprises base pairs 1177 through 1425, the stuffer sequence comprises base pairs 1 through 221 and the combined splice and poly A sequences comprises base pairs 222 through 481. Base pairs 482 through 494, 602 through 611, and 1165 through 1176 are extraneous, noncoding sequences derived from plasmids from which the component sequences were excised from (sequences in lower case in pITL sequence).

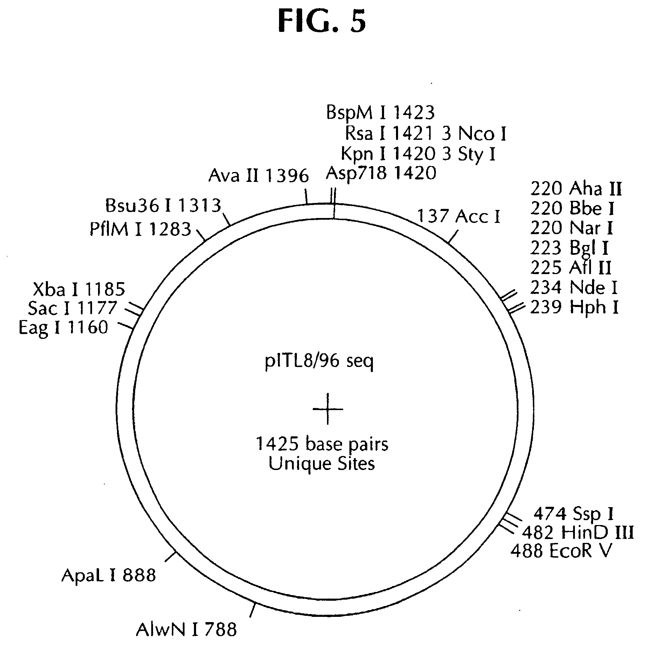

An enzyme restriction map of pITL is provided in Figure 5.

## EXAMPLE 3

### Purification Of Vector

The vector, pITL, requires the presence of the p3 helper plasmid for appropriate selection and subsequent isolation. However, the p3 helper plasmid is not part of the therapeutic product. Standard plasmid DNA isolation techniques cannot selectively isolate pITL (1.5 - 2.0 kb) from the much larger p3 plasmid (60kb). To avoid administering a contaminating plasmid as part of the polynucleotide vaccine preparation, a separate isolation procedure is established based on anion exchange chromatography. Anion exchange resins have a higher affinity for large DNA molecules than small molecules at any given NaCl concentration. The 30 fold larger p3 plasmid will be retained at a NaCl concentration in the elution gradient which releases the pITL. After standard alkaline/SDS lysis of large volume culture, digestion with RNAse A at 100ug/ml, and isopropanol precipitation the resultant pellet is resuspended in TE and applied to the anion exchange column. Small disposable Nucleobond AX columns (Nest Group Inc., Southborough, MA) or MonoQ column (Pharmacia Biotech, Piscataway, NJ) are run on an FPLC system (Pharmacia Biotech, Piscataway, NJ) with a NaCl gradient from 0.01M to 3M. The covalently closed circular (CCC) form of the pITL construct is eluted at less than 1M NaCl while the p3 plasmid is retained. The open circular (OC) form of the pITL plasmid is eluted at a lower NaCl concentration in the gradient and is discarded. Columns are used only once to avoid cross contamination. The resultant retained (CCC) fractions are precipitated with ethanol at -20°C. The pellet is resuspended with sterile PBS and stored aseptically at-20°C until use. Purity of the preparation is assessed by agarose electrophoresis and ethidium bromide staining of undigested and HindIII digested samples. Hind III digests linearize both pITL and the p3 helper plasmid. An ultraviolet spectra across the 240nm to 320 nm range is also obtained on an existing DU 65 UV/Vis spectrophotometer (Beckman, Fullerton, CA). Sample aliquots are retained for microbiological evaluation as needed in the animal experiments. The polynucleotide vector of the present invention is greater than 95% pure, preferably greater than 99% pure, more preferably greater than 99.9% for use in humans.

**EXAMPLE 4**

**Evaluation of the kinetics of expression of a reporter sequence from the plasmid pITL in an Animal Model**

A rat model using Fisher 344 rats is used to evaluate the polynucleotide vaccine vector. The rat model was chosen over mouse to avoid the complication of the mouse mammary tumor virus which is present in most mouse mammary tumor lines and which can function as a superantigen causing significant alterations in the immune system. Cohorts of 45 rats, vaccinated with a given dose of DNA are used, for evaluation of toxicity and target sequence expression over a period of 60 days. Three individual members of each cohort are sacrificed and submitted for necropsy on days; 0, 1, 2, 3, 5, 7, 14, 21, 28, 35, 42, 49, and 56 in order to evaluate sequence expression and toxicity. Techniques of polynucleotide vaccination have been described previously (102, 132a, 133a, 134a, 107, 119). All animals receive intramuscular injections using standard sterile technique and 27 gauge needles however, in an effort to more accurately mimic the clinical situation the muscle is not surgically exposed. The lateral limb of individual animals is clipped, as needed, and cleansed with 70 % ethanol prior to injections. On day # -1 the left and right gastrocnemius muscle is injected with 200 microlitres of 0.5% bupivacaine-HCL and 0.1 % methylparaben in isotonic NaCl ( 0.5% Marcaine, Sanofi Winthrop Pharmaceuticals or 0.5 % Sensorcaine, Astra USA, Inc.). The injection site is identified by a cutaneous tattoo, < 1 mm diameter, with sterile Indian Ink (autoclaved 20 min.) placed at the time of bupivacaine injection. On Day 0 the closed circular DNA preparation in a volume of 200 microlitres of injection grade normal saline is injected into one limb only at the same site as the bupivacaine injection as noted by the tattoo. The opposite limb will be used as a control with an equal volume of sterile injection grade normal saline, without any DNA, administered in the same fashion as above.

The "humanized" nucleic acid sequence encoding a green fluorescent protein sequence portion or variant thereof present in the Green Lantern vector (Gibco BRL, Gaithersburg, MD) is utilized. This vector results in production of a green fluorescent protein in human skeletal muscle cells in vitro and in vivo. This reporter protein has the advantage of not requiring any treatment of tissue to visualize expression and it is stable in both frozen section and formalin fixed paraffin embedded tissues. Using fluorescent microscopy trace expression of this protein is accurately identified using conditions used for evaluation of FITC labeled antibodies. Therefore, this reporter also provides the opportunity to evaluate reticuloendothelial cells by FACS to evaluate possible uptake and expression of the reporter vector. PCR primers to the green lantern protein are used with the appropriate Bgl I 5' extensions to amplify and clone the cDNA encoding green fluorescent protein into pITL, in a manner identical to that used to insert tumor target antigen sequences.

The construct containing the green fluorescent protein, pITL-GFP, is sequenced by standard dideoxy sequencing techniques to confirm the fidelity of the cloning process. The nucleic acid sequence for GFP target sequence contained in pITL is as follows:

ATGAGCAAG GGCGAGGAAC TGTTCACTGG CGTGGTCCCA ATTCTCGTGG
AACTGGATGG CGATGTGAAT GGGCACAAAT TTTCTGTCAG CGGAGAGGGT
GAAGGTGATG CCACATACGG AAAGCTCACC CTGAAATTCA TCTGCACCAC
TGGAAAGCTC CCTGTGCCAT GGCCAACACT GGTCACTACC TTCACCTATG
GCGTGCAGTG CTTTTCCAGA TACCCAGACC ATATGAACGA GCATGACTTT
TTCAAGAGCG CCATGCCCGA GGGCTATGTG CAGGAGAGAA CCATCTTTTT
CAAAGATGAC GGGAACTACA AGACCCGCGC TGAAGTCAAG TTCGAAGGTG
ACACCCTGGT GAATAGAATC GAGTTGAAGG GCATTGACTT TAAGGAAGAT
GGAAACATTC TCGGCCACAA GCTGGAATAC AACTATAACT CCCACAATGT
GTACATCATG GCCGACAAGC AAAAGAATGG CATCAAGGTC AACTTCAAGA
TCAGACACAA CATTGAGGAT GGATCCGTGC AGCTGGCCGA CCATTATCAA
CAGAACACTC CAATCGGCGA CCGCCCTGTG CTCCTCCCAG ACAACAATTA
CCTGTCCACC CAGTCTGCCC TGTCTAAAGA TCCCAACGAA AAGAGAGACC
ACATGGTCCT GCTGGAGTTT GTGACCGCTG CTGGGATCAC ACATGGCATG
GACGAGCTGT ACAAGTGAGC (SEQ. ID. NO. 17), and analogs thereof.

The pITL-GFP DNA is prepared as described above, for the vaccination procedure. In one embodiment, the computer generated sequence which approximates the full pITL-GFP sequence comprises:

Tatgagcaagggcgaggaactgttcactggcgtggtcccaattctcgtggaactggat
ggcgatgtgaatgggcacaaattttctgtcagcggagagggtgaaggtgatgccacat
acggaaagctcaccctgaaattcatctgcaccactggaaagctccctgtgccatggccaa
cactggtcactaccttcacctatggcgtgcagtgcttttccagatacccagaccatat
gaagcagcatgactttttcaagagcgccatgcccgagggctatgtgcaggagagaacc
atctttttcaaagatgacgggaactacaagacccgcgctgaagtcaagttcgaaggtg
acaccctggtgaatagaatcgagttgaagggcattgactttaaggaagatggaaacat
tctcggccacaagctggaatacaactataactcccacaatgtgtacatcatggccgac
aagcaaaagaatggcatcaaggtcaacttcaagatcagacacaacattgaggatggat

**ccgtgcagctggccgaccattatcaacagaacactccaatcggcgacggccctgtgct**
**cctcccagacaaccattacctgtccacccagtctgcccgtctaaagatcccaacgaaa**
**agagagaccacatggtcctgctggagtttgtgaccgctgctgggatcacacatggcat**
**ggacgagctgtacaagtgagc**CATATGGTGAGTGGATGCCTTGACCCCAGGCGGGAT
GGGGGAGACCTGTAGTCAGAGCCCCCGGGCAGCACAGGCCAATGCCCGTCCTTCCCCT
GCAGTGAGTAGTGACTGCCCGGGTGGGATCCCTGTGACCCCTCCCCAGTGCCTCTCCT
GGCCCTGGAAGTTGCCACTCCAGTGCCCACCAGCCTTGTCCTAATAAAATTAAGTTGC
ATCATTTTGTCTGACTAGGTGTCCTCTATAATATTATaagcttgatatcgAATTCTTT
CTCAACGTAACACTTTACAGCGGCGCGTCATTTGATATGATGCGCCCCGCTTCCCGAT
AAGGGAGCAGGCCAGTAAAAGCATTACCCGTGGTGGGGTTCCCGAGCGGCCAAAGGGA
GCAGACTCTAAATCTGCCGTCATCGACTTCGAAGGTTCGAATCCTTCCCCCACCACCA
TCACTTTCAAAAGTCCGAAAGAATTcctgcagcccGTGTAGCCGTAGTTAGGCCACCA
CTTCAAGAACTCTGTAGCACCGCCTACATACCTCGCTCTGCTAATCCTGTTACCAGTG
GCTGCTGCCAGTGGCGATAAGTCGTGTCTTACCGGGTTGGACTCAAGACGATAGTTAC
CGGATAAGGCGCAGCGGTCGGGCTGAACGGGGGGTTCGTGCACACAGCCCAGCTTGGA
GCGAACGACCTACACCGAACTGAGATACCTACAGCGTGAGCATTGAGAAAGCGCCACG
CTTCCCGAAGGGAGAAAGGCGGACAGGTATCCGGTAAGCGGCAGGGTCGGAACAGGAG
AGCGCACGAGGGAGCTTCCAGGGGGAAACGCCTGGTATCTTTATAGTCCTGTCGGGTT
TCGCCACCTCTGACTTGAGCGTCGATTTTTGTGATGCTCGTCAGGGGGGCGGAGCCTA
TGGAAAAACGCCAGCAACGCGGCCggggatccggaGAGCTCACTCTAGATGAGAGAG
CAGTGAGGGAGAGACAGAGACTCGAATTTCCGGAGCTATTTCAGTTTTCTTTTCCGTT
TTGTGCAATTTCACTTATGATACCGGCCAATGCTTGGTTGCTATTTTGGAAACTCCCC
TTAGGGGATGCCCCTCAACTGGCCCTATAAAGGGCCAGCCTGAGCTGCAGAGGATTCC
TGCAGAGGATCAAGACAGCACGTGGACCTCGCACAGCCTCTCCCACAGGTACC (SEQ ID NO. 18) and analogs thereof. The nucleic acid sequence encoding the green fluorescent protein sequence are in lower case and bolded.

Toxicity from polynucleotide vaccination, as reported in the literature (102, 132*a*, 133*a*, 134*a*, 135*a*, 103, 104-131), has been limited to minimal local inflammatory responses at the site of injection. Expression of the target protein, GFP, is expected through 60 days. The literature supports extended expression, upwards of 18 months, with other conventional vectors.

Cohort #1 consists of 45 rats each 200 microlitres of normal saline without DNA. Cohort #2 consists of 45 rats each receiving 1 microgram of pITL-GFP closed circular plasmid DNA. Cohort # 3 consists of 45 rats each receiving 10 micrograms of pITL-GFP closed circular plasmid DNA. Cohort # 4 consists of 45 rats each receiving 100 micrograms of pITL-GFP closed circular plasmid DNA. Cohort # 5 consists of 45 rats each receiving 1 milligram of pITL-GFP closed circular plasmid DNA.

Three animals from each of the cohorts # 1 through 5 are euthanized on day # 0, 1, 2, 3, 5, 7, 14, 21, 28, 35, 42, 49, and 56, either by inhaled carbon dioxide or cervical dislocation. Animals are examined at necropsy immediately following euthanasia. The following tissue specimens are submitted for histologic evaluation by H & E stained sections: skeletal muscle and skin from both injection sites, normal skin and skeletal muscle, inguinal lymph node(s), thymus, bone marrow (femur), spleen, gastrointestinal tract with Peyer's patches, heart, lung, liver, kidney, brain/spinal cord, and any other tissues which appear grossly abnormal at the time of necropsy. Tissues from these same organs are prepared and frozen in OCM media for frozen section evaluation of GFP expression by fluorescent microscopy and stored at -70°C for future studies. Blood specimens not to exceed 2 ml are collected by retro-orbital plexus or tail vein phlebotomy no more than weekly for evaluation of hematologic parameters.

If distant or significant local toxicity is detected at any of the DNA dose levels using the pITL-GFP, additional cohorts of 45 animals is treated identically but, administered pITL (without the reporter sequence) at identical dose levels to pITL-GFP and at all subsequent increased dose levels. If no toxicity is seen at any level a single cohort, #5a, 45 additional rats will receive the highest DNA dose level of pITL to confirm the absence of vector derived toxicity.

## EXAMPLE 5

### Evaluation of the impact of tumor presence on the kinetics of polynucleotide vaccine expression, pITL

Previous work by Ochoa and colleagues has suggested that in tumor bearing mice there is a profound immune deficit characterized by T cell receptor and signal transduction defects (149). In light of these observations, the presence of tumor might alter the stability and expression by the polynucleotide vaccine. It is possible that these defects will be manifest in this model and may result in alterations of the immune response to the reporter protein. This maybe manifest by increased and persistent expression or a skewing of the immune response with a predominance of antibodies and subsequent ADCC or immune complex deposition. Therefore, these parameters are evaluated in tumor bearing animals. The syngeneic rat mammary tumor line 13762 (ATCC #1666-CRL) which is known to express rat HER2/neu is utilized.

Cohorts # 6 through 10, 30 rats per cohort, involve animals with established 13762 mammary tumors. Rats are injected with 5 x $10^5$ cells into the subcutaneous space 14 days prior to receiving polynucleotide vaccination. All rats receive an identical vaccine sequence as that described above. Cohort #6 receive 200 microlitres of normal saline without DNA. Cohort #7 receive 1 microgram of pITL-GFP closed circular plasmid DNA. Cohort #8 receive 10 micrograms of pITL-GFP closed circular plasmid DNA. Cohort #9 receive 100 micrograms of pITL-GFP closed circular plasmid DNA. Cohort #10 receive 1 milligram of pITL-GFP closed circular plasmid DNA.

Three animals from each of the cohorts #6 through 10 are euthanized on Day # 3, 7, 14, 21, 28, 35, 42, 49, and 56 or earlier if animals become moribund due to tumor growth. Animals are necropsied immediately after euthanasia. Tissue specimens are identical to cohorts #1 through 4 with the exception that sections of the tumor nodule is submitted for H&E stained histochemistry and a specimen is frozen as above. Blood specimens not to exceed 2 ml is collected by retro-orbital plexus or tail vein phlebotomy no more than weekly for evaluation of hematologic parameters. Clearly, if tumor growth is either accelerated or sustained this will cause morbidity to the animals.

## EXAMPLE 6

### Evaluation of immune response and toxicities to anti-tumor vaccination, using constructs with partial sequences of rat HER2/neu, in normal and tumor bearing rats

The C-erbB-2 or HER-2/neu gene product, originally isolated from neuroblastoma tumor lines, is homologous to the epidermal growth factor receptor (150-154) and expressed in up to 40% of breast carcinomas (26). Since the original characterization of this sequence the amplification and overexpression of the protein has been variably associated with prognosis in both ovarian and breast carcinomas (155-165). In murine cell lines, activation of HER-2/neu has been associated with mutations in the transmembrane domain. However, to date most tumor specimens demonstrate gene amplification rather than point mutations (155, 157, 158, 166). HER2/neu amplification occurs in up to one third of breast carcinomas. Monoclonal antibodies recognizing the c-erbB-2 or HER2/neu protein have been shown to be cytostatic in vitro for cell lines overexpressing the c-erbB-2 or HER2/neu protein (167-170). Additionally, CTL lines isolated from patients with ovarian carcinoma have been shown to recognize synthetic peptides corresponding to two regions on the HER2/neu protein (74) despite the fact that c-erbB-2 has been reported to be expressed in the aerodigestive and urologic tract (171). These findings suggest that HER2/neu can be a source of tumor antigens for an anti-tumor immune response.

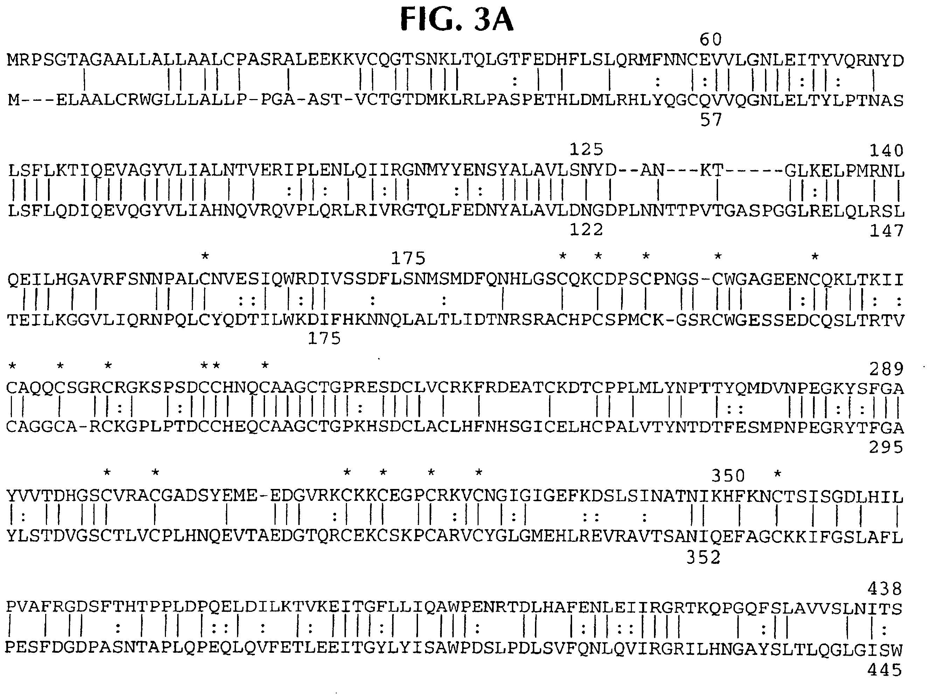

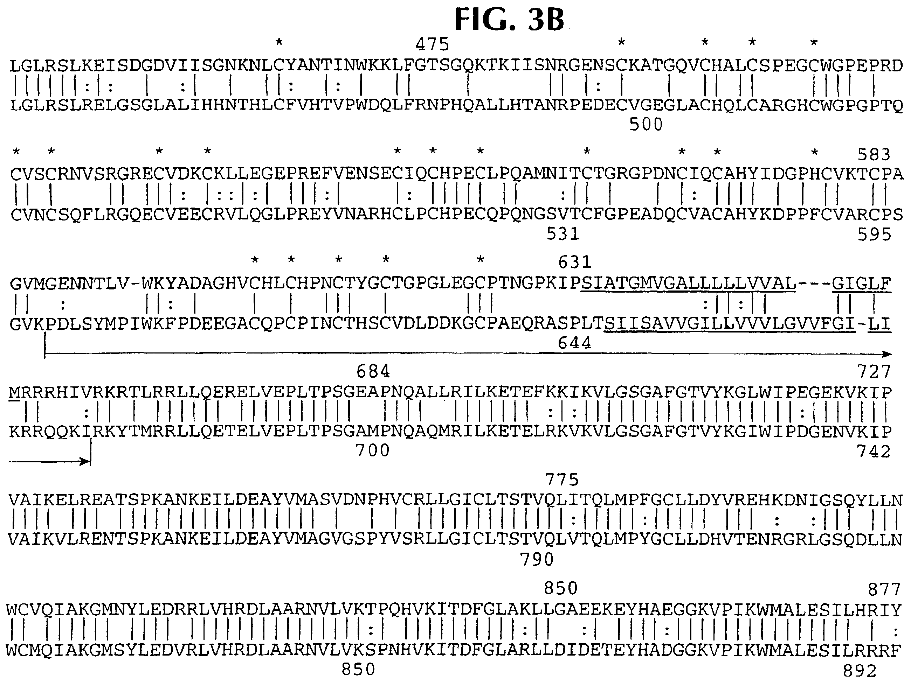

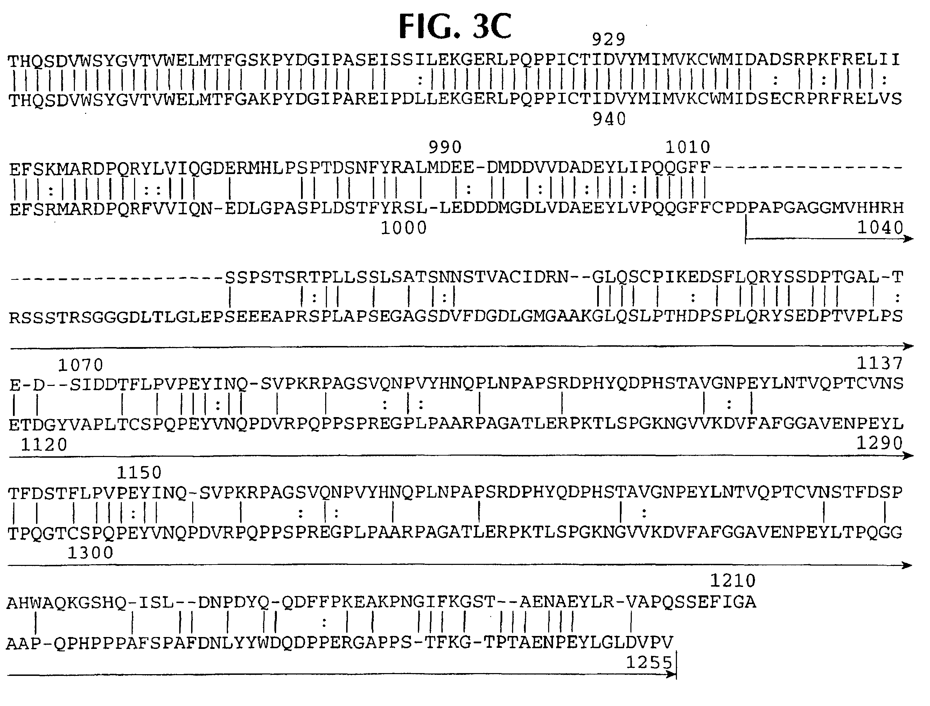

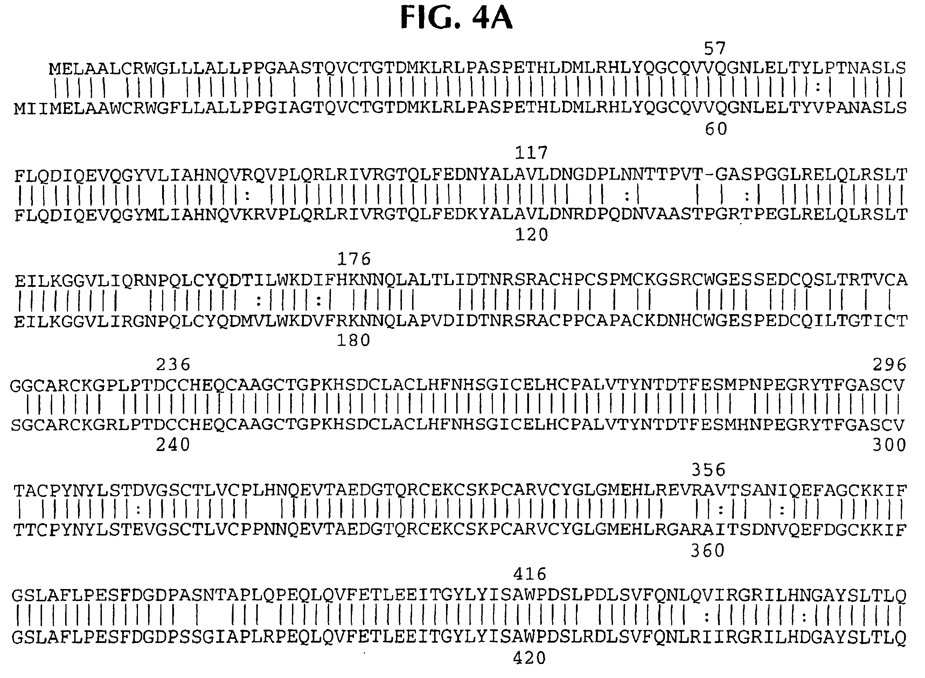

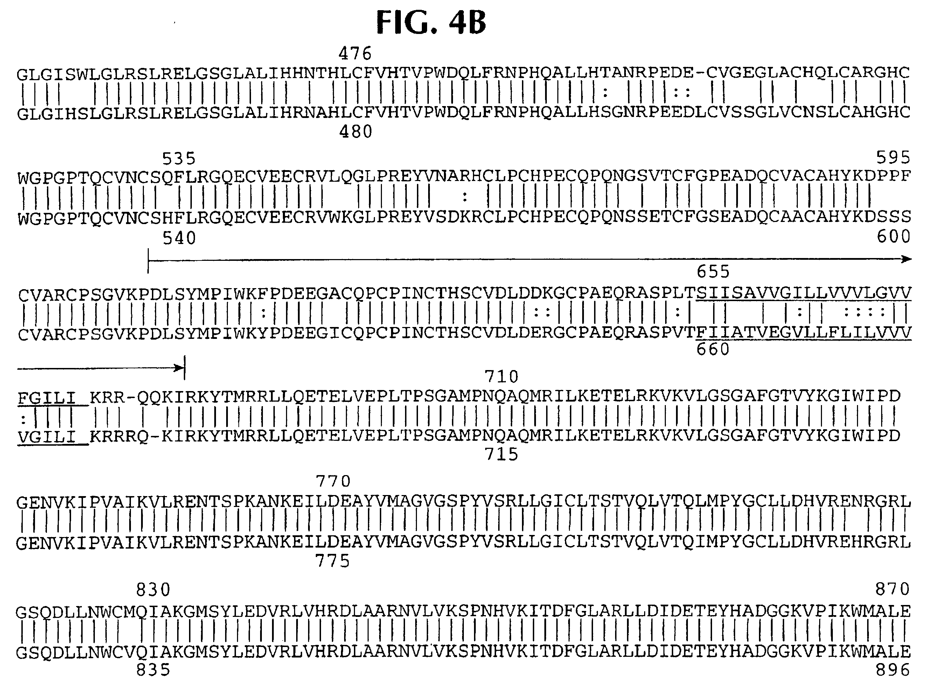

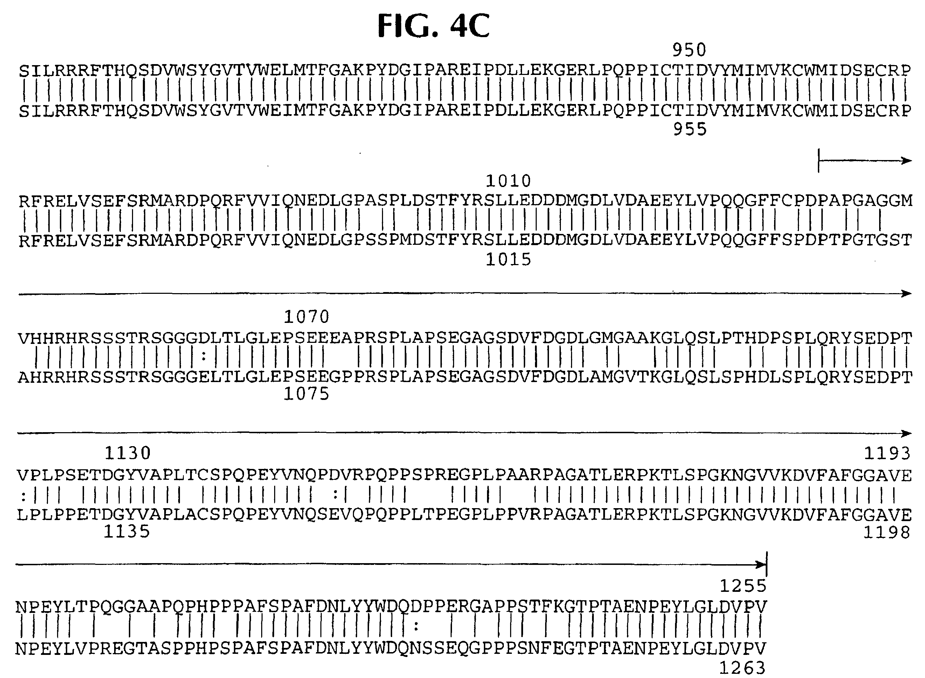

The choice of HER2/neu as the initial target, tumor-associated, antigen is predicated upon considerations of the animal model and the human clinical trial. A concern of polynucleotide vaccination is the theoretical induction of an autoimmune phenomena to related normal proteins. HER2/neu shares the high degree of homology with other normal cellular proteins, i.e. with other members of the EGFR family (150, 154) as shown in Figure 3. Therefore, a phase 1 clinical trial with this target antigen is most likely to expose this potential toxicity. The human Her2/neu target antigen corresponds with amino acid residues in Figure 3. Additionally, it has been demonstrated that the rat mammary tumor 13762 expresses rat neu. To minimize the potential for autoimmune phenomena a limited partial sequence, including the transmembrane domain (the site of the activating mutation in rat neu), and a segment of the cytoplasmic domain with limited homology to EGFRs is constructed using rtPCR techniques from rat 13762 and the human breast cancer cell line SK-BR-3 (ATCC # HTB-30). These sequences share very limited homology with other EGFR members. Identical sections of the rat and human sequence are utilized. The target amino acid sequence for both human Her2/neu and rat Her2/neu is depicted in Figure 4.

The nucleic acid sequence encoding the partial human Her 2/neu target sequence is ligated at the initiation site of the polynucleotide vector. In one embodiment, the nucleic acid sequences encode at least one or a combination of the following amino acid sequences or portion thereof of Her 2/neu:

PDLSYMPIWKF PDEEGACQPC PINCTHSCVD LDDKGCPAEQ RASPLTSIIS AVVGILLVVV LGVVFGIL (SEQ. ID NO. 19), or portion or mammalian homolog thereof and, PAPGAGGMVH HRHRSSSTRS GGGDLTLGLE PSEEEAPRSP LAPSEGAGSD VFDGDLGMGA AKGLOSLPTH DPSPLQRYSE DPTVPLPSET DGYVAPLTCS PQPEYVNQPD VRPOPPSPRE GPLPAARPAG ATLERPKTLS PGKNGVVKDV FAFGGAVENP EYLTPQGTCS PQPEYVNQPD VRPQPPSPRE GPLPAARPAG ATLERPKLSP GKNGVVKDVF AFGGAVENPE YLTPQGGAAP QPHPPPAFSP AFDNLYYWDO DPPERGAPPS TFKGTPTAEN PEYLGLDVPV (SEQ. ID NO. 20), or portion or mammalian homolog thereof.

In another embodiment, the nucleic acid sequences encode at least one region corresponding to the transmembrane domain of Her 2/neu comprising the amino acid sequence:

IISAVVGILLVVVLGVVFGILI (SEQ. ID NO. 21), or portion or mammalian homolog thereof.

The respective nucleic acid sequences encoding the target antigen are ligated into pITL. Subsequent clones are sequenced by standard dideoxy sequencing techniques (Sequenase, USB/Amersham, Arlington Heights, IL) to assure fidelity of the insert. Appropriate clones of the vector are transformed into DH10 b/p3, amplified, purified, and characterized as described above. These preparations are used in the following animal experiments.

Cohorts #11 through 15, 20 rats per cohort, receive three repeated injections in the same limb, every three weeks, administered exactly as described above with the exception that the tattoo is placed only once. The polynucleotide vaccine preparation consist of pITL-rHER2/neu, i.e., pITL with a partial rat HER2/neu cDNA sequence. Two weeks after the final injection the rats are challenged with $5 \times 10^5$ 13762 tumor cells in no more than 0.5 ml sterile normal saline administered into the subcutaneous space. Cohort #11 receives only sterile saline and no closed circular plasmid DNA per injection. Cohort #12 receives 1 microgram of pITL-rHER2/neu closed circular plasmid DNA per injection. Cohort #13 receives 10 micrograms of pITL-rHER2/neu closed circular plasmid DNA per injection. Cohort #14 receives 100 micrograms of pITL-rHER2/neu closed circular plasmid DNA per injection. Cohort #15 receives 1 milligram of pITL-rHER2/neu closed circular plasmid DNA per injection. Cohorts #16 through #20, 20 rats per cohort, have $5 \times 10^5$ cells in no more than 0.5 ml sterile normal saline administered into the subcutaneous space 10 to 14 days prior to initiating a polynucleotide vaccine sequence of three injections administered every three weeks so as to have an established tumor not to exceed 1.0 cm in diameter. Cohort #16 receives only sterile saline and no closed circular plasmid DNA per injection. Cohort #17 receives 1 microgram of pITL-rHER2/neu closed circular plasmid DNA per injection. Cohort #18 receives 10 micrograms of pITL-rHER2/neu closed circular plasmid DNA per injection. Cohort #19 receives 100 micrograms of pITL-rHER2/neu closed circular plasmid DNA per injection. Cohort #20 receives 1 milligram of pITL-rHER2/neu closed circular plasmid DNA per injection.

Animals from cohorts #11 through 15 have venous blood samples obtained pre-vaccine initiation, at the time of each vaccination, at the time of tumor challenge, and every three weeks thereafter. These samples are used to assess humoral immune responses by FACS analysis on both 13762 tumor cells and RAT2 cells transiently transfected with the vaccine construct, pITL-rHER2/neu, using cationic lipid techniques as described above for promoter evaluation. A 'sandwich' ELISA is also used to evaluate the development of anti-HER2/neu antibodies, i.e. using test serum as the capture antibody, application of either SKBR3 (HER2/neu + human breast cancer cell line) or 13762 cellular lysates, detection with anti-rat or anti-human HER2/neu antibody ( Ab-4 and Ab-2 or Ab-5 respectively, Oncogene Sciences) and followed by an appropriate anti-isotope enzyme labeled detection antibody and calorimetric determination.

Three individual animals from each cohort are euthanized at the following time points: at the time of tumor challenge, two weeks after tumor challenge, four weeks after tumor challenge, six weeks after tumor challenge, and the remaining animals when moribund or no later than one year after tumor challenge. Euthanized animals have spleens harvested for isolation of T lymphocytes, tumor nodule sampling for histochemical examination (both fixed and frozen section), and necropsied with gross abnormalities directing further tissue section submission. Splenic T lymphocytes are isolated used to assay tumor specific CTL precursor frequency using limiting dilution assays (89) and signal transduction defects as previously described for mouse model systems (149, 172).

Animals from cohorts #16 through #20 have venous blood samples obtained pre-tumor inoculation, pre-vaccine initiation, at the time of each vaccination, and every three weeks thereafter. These samples are used to assess humoral immune responses as described above. Three individual animals from each cohort are euthanized at the following time points: at the time of first vaccination (pre treatment), at the time of second vaccination, at the time of the third vaccination, 3 weeks after completion of vaccination, 6 weeks after completion of the vaccination sequence, 12 weeks after completion of the vaccination sequence, and the remaining animals when moribund or no later than one year after completion of the vaccination sequence. Euthanized animals have spleens harvested for isolation of T lymphocytes, tumor nodule sampling for histochemical examination (both fixed and frozen section), and necropsied with gross abnormalities directing further tissue section submission. Splenic T lymphocytes are isolated used to assay tumor specific CTL precursor frequency and signal transduction defects.

## EXAMPLE 7

### Evaluation of toxicity of anti-tumor vaccination with constructs containing partial sequences of human HER2/neu in rats

Cohorts #21 through 25, 45 rats per cohort, receive three repeated injections in the same limb, every three weeks, administered exactly as described above with the exception that the tattoo is placed only once. The construct used is the base vector with the human partial HER2/neu sequence construct, pITL-hHER2/neu. The nucleic acid sequence of one embodiment of a pITL-hHer2/neu polynucleotide vector vaccine comprises:

GCCACCATGGCCcctgacctctcctacatgcccatctggaagtttccagatgaggagggcgcatgccagcc
ttgccccatcaactgcacccactcctgtgtggacctggatgacaagggctgccccgccgagcagagagccagccct
ctgacgtccatcatctctgcggtggttggcattctgctggtcgtggtcttgggggtggtctttgggatcctcatcaagcg
acggcagcagaagatcacatgtccagaccctgccccgggcgctgggggcatggtccaccacaggcaccgcagctc
atctaccaggagtggcggtggggacctgacactagggctggagccctctgaagaggaggcccccaggtctccactg
gcaccctccgaaggggctggctccgatgtatttgatggtgacctgggaatgggggcagccaaggggctgcaaagcc
tccccacacatgaccccagccctctacagcggtacagtgaggaccccacagtacccctgccctctgagactgatggc
tacgttgccccctgacctgcagcccccagcctgaatatgtgaaccagccagatgttcggccccagcccccct
tcgccccgagagggccctctgcctgctgcccgacctgctggtgccactctggaaaggcccaagactctctccccagg
gaagaatggggtcgtcaaagacgtttttgcctttgggggtgccgtggagaaccccgagacttgacaccccagggag
gagctgcccctcagccccaccctcctcctgccttcagcccagccttcgacaacctctattactgggaccaggacccac
cagagcgggggctccacccagcaccttcaaagggacacctacggcagagaacccagagtacctgggtctggac
gtgccagtgtgaaGCCTTAAGGGCCATATGGTGAGTGGATGCCTTGACCCCAGG
CGGGGATGGGGGAGACCTGTAGTCAGAGCCCCCGGGCAGCACAGGCCAA
TGCCCGTCCTTCCCCTGCAGTGAGT AGTGACTGCCCGGGTGGGATCCCTG
TGACCCCTCCCCAGTGCCTCT CCTGGCCCTGGAAGTTGCCACTCCAGTGC
CCACCAGCCTTGTCCTAATAAAATTAAGTTGCATCATTTTGTCTGACTAGG
TGTCCTCTATAATATTATaagcttg atatcgAATTCTTTCTCAACGTAACACTTTA
CAGCGGCGCGTCATTTGATATGATGCGCCCCGCTTCCCGATAAGGGAGCA
GGCCAGTAAAAGCATTACCC GTGGTGGGGTTCCCGAGCGGCCAAAGGGA
GCAGACTCTAAATCTGCCGTC ATCGACTTCGAAGGTTCGAATCCTTCCCC
CACCACCATCACTTTCAAAAGTCCGAAAGAATTcctgcagcccGTGTAGCCGTA
GTTAGGCCACCACTTCAAGAACTCTGTAGCACCGCCTACATACCTCGCTC
TGCTAATCCTGTTACC AGTGGCTGCTGCCAGTGGCGATAAGTCGTGTCTT
ACCGGGTTGGACTCAAGA CGATAGTTACCGGATAAGGCGCAGCGGTCGG
GCTGAACGGGGGGTTCGTGCACACAGCCCAGCTTGGAGCGAACGACCTAC
ACCGAACTGAGATACCTACAGCGTGAGCATTGAGAAAGCGCCACGCTTCC
CGAAGGGAGAAAGGCGGACAGGTATCCGGTAAGCGGCAGGGTCGGAACA
GGAGAGCGCACGAGGGAGCTTCCAGGGGGAAACGCCTGGTATCTTTATAG
TCCTGTCGGGTTTCGCCACCTCTGACTTGAGCGTCGATTTTTGTGATGCTC
GTCAGGGGGGCGGAGCCTATGGAAAAACGCCAGCAACGCGGCCgggggatcc

ggaGAGCTCACTCTAGATGAGAGAGCAGTGAGGGAGAGACAGAGACTCGA
ATTTCCGGAGCTATTTCAGTTTTCTTTTCCGTTTTGTGCAATTTCACTTATG
ATACCGGCCAATGCTTGGTTGCTATTTTGGAAACTCCCCTTAGGGGATGC
CCCTCAACTGGCCCTATAAAGGGCCAGCCTGAGCTGCAGAGGATTCCTGC
AGAGGATCAAGACAGCACGTGGACCTCGCACAGCCTCTCCCACAGGTACCT
(SEQ ID NO. 22)

and analogs thereof. The nucleic acid sequence encoding the human Her 2/neu target sequence are in lower case and bolded.

Three animals from each of the cohorts # 21 through 25 are euthanized on Day # 0, 1, 2, 3, 5, 7, 14, 21, 28, 35, 42, 49, and 56, either by inhaled carbon dioxide or cervical dislocation. Animals are examined at necropsy immediately following euthanasia. The following tissue specimens are submitted for histologic evaluation by H & E stained sections: skeletal muscle and skin from both injection sites, normal skin and skeletal muscle, inguinal lymph node(s), thymus, bone marrow (femur), spleen, gastrointestinal tract with Peyer's patches, heart, lung, liver, kidney, brain/spinal cord, and any other tissues which appear grossly abnormal at the time of necropsy. Tissues from these same organs are prepared and frozen in OCM media for frozen section evaluation if indicated and stored at -70°C for future studies. Blood specimens not to exceed 2 ml are collected by retro-orbital plexus or tail vein phlebotomy no more than weekly for evaluation of hematologic parameters.

**EXAMPLE 8**

**Phase I Trial**

The Phase I study is designed to determine the maximal tolerated dose of a polynucleotide vaccine, evaluate potential toxicities, and immunological effect of the polynucleotide vector vaccine.

**VACCINE PREPARATION**

pITL plasmid vector and constructs containing the various target sequences are detailed herein. Individual plasmid constructs have been sequenced to confirm target sequence fidelity. These vectors are transfected using the standard heat shock method into frozen competent DH10 β/p3 E. Coli. bacteria (Gibco BRL, Gaithersburg, MD), grown under ampicillin and tetracycline selection as previously described. The vector pITL requires the presence of the p3 helper plasmid for appropriate selection and subsequent isolation. However, all current plasmid DNA isolation techniques cannot selectively isolate pITL (1.5 - 2.0 kb) from the much larger p3 plasmid (60kb). To avoid administering a contaminating plasmid as part of the polynucleotide vaccine preparation a separate isolation procedure is disclosed herein based on anion exchange chromatography. Anion exchange resins have a higher affinity for large DNA molecules than small molecules at any given NaCl concentration. The 30 fold larger p3 plasmid is retained at a NaCl concentration in the elution gradient which releases the pITL.

After standard alkaline/SDS lysis of large volume culture, digestion with RNAse A at 100ug/ml, and isopropanol precipitation the resultant pellet is resuspended in TE and applied to the anion exchange column. Small disposable Nucleobond AX columns (Nest Group Inc., Southborough, MA) or MonoQ column (Pharmacia Biotech, Piscataway, NJ) run on an FPLC system (Pharmacia Biotech, Piscataway, NJ) are used with a NaCl gradient form 0.01M to 3M. The covalently closed circular (CCC) form of the pITL construct is eluted at less than 1M NaCl while the p3 plasmid is retained and the open circular (OC) form of the pITL plasmid is eluted at a lower NaCl concentration in the gradient and is discarded. Columns are used only once to avoid cross contamination. The resultant retained fractions are precipitated with ethanol at -20°C. The pellet is resuspended with sterile PBS and stored aseptically at -20°C until use. Purity of the preparation is assessed by agarose electrophoresis and ethidium bromide staining of undigested and Hind III digested samples. Hind III digests linearizes both pITL and the 3 helper plasmid. An ultraviolet spectra across the 240nm to 320nm range is also used. Sample aliquots are retained for microbiological evaluation as needed.

**STUDY DESIGN**

The patients have histopathological confirmation of the diagnosis of adenocarcinoma of the breast. The specimen submitted to pathology is evaluated for surface expression of HER-2/neu immunohistochemistry. If tumor is available for biopsy the immunotherapy laboratory receives 2 $cm^3$ (minimum of 1 $cm^3$) of fresh tumor transported on ice. The sample is used to generate tumor cell and autologous normal breast epithelium or normal fibroblast cell cultures and for mRNA isolation for rtPCR analysis.

The following examples illustrate the use of the vectors of the present invention for the preparation of a pharmaceutical composition for determining the maximal tolerated dose of said polynucleotide vaccine.

All patients receive intramuscular (IM) injections (vaccine or controle). The vaccine consists of the Her 2/neu polynucleotide vaccine construct as detailed herein at the appropriate dose level per cohort, resuspended in 0.5 ml injection grade, sterile, normal saline and administered in the lateral quadriceps with the patient in the supine position. Cohort #1 receives three initial IM injections of normal saline control (no DNA) prior to receiving three polynucleotide vaccinations.

All intramuscular injections are preceded by IM injection of 0.5 ml. 0.5% Bupivacaine 24 hours before vaccine administration.

The injection site is marked with a sterile indian ink pinpoint tattoo at the time of the Bupivacaine administration.

Cohort #1

Patients receive IM bupivacaine followed by IM injection of sterile saline x 3 at three week intervals. Subsequently, patients receive a dose of 100 microgram of basal plasmid DNA, pITL without the Her 2/neu target sequence insert, per vaccination administered in the contralateral lower extremity at three week intervals x 3.

The first three patients have a muscle biopsy of the sterile saline injection site at week 12. The last three patients have a muscle biopsy of the basal plasmid injection site three weeks after the last vaccination, i.e. week 21.

In this cohort alone, if no toxicity is identified, the patients are vaccinated with the pITL-her 2/neu construct, in the lower extremity originally injected with bupivacaine and saline. The dose of the pITL-Her 2/neu construct is the highest dose at which two patients have been vaccinated at least twice without observed toxicity greater than grade 2.

Cohort #2

The initial vaccination consists of 1 microgram of the reporter plasmid, pITL with the PCR amplified GFP sequence. Patients receive a dose of 1 micrograms of pITL-Her 2/neu DNA per vaccination administered in the contralateral lower extremity at three week intervals x 3. Groups of 2 patients have a muscle biopsy of the reporter plasmid vaccination site at weeks 3, 6 and 9 respectively.

Cohort #3

The initial vaccination consists of 10 micrograms of the reporter plasmid, pITL with the PCR amplified GFP sequence. A dose of 25 micrograms pITL-Her 2/neu DNA per vaccination is administered in the contralateral lower extremity at three week intervals x 3. Groups of 2 patients have a muscle biopsy of the reporter plasmid vaccination site at weeks 3, 6, and 9 respectively.

Cohort #4

The initial vaccination consists of 25 micrograms of the reporter plasmid, pITL with the PCR amplified GFP sequence. A dose of 100 micrograms pITL-Her 2/neu DNA per vaccination is administered in the contralateral lower extremity at three week intervals x 3. Groups of 2 patients have a muscle biopsy of the reporter plasmid vaccination site at weeks 3, 6, and 9 respectively.

Cohort #5

The initial vaccination consists of 100 micrograms of the reporter plasmid, pITL with the PCR amplified GFP sequence. A dose of 1000 micrograms pITL-Her 2/neu DNA per vaccination is administered in the contralateral lower extremity at three week intervals x 3. Groups of 2 patients have a muscle biopsy of the reporter plasmid vaccination site at weeks 3, 6, and 9 respectively.

All patients are evaluated for anti-tumor responses at the conclusion *of* the vaccination sequence, every two months x 3, then every three months. Patients free from progression at 6 months are eligible for repeated vaccination sequences identical to that initially administered minus the initial vaccination with the reporter plasmid.

EVALUTATION

Medical history, T lymphocyte function, fluorescent cell sorting, antigen specific responses are performed as detailed in Example 9 below.

DOSE ADJUSTMENT AND MODIFICATIONS

The maximally tolerated dose (MTD) of total DNA per vaccination is defined as that dose at which 0 or 1/6 patients experience polynucleotide vaccine related dose limiting toxicity (DLT) with the next higher dose level provoking DLT in any 2 patients from that cohort.
If does limiting toxicity is observed in the first two patients of any cohort, the dose is reduced by 50% and the equivalent of a full cohort enrolled at this reduced dose unless dose limiting toxicity again encountered at this new dose.

**EXAMPLE 9**

**Phase I/II Evaluation Of Polynucleotide Vaccination in Advances Breast Cancer**

The following examples illustrate the use of the vectors of the present invention for the preparation of a pharmaceutical composition for the evaluation of polynucleotide vaccination in advanced breast cancer.

Polynucleotide Vaccine Preparation

The patients undergo an excisional biopsy with histopathological confirmation of the diagnosis of adenocarcinoma of the breast. The specimen submitted to pathology is also used for evaluation of surface expression of HER-2/neu and MUC-1 by immunohistochemistry. The immunotherapy laboratory receives 2 cm3 (minimum of lcm3) of fresh tumor transported on ice. This specimen is divided with 50% of the sample dispersed with collagenase and the resultant cellular preparation cryopreserved for use as described below. 10-15% of the sample is used to generate tumor cell and autologous normal breast epithelium or normal fibroblast cell cultures. The remainder is used for mRNA isolation and vaccine preparation with an immediately adjacent slice/section held for histologic confirmation and correlation. The sample is homogenized in TriZol solution (Gibco BRL) for isolation of total RNA. Messenger RNA is isolated with oligo dT agarose (Pharmacia) and subjected to rtPCR with first strand reverse transcription initiated from oligo d(T) 12N using StrataScript Rnase H- Reverse Transcriptase (Stratagene) and the high fidelity pfu (Stratagene) thermostable polymerase for the PCR reaction. (Chapter 15 in: Current Protocols in Molecular Biology, Vol. 2; Ausubel, F.M. et al (eds) John Wiley & Sons, Inc. 1995).
Specific PCR primers for the individual transcripts of interest are used in individual PCR reactions using an aliquot of the cDNA from the reverse transcription of the mRNA. An embodiment of rtPCR primers for the target sequence p53 is as follows:

p 53 rtPCR primer 1: g tctgccacca tggcctactc ccctgc (SEQ. ID. NO. 23) and variants thereof;
p 53 rtPCR primer 2: ttc tttggtgacc tacctcttcg gaattgccga gtc (SEQ. ID. NO. 24) and variants thereof.

The location of the primer extension on the P53 cDNA using primer 1 and primer 2 may be depicted as follows:

atggaggagccgcagtcagatcctagcgtcgagccccctctgagtcaggaaacattttca
gacctatggaaactacttcctgaaaacaacgttctgtcccccttgccgtcccaagcaatg
gatgatttgatgctgtccccggacgatattgaacaatggttcactgaagacccaggtcca
gatgaagctcccagaatgccagaggctgctccccgcgtggcccctgcaccagcagctcct
acaccggcggcccctgcaccagccccctcctggcccctgtcatcttctgtcccttcccag
aaaacctaccagggcagctacggtttccgtctgggcttcttgcattctgggacagccaa

gtctgccacca tggcctactc ccctgc →

(primer 1)

gtctgtgacttgcacgtactcccctgccctcaacaagatgttttgccaactggccaagacc
tgccctgtgcagctgtgggttgattccacacccccgcccggcacccgcgtccgcgccatg
gccatctacaagcagtcacagcacatgacggaggttgtgaggcgctgcccccaccatgag
cgctgctcagatagcgatggtctggcccctcctcagcgtcttatccgagtggaaggaaat
ttgcgtgtggagtatttggatgacagaaacacttttcgacatagtgtggtggtgccctat
gagccgcctgaggttggctctgactgtaccaccatccactacaactacatgtgtaacagt
tcctgcatgggcggcatgaaccggaggcccatcctcaccatcatcacactggaagactcc
agtggtaatctactgggacggaacagctttgaggtgcgtgtttgtgcctgtcctgggaga
gaccggcgcacagaggaagagaatctccgcaagaaaggggagcctcaccacgagctgccc
ccagggagcactaagcgagcactgcccaacaacaccagctcctctccccagccaaag
aagaaaccactggatggagaatatttcacccttcagatccgtgggcgtgagcgcttcgagatg

←ttctttggtgacc tacctcttcg gaattgccga gtc

(primer 2)

ttccgagagc tgaatgaggc cttggaactc aaggatgccc aggctgggaa ggagccaggg
gggagcaggg ctcactccag ccacctgaag tccaaaaagg gtcagtctac ctcccgccat
aaaaaactca tgttcaagac agaagggcct gactcagac (SEQ. ID. NO. 25)

Primers from cDNA of ras, RB1, BRCA-1, and TRE17 which incorporate the underlined mutated sequences as shown above for the p53 primers allow the target sequences to be ligated into the polynucleotide vector.

The amplified fragments is digested with the restriction endonuclease Bgl I and ligated, using T4 DNA ligase, into a similarly digested pITL vector which has been dephosphorylated with calf alkaline phosphatase. Ligation products is electroporated into DH10/p3 bacteria and selected on LB ampicillin and tetracycline plates. Ten to twenty individual colonies from each ligation/transfection are randomly selected and subjected to PCR-based sequencing. The nucleic acid sequences from clones positive for mutations are used in preparing a polynucleotide vector vaccine. In one embodiment, a mutation from germline is considered a true mutation and selected for use if present in 20% or greater of the colonies evaluated. If two or more mutations meet the above criteria all clones considered valid is used in the polynucleotide vaccine preparation. Individual clones are isolated from the strain DH10/p3 of *E. coli* using standard molecular biological techniques.

The plasmid of interest is separated from the p3 helper plasmid, required for selection, via ion exchange chromatography using the DEAE 4000-7 resin of the Nest Group Inc. An aliquot of each component of the vaccine preparation is analyzed by agarose gel electrophoresis and ethidium bromide visualization to assure purity of DNA. An aliquot of these large scale preparations is evaluated for nucleotide sequence to confirm the inserted target sequence. Individual aliquots of plasmid preparations is stored at -20°C as an ethanol precipitate (under 70% ethanol in water). Prior to use, these precipitates are resuspended at a concentration of 1 milligram per milliliter (mg/ml) in sterile, injection grade, normal saline. Appropriate volumes are diluted as required to match the individual and total dose parameters of each cohort.

All patients receive intramuscular (IM) injections (vaccine or control). The vaccine consist of individual polynucleotide vaccine construct mixtures at the appropriate dose level per cohort, resuspended in 0.5 ml injection grade, sterile, normal saline and administered in the lateral quadriceps with the patient in the supine position. Cohort #1 receives three initial IM injections of normal saline control (no DNA) prior to receiving three polynucleotide vaccinations.

All intramuscular injections are preceded by IM injection of 0.5 ml. 0.5 % Bupivacaine 24 hours before vaccine administration. The injection site are marked with a sterile indian ink pinpoint tatoo at the time of the bupivacaine HCl administration.

Cohort #1

Patients receive IM bupivacaine HCl followed by IM injection of sterile saline x 3 at three week intervals.

Subsequently, patients receive full compliment polynucleotide vaccination, a total dose of 1 microgram of DNA per construct per vaccination with a total dose of DNA not to exceed 5 micrograms per injection, is administered in the lower extremity at three week intervals x 3.

Cohort #2

The initial vaccination consists of 10 micrograms of the control plasmid, pITL without any PCR amplified sequences.

Full compliment polynucleotide vaccination, a total dose of 10 micrograms of DNA per construct per vaccination with a total dose of DNA not to exceed 50 micrograms per injection, is administered in the contralateral lower extremity at three week intervals x 3.

The first three patients have a muscle biopsy of the control plasmid injection site at week three. The second three patients have a muscle biopsy of the control plasmid injection site at week 6. The final four patients have a muscle biopsy of the control plasmid injection site at week 9.

Cohort #3

The initial vaccination consists of 10 micrograms of the reporter plasmid, pITL with the PCR amplified GFP sequence.

Full compliment polynucleotide vaccination, a total dose of 25 micrograms of DNA per construct per vaccination with a total dose of DNA not to exceed 125 micrograms per injection, will be administered in the contralateral lower extremity at three week intervals x 3.

The first three patients have a muscle biopsy of the control plasmid injection site at week three. The second three patients have a muscle biopsy of the control plasmid injection site at week 6. The final four patients have a muscle biopsy of the control plasmid injection site at week 9.

Cohort #4

Full compliment polynucleotide vaccination, at a total dose of 100 micrograms of DNA per construct per vaccination with the total dose of DNA per injection not to exceed 500 micrograms, is administered in one lower extremity at three week intervals x 4.

All patients are evaluated for anti-tumor responses at the conclusion of the vaccination sequence, every two months x 3, then every three months. Patients free from progression at 6 months are eligible for repeated vaccination sequences identical to that initially administered.

Freshly excised tumor is used not only to generate individual patient polynucleotide vaccine preparation but, also for the establishment of tumor cell lines using previously described techniques (173-175) and cryopreserved to evaluate tumor specific CTLp. Normal skin fibroblasts or normal autologous breast epithelium derived from the excisional biopsy is used in transfection studies for analysis of immune responses to individual vaccine components. In the absence of established cultures of autologous fibroblasts or breast epithelium, autologous PBMC is used in the same fashion although, transfection procedures require non-specific activation and will be adjusted accordingly.

Patients of cohort #1 have leukapheresis performed pre-treatment, after the first three injections (just prior to first polynucleotide vaccination), at the first post treatment follow-up visit, and at the 6 month time point. Patients of cohorts #2, #3 and #4 have leukopharesis performed pre-treatment, at the first post treatment follow-up visit, and at the 6 month time point. Manual leukapheresis of not less than 100 cc is performed for the purpose of evaluation of tumor-specific CTLp and peripheral blood mononuclear cell phenotype at one year after initiating treatment in all surviving patients.

T Lymphocyte Function

The parameters monitored are as follows:

1. z chain, lck, and fyn proteins by western blot,
2. nuclear binding factors for γIFN and NFkB probe sequences,
3. Lymphokine production (IL-2, IL-4, IL-6, IL-10, and γIFN), and
4. Tumor specific CTL precursors (evaluated by limiting dilution assay)

Fluorescent Activated Cell Sorting (FACS)

The phenotype of peripheral blood mononuclear cells is evaluated by flow cytometry from pretreatment, pre-third vaccination, first post-vaccination follow-up visit, and 6 month samples. The cell markers to be determined are; CD3, CD4, CD8, CD16, CD25, CD56, TCR α/β, TCR γ/δ, CD45 RO, CD45 RA, CD20.

Antigen Specific Responses

Non-malignant autologous cell cultures, either fibroblast or breast epithelium depending upon materials obtained from the excisional biopsy, is transiently transfected with a mixture of pITL-GFP and an individual pITL-target sequence construct. Either lipofectin or other established transfection procedures (176) are used. A control transfection of pITL-GFP is utilized for background, non-target sequence, immune responses while the mixture assures that transfection efficiency can be controlled for.

Standard proliferation assays using $^3$H-thymidine incorporation (177) are performed using PBMC from individual time points. PBMC is placed into culture with irradiated (10-20cGy) transfected cells as stimulators and after 48, 3H-thymidine is added for a 16 hour pulse. Cells is harvested and counted in the standard fashion.

The frequency of CTL precursors (CTLp) for any individual component is expected to be on the order of 1/10-6 (178) and therefore to assess changes in the CTL compartment isolated T cells from PBMC preparations are non-specifically stimulated with OKT3 and provide IL-2 for one week. At the conclusion of this period the bulk culture are divided and assayed for cytotoxicity on the individually transfected autologous cells noted above along with K562 as a control for NK activity.

Tumor specific CTL precursors are analyzed using irradiated (10-20cGy) autologous tumor cells as stimulators in limiting dilution cultures of PBMC from individual time points, using a previously established technique (178). Standard cytotoxicity assays again using autologous tumor cells and K562 as target cells, are scored and tumor specific CTLp frequency calculated.

Muscle biopsies is performed as out patient minor surgery procedures. The tissue samples is multiply divided cross sectionally and separated in an alternating fashion into two groups; the first for standard formalin fixation with H&E histologic examination along with fluorescent microscopy on unstained sections for detection of GFP, the second for frozen section examination and use for immunohistochemical staining. Standard post biopsy care with adequate analgesia and wound care is administered.

CRITERIA FOR RESPONSE

Complete Response

Disappearance of all clinical and laboratory signs and symptoms of active disease for a minimum period of four weeks. Persistently abnormal bone scans, if stable for a period of not less than 8 weeks is allowed.

Partial Response

A minimum of 50% reduction in the sum of the bidimensional product of measurable lesions persisting for a minimum period of four weeks. Although every lesion need not demonstrate a full 50% reduction in bidimensional product, no lesion may grow nor can there be new lesions demonstrated in this category of response.

Stable Disease

No change or decrease in the sum of the bidimensional product less than 50% persisting for a minimum period of four weeks. No lesion may grow and no new lesions can appear in this category of response.

Progressive Disease

25% increase in the sum of the bidimensional product or the appearance of new lesions.

TOXICITY

Toxicities is graded according to the NCI Common Toxicity Criteria.

DOSE ADJUSTMENT AND MODIFICATIONS

The maximally tolerated dose (MTD) of total DNA per vaccination is defined as that dose at which 0 or 1/6 patients experience polynucleotide vaccine related dose limiting toxicity (DLT) with the next higher dose level provoking DLT in any 2 patients from that cohort.

If dose limiting toxicity is observed in the first two patients of any cohort, the dose is reduced by 50% and the equivalent of a full cohort is enrolled at this reduced dose unless dose limiting toxicity is again encountered at this new dose.

If an individual component at a given dose is suspected to be responsible for undue toxicity due to its association with toxicities in separate patients, it is decreased to the previously tolerated dose or dropped from the vaccine preparation if no tolerated dose is previously documented.

If the patient has sufficient potential tumor antigenic templates identified so as to exceed the total dose limitations, if all constructs were to be used, the constructs included in the vaccine preparation is prioritized as follows: 1.) constructs expressing mutated sequences isolated from the patient's tumor, 2.) sequences for Int-2 and Hst and 3.) muc-1 and c-erbB-2/HER2/neu.

REMOVAL OF PATIENTS FROM PROTOCOL

A. All three of the following, which preclude polynucleotide vaccine production:

1.) No detectable genetic deviation from germline in p53, H or K ras, RB, BRCA-1, TRE2/TRE17,
2.) Absence of overexpression of c-erbB-2HER 2/neu
3.) No expression of MUC-1

B. Progressive disease
C. Unacceptable toxicity
D. Intercurrent Illness which prevents further administration of the treatment
E. Decision of the patient to withdraw from the study
F. General or specific changes in the patient's condition which render the patient unacceptable for further treatment in the judgement of the investigator.

**EXAMPLE 10**

**Construction of pITL-1**

In this embodiment, the polynucleotide vector comprises a 440 base pair region from the RANTES promoter in combination with a 635 base pair region of colE1 origin of replication. The polynucleotide vector was constructed as follows:

(A) Splice & poly A sequence was synthesized and inserted into the vector pGEM between the Hind III and Xho I site forming pGEM ELN.
(B) The stuffer region was isolated from pCDM8 by PCR amplification of an approximately 200 base pair portion and blunt end ligated into Xho I site of pGEM ELN forming pGEM ELN/Stuffer.
(C) A 440 base pair region of the RANTES promoter was excised from pGL RANTES using Xho I and Nco I. This fragment is klenow filled (polished) and blunt end ligated into pGEM ELN at the Hind III and Nsi site forming pGEM ELN/RANTES.
(D) The 440 base pair region of the RANTES promoter was next excised from pGEM ELN/RANTES using Nsi and Hind III and Inserted into pGEM ELN/Stuffer at its analogous site forming pGEM/ELN/Stuffer/RANTES.

(E) A 635 base pair region of the colEI origin of replication was excised from pBR327 using Bst YI and Ava I. This fragment was polished and blunt end ligated into pBluescript/Sup F forming pBluescript/Sup F/colE1.

(F) Sup F was excised from πVX using Eco RI and ligated into the Eco RI site of pBluescript forming pBluescript/ Sup F.

(G) The Sup F and colEI region was removed from pBluescript/ Sup F/colE1 using Hind III and Bam HI and inserted into pGEM/ ELN/Stuffer/RANTES at its analogous site forming pGEM/pITL-1.

(H) pITL-1 is separated from pGEM by excising with Kpn I. pITL-1 is then religated.

The resulting vector is designated herein as pITL-1. The pITL-1 vector contains the fragment of the RANTES promoter that corresponds with the region in genomic RANTES promoter from about NCO site through the KpnI site. The plasmid polynucleotide vector in *Escherichia Coli* strain DH 10β/P3 was deposited under terms of the Budapest treaty with the American Type Culture Collection, 12301 Parklawn Drive, Rockville MD 20852 USA on April 9, 1997 under ATCC Designation 98400.

The computer generated sequence which approximates the sequence of pITL-1 comprises the sequence:

GgtacctgccaccaTGGCGCGGATTCTTTATCACTGATAAGTTGGTGGACATATT
ATGTTTATCAGTGATAAAGTGTCAAGCATGACAAAGTTGCAGCCGAATAC
AGTGATCCGTGCCGGCCCTGGACTGTTGAACGAGGTCGGCGTAGACGGTC
TGACGACACGCAAACTGGCGGAACGGTTGGGGGTGCAGCAGCCGGCGCT
TTACTGGCACTTCAGGAACAAGCGGGCGCcttaagggccaTATGGTGAGTGGAT
GCCTTGACCCCAGGCGGGGATGGGGGAGACCTGTAGTCAGAGCCCCCGG
GCAGCACAGGCCAATGCCCGTCCTTCCCCTGCAGGATgAGTagtgagtgcctctcct
gGCCCtGGaagttgccactccagtgCCCaccagccttgtcctaataaaattaagttgcatcattttgtctgactaggtgt
cctctataatattataagcttgatatcGAATTCTTTCGGACTTTTGAAAGTGATGGTGGTGG
GGGAAGGATTCGAACCTTCGAAGTCGATGACGGCAGATTTAGAGTCTGCT
CCCTTTGGCCGCTCGGGAACCCCACCACGGGTAATGCTTTTACTGGCCTG
CTCCCTTATCGGGAAGCGGGGCGCATCATATCAAATGACGCGCCGCTGTA
AAGTGTTACGTTGAGAAAGAATTCctgcagcccGCCGCGTTGCTGGCGTTTTTC
CATAGGCTCCGCCCCCCTGACGAGCATCACAAAAATCGACGCTCAAGTCA
GAGGTGGCGAAACCCGACAGGACTATAAAGATACCAGGCGTTTCCCCCTG
GAAGCTCCCTCGTGCGCTCTCCTGTTCCGACCCTGCCGCTTACCGGATAC
CTGTCCGCCTTTCTCCCTTCGGGAAGCGTGGCGCTTTCTCAATGCTCACGC
TGTAGGTATCTCAGTTCGGTGTAGGTCGTTCGCTCCAAGCTGGGCTGTGTG
CACGAACCCCCCGTTCAGCCCGACCGCTGCGCCTTATCCGGTAACTATCG
TCTTGAGTCCAACCCGGTAAGACACGACTTATCGCCACTGGCAGCAGCCA
CTGGTAACAGGATTAGCAGAGCGAGGTATGTAGGCGGTGCTACAGAGTTC
TTGAAGTGGTGGCCTAACTACGGCTACACTAGAAGGACAGTATTTGGTAT
CTGCGCTCTGCTGAAGCCAGTTACCTTCGGAAAAAGAGTTGGTAGCTCTT
GATCCGGCAAACAAACCACCGCTGGTAGCGGTGGTTTTTTTGTTTGCAAG
CAGCAGATTACGCGCAGAAAAAAAGGATCTgggggatccggagagctcccaacgcgttgg
atgcatggatgagggaaaggaggtaagatctgtaatgaataagcaggaactttgaagactcagtgactcagtgagtaataaa
gactcagtgacttctgatcctgtcctaactgccactccttgttgtcccaagaaagcggcttcctgctctctgaggaggaccccctt
ccctggaaggtaaaactaaggatgtcagcagagaaatttttccaccattggtgcttggtcaaagaggaaactgatGAGC
TCACTCTAGATGAGAGAGCAGTGAGGGAGAGACAGAGACTCGAATTTCC
GGAGCTATTTCAGTTTTCTTTTCCGTTTTGTGCAATTTCACTTATGATACC
GGCCAATGCTTGGTTGCTATTTTGGAAACTCCCCTTAGGGGATGCCCCTC
AACTGGCCCTATAAAGGGCCAGCCTGAGCTGCAGAGGATTCCTGCAGAGG
ATCAAGACAGCACGTGGACCTCGCACAGCCTCTCCCACA (SEQ ID NO. 28)

It can be appreciated by those in the art that the actual nucleic acid sequence of the ATCC deposited plasmid may vary slightly from the computer generated sequence due to slight variations in the ligation sites of the various components that comprise the vector. Such variations will not affect the functioning of the vector. The actual sequence of the deposited vector including the ligation sites may be performed using routine sequencing methods as are known in the art.

A smaller polynucleotide vector was constructed, differing from pITL-1 by about 200 base pairs, was constructed as described above with the exception that a base pair region of approximately 220 base pairs of the RANTES promoter was used. The resulting vector is referred to herein as pITL-A. This smaller vector in *E. coli strain* DH-10β/P3 was deposited under terms of the Budapest treaty with the ATCC on April 9, 1997 under ATCC designation 98401. The computer generated sequence which approximates the sequence of pITL-A comprises the sequence:

GgtacctgccaccaTGGCGCGGATTCTTTATCACTGATAAGTTGGTGGACATATT
ATGTTTATCAGTGATAAAGTGTCAAGCATGACAAAGTTGCAGCCGAATAC
AGTGATCCGTGCCGGCCCTGGACTGTTGAACGAGGTCGGCGTAGACGGTC
TGACGACACGCAAACTGGCGGAA CGGTTGGGGGTGCAGCAGCCGGCGCT
TTACTGGCACTTCAGGAACAAGCGGGCGCcttaagggccaTATGGTGAGTGGAT
GCCTTGACCCCAGGCGGGG ATGGGGGAGACCTGTAGTCAGAGCCCCCGG
GCAGCACAGGCCAATGCCCGTCCTTCCCCTGCAGGATgAGTagtgagtgcctctcct
gGCCCtGGaagttgccactccagtgCCCaccagccttgtcctaataaaattaagttgcatcattttgtctgactaggtgt
cctctataatattataagcttgatatcGAATTCTTTCGGACTTTTGAAAGTGATGGTGGTGG
GGGAAGGATTCGAACCTTCGAAGTCGATGACGGCAGATTTAGAGTCTGCT
CCCTTTGGCCGCTCGGGAACCCC ACCACGGGTAATGCTTTTACTGGCCTG
CTCCCTTATCGGGAAGCGGGGCGCATCATATCAAATGACGCGCCGCTGTA
AAGTGTTACGTTGAGAAAGAATTCctgcagcccGCCGCGTTGCTGGCGTTTTTC
CATAGGCTCCGCCCCCCTGACGAGCATCACAAAAATCGACGCTCAAGTCA
GAGGTGGCGAAACCCGACAGGACTATAAAGATACCAGGCGTTTCCCCCTG
GAAGCTCCCTCGTGCGCTCTCCTGTTCCGACCCTGCCGCTTACCGGATAC
CTGTCCGCCTTTCTCCCTTCGGGAAGCGTGGCGCTTTCTCAATGCTCACGC
TGTAGGTATCTCAGTTCGGTGTAGGTCGTTCGCTCCAAGCTGGGCTGTGTG
CACGAACCCCCCGTTCAGCCCGACCGCTGCGCCTTATCCGGTAACTATCG
TCTTGAGTCCAACCCGGTAAGACACGACTTATCGCCACTGGCAGCAGCCA
CTGGTAACAGGATTAGCAGAGCGAGGTATGTAGGCGGTGCTACAGAGTTC
TTGAAGTGGTGGCCTAACTACGGCTACACTAGAAGGACAGTATTTGGTAT
CTGCGCTCTGCTGAAGCCAGTTACCTTCGGAAAAAGAGTTGGTAGCTCTT
GATCCGGCAAACAAACCACCGCTGGTAGCGGTGGTTTTTTTGTTTGCAAG
CAGCAGATTACGCGCAGAAAAAAAGGATCTggggatccggagagctcACTCTAGA
TGAGAGAGCAGTGAGGGAGAGACAGAGACTCGAATTTCCGGAGCTATTTC
AGTTTTCTTTTCCGTTTTGTGCA ATTTCACTTATGATACCGGCCAATGCTT

GGTTGCTATTTTGGAAACTCCCCTTAGGGGATGCCCCTCAACTGGCCCTAT
AAAGGGCCAGCCTGAGCTGCAGAGG ATTCCTGCAGAGGATCAAGACAGC
ACGTGGACCTCGCACAGCCTCTCCCACA (SEQ ID NO. 27)

**EXAMPLE 11**

**pITL-1-GFP**

The target reporter gene encoding green fluorescent protein was inserted into the above pITL-1 polynucleotide vector forming the vector, pITL-1GFP. The computer generated sequence which approximates the sequence of pITL-1 GFP comprises:

GgtacctgccaccaTGGCGAAGGGCGAGGAACTGTTCACTGGCGTGGTCCCAATT
CTCGTGGAACTGGATGGCGATGTGAATGGGCACAAATTTTCTGTCAGCGG
AGAGGGTGAAGGTGATGCCACATACGGAAAGCTCACCCTGAAATTCATCT
GCACCACTGGAAAGCTCCCTGTGCCATGGCCAACACTGGTCACTACCTTC
ACCTATGGCGTGCAGTGCTTTTCCAGATACCCAGACCATATGAAGCAGCA
TGACTTTTTCAAGAGCGCCATGCCCGAGGGCTATGTGCAGGAGAGAACCA
TCTTTTTCAAAGATGACGGGAACTACAAGACCCGCGCTGAAGTCAAGTTC
GAAGGTGACACCCTGGTGAATAGAATCGAGTTGAAGGGCATTGACTTTAA
GGAAGATGGAAACATTCTCGGCCACAAGCTGGAATACAACTATAACTCCC
ACAATGTGTACATCATGGCCGACAAGCAAAAGAATGGCATCAAGGTCAA
CTTCAAGATCAGACACAACATTGAGGATGGATCCGTGCAGCTGGCCGACC
ATTATCAACAGAACACTCCAATCGGCGACGGCCCTGTGCTCCTCCCAGAC
AACCATTACCTGTCCACCCAGTCTGCCCTGTCTAAAGATCCCAACGAAAA
GAGAGACCACATGGTCCTGCTGGAGTTTGTGACCGCTGCTGGGATCACAC
ATGGCATGGACGAGCTGTACAAGTGAGCGCcttaagggccaTATGGTGAGTGGA
TGCCTTGACCCCAGGCGGGGATGGGGGAGACCTGTAGTCAGAGCCCCCGG
GCAGCACAGGCCAATGCCCGTCCTTCCCCTGCAGGATgAGTagtgagtgcctctcct
gGCCCtGGaagttgccactccagtgCCCaccagccttgtcctaataaaattaagttgcatcattttgtctgactaggtgt
cctctataatattataagcttgatatcGAATTCTTTCGGACTTTTGAAAGTGATGGTGGTGG
GGGAAGGATTCGAACCTTCGAAGTCGATGACGGCAGATTTAGAGTCTGCT
CCCTTTGGCCGCTCGGGAACCCCACCACGGGTAATGCTTTTACTGGCCTG
CTCCCTTATCGGGAAGCGGGGCGCATCATATCAAATGACGCGCCGCTGTA
AAGTGTTACGTTGAGAAAGAATTCctgcagcccGCCGCGTTGCTGGCGTTTTTC
CATAGGCTCCGCCCCCCTGACGAGCATCACAAAAATCGACGCTCAAGTCA
GAGGTGGCGAAACCCGACAGGACTATAAAGATACCAGGCGTTTCCCCCTG
GAAGCTCCCTCGTGCGCTCTCCTGTTCCGACCCTGCCGCTTACCGGATAC
CTGTCCGCCTTTCTCCCTTCGGGAAGCGTGGCGCTTTCTCAATGCTCACGC
TGTAGGTATCTCAGTTCGGTGTAGGTCGTTCGCTCCAAGCTGGGCTGTGTG
CACGAACCCCCCGTTCAGCCCGACCGCTGCGCCTTATCCGGTAACTATCG
TCTTGAGTCCAACCCGGTAAGACACGACTTATCGCCACTGGCAGCAGCCA
CTGGTAACAGGATTAGCAGAGCGAGGTATGTAGGCGGTGCTACAGAGTTC
TTGAAGTGGTGGCCTAACTACGGCTACACTAGAAGGACAGTATTTGGTAT

CTGCGCTCTGCTGAAGCCAGTTACCTTCGGAAAAAGAGTTGGTAGCTCTT
GATCCGGCAAACAAACCACCGCTGGTAGCGGTGGTTTTTTTGTTTGCAAG
CAGCAGATTACGCGCAGAAAAAAAGGATCTggggatccggagagctcccaacgcgttgg
atgcatggatgagggaaaggaggtaagatctgtaatgaataagcaggaactttgaagactcagtgactcagtgagtaataaa
gactcagtgacttctgatcctgtcctaactgccactccttgttgtcccaagaaagcggcttcctgctctctgaggaggaccccttt
ccctggaaggtaaaactaaggatgtcagcagagaaatttttccaccattggtgcttggtcaaagaggaaactgatGAGC
TCACTCTAGATGAGAGAGCAGTGAGGGAGAGACAGAGACTCGAATTTCC
GGAGCTATTTCAGTTTTCTTTTCCGTTTTGTGCAATTTCACTTATGATACC
GGCCAATGCTTGGTTGCTATTTTGGAAACTCCCCTTAGGGGATGCCCCTC
AACTGGCCCTATAAAGGGCCAGCCTGAGCTGCAGAGGATTCCTGCAGAGG
ATCAAGACAGCACGTGGACCTCGCACAGCCTCTCCCACA (SEQ ID NO. 29)

The pITL-1-GFP vector was tested for functional expression in primary human skeletal muscle cells. The vector pITL-GFP expressed the reporter protein, GFP in the human skeletal muscle cells in vitro.

REFERENCES

1. Wingo PA, Tong T, Bolden S. Cancer Statistics, 1995. CA Cancer J Clin 1994; 45:8-30.

2. Harris JR, Marrow M, Bonadonna G. Cancer of the Breast in CANCER: Principles and Practice of Oncology, 4th Edition, Devita VT, Hellman S, Rosenberg SA, editors. 1993 J.B. Lippincott Co. Philadelphia, PA.

3. Bishop JM. Molecular themes in Oncogenesis. Cell 1991; 64:235-48.

4. Hunter T. Cooperation between Oncogenies. Cell 1991; 64:249-70.

5. Marshall CJ. Tumor suppressor genes. Cell 1991; 64:313-26.

6. Weinberg RA. Tumor suppressor genes. Science 1991; 254-1138-46.

7. Anderson MW, Reynolds SH, You M, Maronpot RM. Role of proto-oncogene activation in carcinogenesis. 1992, Environ Health Perspect. 98:13-24.

8. Singluff CL, Hunt DF, Engelhard VH, Direct analysis of tumor associated peptide antigens. Current Opinion in Immunology, 1994 Oct; 6(5):733-40.

9. Toes REM, Offringa R, Feltkamp MCW, Visseren MJW, Schoenberger SP, Melief CJM, Kast WM. Tumor rejection antigens and tumor specific cytotoxic T lymphocytes. Behring Inst Mitt. 1994; 94:72-86.

10. Grizzle WE, Meyers RB, Arnold MM, and Srivastava S. Evaluation of Biomarkers in Breast and Prostate Cancer. J Cell Biochem 1994; S19:259-66.

11. Barnes DM, Dublin EA, Fisher CJ, Levinson DA, Millis RR. Immunohistochemical detection of p53 protein in mammary carcinoma:An important new independent indicator of prognosis? Hum Pathol 1993; 24:469-76

12. Thor AD, Moore DH, Edgerton SM, et al. Accumulation of p53 suppressor gene protein ; An independent marker of prognosis in breast cancers. J Natl Cancer Inst 1992; 84:845-55.

13. Cattoretti, Rilke F, Andreola S, D'amato L, Delia D. p53 Expression in breast cancer. Int J Cancer 1988; 41: 178-183.

14. Horak E, Smith K, Bromley L, et al. Mutant p53, EGF receptor, and c-erbB-2 expression in human breast cancer. Oncogene 1991; 6:1699-1703.

15. Hollestein M, Sidransky D, Vogelstein B, Harris CC. p53 mutations in human cancers Science 1991; 253:49-53.

16. Tominaga O, Hamelin R, Remvikos Y, Salmon RJ, Thomas G. p53 from basic research clinical applications. Critical Rev Oncog 1992; 3:257-282.

17. Zambetti GP, Levine AJ. A Comparison of the biological activities of wild type and mutant p53. FASEB J. July 1993; 7:855-65

18. Greenblatt MS, Bennett WP, Hollstein M, Harris CC. Mutations in the p53 tumor suppressor gene: clues to cancer etiology and molecular pathogenesis. Cancer Res. 1994; 54:4855-78.

19. Milner J. DNA damage, p53 and anticancer therapies. Nature Med 1995; 9(1):879-80.

20. Montenarh M. Biochemical, immunological, and functional aspects of the growth-suppressor/oncoprotein p53. Crit Rev Oncog 1992; 3:233-56.

21. Downward J, Yarden Y, Mayes E, et al. Close similarity of epidermal growth factor receptor and v-erb B oncogene protein sequences. Nature 1984; 307:521-27.

22. Bargmann CI, Hung MC, Weinberg RA. The neu oncogene encodes an epidermal growth factor receptor-related protein. Nature 1986; 319:226-230.

23. Yamamoto T, Ikawa S, Akiyama T, et al. Similarity of protein encoded by the humanc-erb-B2 gene to epidermal growth factor receptor. Nature 1986; 3139:230-34.

24. Schechter AL, Hung MC, Vaidyanathan L, et al. The neu gene: An erbB-homologous gene distinct from and unlinked to the gene encoding the EGF receptor. Science 1985; 229:976-78.

25. Gullick WJ. The role of the epidermal growth factor receptor and the c-erbB-2 protein in breast cancer. Int J Cancer 1990 ; 5:55-61.

26. Nagayama K, Watatani M. Analysis of genetic alterations related to the development and progression of breast carcinoma. Jpn J Cancer Res 1993; 84:1159-64.

27. Klijn JGM, Berns EMJJ, Foekens JA. Prognostic factors and response to therapy in breast cancer. Cancer Surveys 1993; 18:165-198.

28. Leslie KO, Howard P. Oncogenies and antioncogenes in human breast carcinoma. Pathol Annu 1992; 27(1): 321-42.

29. Lee EY, Bookstein R, Lee WH. Role of the retinoblastoma gene in the oncogenesis of breast carcinoma. Cancer Treat Res 1991; 53:23-44.

30. Langlade-Demoyen P, Levraud JP, Kourilsky P, Abastado JP. Primary Cytotoxic T lymphocyte induction using peptide-stripped autologous cells. International Immunology 1994 Nov; 6(11):1759-66.

31. Clair T, Miller W, Cho-Chung Y. Prognostic significance of the expression of the ras protein with a molecular weight of 21,000 by human breast cancer. Cancer Res 1987; 47(20):5290-93.

32. Theillet C, Lidereau R, Escot C, et al. Loss of a c-H-ras-1 allele and aggressive human promary breast carcinomas Cancer Res 1986; 46(9):4776-81.

33. Prosperi M, Dupre' G, Lidereau R, Point mutation at codon 12 of the ki-ras gene in a primary breast carcinoma and the MDA-MB-134 human mammary carcinoma cell line. Cancer Let 1990; 51(2):169-74.

34. Burchell J, Graham R, Taylor-Papadimitriou J. Active Specific immunotherapy: PEM as a potential target molecule. Cancer Survey 1993; 18:135-47.

35. Taylor Papadimitriou J, Stewart L, Burchell J, Beverly P, The polymorphic epithelial mucin as a target for immunotherapy. Cancer Vaccines 1993; 3(1327):73-93.

36. Siddiqui J, Abe M, Hayes D, Shani E, Yunis E, Kufe D. Isolation and sequencing of a cDNA coding for the human DF3 breast carcinoma-associated antigen. Proc Natl Aced Sci USA 1988; 85:2320-23.

37. Girling A, Bartkova J, Burchell J, Gendler S, Gillet C, Taylor-Papadimitriou J. A core protein epitope of the polymorphic epithelial mucin detected by the monoclonal antibody SM3 is selectively exposed in a range of primary carcinomas. Int J Cancer 1989; 43:1072-76.

38. Miki Y, Swensen J, Shattuck-Eidens D, et al. A strong candidate for the breast and ovarian cancer susceptibility gene BRCA1. Science 1994; 266:66-71.

39. Struewing JP, Brody LC, Erdos MR et al. Detection of eight BRCA1 mutations in 10 breast/ovarian cancer families, including 1 family with male breast cancer. Am J Hum Genet 1995; 57:1-7.

40. Shattuck-Eidens D, McClure M, Simard J, et al. A collaborative survey of 80 mutations in the BRCA1 breast and ovarian cancer susceptibility gene. Implications for presymptomatic testing and screening. JAMA 1995; 273 (7):535-41.

41. Castilla LH, Couch FJ, Erdos MR, et al. Mutations in the BRCA1 gene in families with early-onset breast and ovarian cancer. Nature Genetics 1994; 8:387-391.

42. Simard J, Tonin P, Durocher F, et al. Common origins of BRCA1 mutations in Canadian breast and ovarian cancer families. Nature Genetics 1994; 8:392-98.

43. Friedman LS, Ostermeyer EA, Szabo CI, et al. Confirmation of BRCAI by analysis of germline mutations linked to breast and ovarian cancer in ten families. Nature Genetics 1994; 8:300-4404.

44. Yoshida T, Miyagawa K, Odagiri H, Sakamoto H, Little PFR, Terada M, Sugimura T. Genomic sequence of hst, a transforming gene encoding a protein homologous to fibroblast growth factors and the int-2 encoded protein. Proc Natl Acad Sci USA 1987; 84:7305-09.

45. Liscia DS, Merlo G, Garrett C, French D, Mariani-Constantini R, Callahan R. Expression of int-2 mRNA in human tumors amplified at the int-2 locus. Oncogene 1989; 4:1219-24.

46. Tsuda H, Hirohashi S, Shimosato Y, et al. Correlation between long term survival in breast cancer patients and amplification of two putative oncogene-coamplification units: hst-1/int-2 and c-erbB-2/ear-1. Cancer Res 1989; 49 (11):3104-8.

47. Fantl V, Richards M, Smith R, et al. Gene amplification an chromosome band 11q13 and oestrogen receptor status in breast cancer. Eur J Cancer 1990; 26(4):423-29.

48. van de Viver MJ, Peterse JL, Mooi WJ, et al. Oncogene activations in human breast cancer. Cancer Cells 1989; 7:385-391.

49. Callahan R, Crop CS, Merlo GR, Liscia DS, Cappa APM, Lidereau R. Somatic mutations in human breast cancer: a status report. Cancer 1992; 69:1582-88.

50. Dean JH, McCoy JL, Cannon GB, et al. Cell-mediated immune responses of breast cancer patients to autologous tumor associated antigens. J Nail Cancer Inst 1977 March; 58(3):549-55.

51. Davidoff AM, Iglehart JD, Marks JR, Immune response to p53 is dependent upon p53/HSP70 complexes in breast cancers. Proc Natl Acad Sci USA 1992; 89:3439-42.

52. Croce MV, Price MR, Segal-Eiras A. Expression of monoclonal antibody defined antigens in fraction isolated from human breast carcinoma and patient's serum. Cancer Immunol Immunother 1995; 40:132-37.

53. Yin L. Thomas C, Hsuan JJ, Stauss HJ. Unconventional cytotoxic T lymphocyte recognition of synthetic peptides corresponding to residues 1-23 of Ras protein. European J Immunol, 1994 Sep; 24(9):1988-92.

54. Heike M, Blachere NE, Srivastava PK. Protective cellular immunity against spontaneous mammary carcinoma from ras transgenic mice. Immunobiology, 1994 Jun; 190(4-5):411-23.

55. Peace DJ, Smith JW, Chen W, et al. Lysis of ras oncogene-transformed cells by specific cytotoxic T lymphocytes elicited by primary in vitro immunization with mutated ras peptide. J Exp Med 1994 Feb;179(2):473-79.

56. Peoples GE, Goedegeburre PS, Smith R, Linehan DC, Yoshino 1, Eberlein TJ. Breast and ovarian cancer specific cytotoxic T lymphocytes recognize the same HER2/neu-derived peptide. Proc Natl Acad Sci USA, 1995 Jan, 92:432-36.

57. Jung S, Schluesener HJ. Human T lymphocytes recognize a peptide of single point mutated oncogenic ras proteins. J Exp Med 1991; 173:273-76.

58. Disis ML, Smith JW, Murphy AE, Chen W, Cheever MA. In vitro generation of human cytolytic T-cells specific for peptides derived from the HER-2/neu protooncogene protein. Cancer Res 1994 Feb 15; 54(4):1071-6.

59. Jerome KR, Barnd KL, Bendt KM, et al. Cytotoxic T-lymphocytes derived from patients with breast adenocarcinoma recognize an epitope present on the protein core of a mucin molecule preferentially expressed by mallignant cells. Cancer Res 1991; 51:2908-16.

60. Takahashi T, Makiguchi Y, Hinoda Y, et al. Expression of MUC1 on myeloma cells and induction of HLA-unrestricted CTL against MUC 1 from a multiple myeloma patient. J Immunol 1994; 153:2102-09.

61. Barnd DL, Lan MS, Metzgar RS, Finn OJ. Specific, major histocompatibility complex-unrestricted recognition of tumor-associated mucins by human cytotoxic T cells. Proc Natl Acad Sci USA 1989; 86:7159-7164.

62. Apostopoulos V, Xing PX, McKenzie IFC. Murine immune response to cells transfected with human MUC1: immunization with cellular and synthetic antigens. Cancer Res 1994; 54:5186-93.

63. Burchell J, Graham R, Taylor-Papadimitriou J. Active Specific immunotherapy: PEM as a potential target molecule. Cancer Survey 1993; 18:135-47.

64. Bos J. ras oncogenies in human cancer: A review Cancer Res 1989; 49(17):4682-89.

65. Seeburg P, Colby W, Capon D, et al. Biological properties of human c-Ha-rasl genes mutated at codon 12. Nature 1984; 312:71-75.

66. Schubert EL, Hansen MF, Strong LC. The retinoblastoma gene and its significance. Ann Med 1994; 26(3): 177-84.

67. Castilla LH, Couch FJ, Erdos MR, Hoskins KF, Calzone K, Garber JE, Boyd J et al. Mutations in the BRCA1 gene in families with early onset breast and ovarian cancer. Nat Genet 1994 ; 8:387-91.

68. Friedman LS, Ostermeyer EA, Szabo CI, Dowd P, Lynch ED, Rowell SE, King M-C. Confirmation of BRCA1 by analysis of germline mutations linked to breast cancer and ovarian cancer in ten families. Nat Genet 1994; 8: 399-404.

69. Simard J, Tonin P, Durocher F, Morgan K, Rommens J, Gingras S, Samson C, et al. Common origins of BRCA1 mutations in Canadian breast and ovarian families. Nat Genet 1994; 8:392-98.

70. Shattuck-Eidens D, McClure M, Simard J, Labrie F, Narod S, Couch F, Hoskins K, et al. A collaborative survey of 80 mutations in the BRCA1 breast and ovarian cancer susceptibility gene: implications for presymptomatic testing and screening. JAMA 1995; 273-535-41.

71. Fenton RG, Taub DD, Kwak LW, Smith MR, Longo DL. Cytotoxic T-cell response and in vivo protection against tumor cells harboring activated ras proto-oncogenies. J Natl Cancer Inst 1993 Aug 18; 85(16):1294-1302.

72. Schlichtholtz B, Legros Y, Gillet D, et al. The immune response to p53 inbreast cancer patients is directed against immunodominant epitopes unrelated to the mutational hot spot. Cancer Res 1992; 52:6380-84.

73. Yanuck M, Carbone DP, Pendleton CD et al. A mutant p53 tumor suppressor protein is a target for peptide-induced CD8+ cytotoxic T-cells. Cancer Res 1993; 53:3257-61.

74. van der Bruggen P, Van den Eynde B. Molecular definition of tumor antigens recognized by T lymphocytes. Current Opinion in Immunology 1992; 4:608-12.

75. Callahan R, Salomon DS. Oncogenies, tumor suppressor genes, and growth factors in breast cancer: Novel targets for diagnosis, prognosis and therapy. Cancer Surveys 1993; 18:35-56.

76. Potter CR, van Doele S, vande Vijver MJ, et al The expression of the neu oncogene product in breast lesions and in normal and adult human tissues. Histopathology 1989; 15:351-62

77. Gullick WJ, Berger MS, Bennett PLP, Rothbard JB, Waterfield MD. Expression of the c-erbB-2 protein in normal and transformed cells. Int J Cancer 1987; 40:246-54.

78. Nusse R. The int genes in mammary tumorigenesis and in normal development. Trends in Genetics 1988; 4 (10):291-95.

79. Theillet C, Leroy X, Delapeyriere O, et al. Amplification of FGF-related genes in human tumors: possible involvement of hst in breast carcinomas. Oncogene 1989; 4(7):915-22.

80. Ono M, Nakamura T, Hillova J, Hill M. Human TRE17 oncogene is generated from a family of homologous polymorphic sequences by single-base changes. DNA Cell Biol 1993; 12(2):107-18.

81. Hamblin TJ, Abdul-Ahad AK, Gordon J, Stevenson FK, Stevenson GT. Preliminary experience in treating lymphocytic leukemia with antibody to immunoglobulin idiotypes on the cell surface. Br J Cancer 42(4):495, 1980.

82. Miller RA, Maloney DG, Warnke R, Levy R. Treatment of B cell lymphoma with monoclonal and anti-idiotype antibody. N Engl J Med 306(9):517, 1982.

83. Meeker TC, Lowder J, Maloney DG, Miller RA, Thielemans K, Warnke R, Levy R. A clinical trial of anti-idiotype therapy for B cell malignancy. Blood 65(6):1349, 1985.

84. Kaminski MS, Kiamura K, Maloney DG, Levy R. Idiotype Vaccination against B cell lymphomas. Inhibition of tumor immunity by free idiotype protein. J Immunol 138(4):1289, 1987.

85. Stevensen FK, Gordon J. Immunization with idiotypic immunoglobulin protects against development of B lymphocytic leukemia, but emerging tumor cells can evade antibody attack by modulation. J Immunol 130(2):970, 1983.

86. Levy R, Miller RA. Therapy of lymphoma directed at idiotypes. J Natl Cancer Inst, Monograph 10:61, 1990.

87. Kwak LW, Campbell MJ, Czerwinski DK, Hart S, Miller RS, Levy R. Induction of immune responses in patients with B-cell lymphoma against the surface immunoglobulin idiotype expressed by their tumors. N Engl J Med 327 (17):1209, 1992.

88. Hsu FJ, Kwak L, Campbell M, Liles T, Czerwinski D, Hart S, Syrengelas A, Miller R, Levy R. Clinical trials of idiotype specific vaccine in B-cell lymphomas. Ann NY Acad Sci 690:385, 1993.

89. Nelson EL, Li X, Hsu F, Kwak LW, Clayberger C, Levy R, Krensky AM. Tumor Specific, Cytotoxic T Lymphocyte Response after 'Idiotype' Vaccination for B cell, non-Hodgkin's Lymphoma. In Press Blood, July 15, 1996.

90. Dean JH, McCoy JL, Cannon GB, Leonard CM, Perlin E, Kreutner A, Oldham RK, Herberman RB. Cell-mediated immune responses of breast cancer patients to autologous tumor associated antigens. J Natl Cancer Inst 58(3): 549, 1977.

91. Mitchell MS, Harel W, Kan-Mitchell J,LeMay LG, Goedegebuure P, Huang XQ, Hofman F, Groshen S. Active specific immunotherapy of melanoma with allogeneic cell lysates: rationale, results, and possible mechanisms of action. Ann NY Acad Sci 690:153, 1993.

92. Celis E, Fikes J, Wentworth P, Sidney J, Southwood S, Maewal, A, Del Guerrico MF, Sette A, Livingston B. Identification of potential CTL epitopes of tumor-associated antigen MAGE-1 for five common HLA-A alleles. Molecular Immunology, 1994 Dec; 31(18):1423-30.

93. van der Bruggen P, Bastin J, Gajewski T, Coulie PG, Boel P, De Smet C, Traversari C, Townsend A, Boon T. A peptide encoded by human gene MAGE-3 and presented by HLA-A2 induces cytolytic T lymphocytes that recognize tumor cells expressing MAGE-3. European J Immunol, 1994 Dec; 24(12):3038-43.

94. Morioka N, Kikumoto Y, Hoon DS, Morton DL, Irie RF. A decapeptide (Gln-Asp-Leu-Thr-Met-Lys-Tyr-Gln-Ile-Phe) from human melanoma is recognized by CTL in melanoma patients. J Immunol, 1994 Dec 15; 153(12):5650-8.

95. Frassanito MA, Mayordomo JI, DeLeo RM, Storkus WJ, Lotze MT, DeLeo AB. Identification of meth A sarcoma-derived Class I major histocompatibility complex-associated peptides recognized by a specific CD8 cytotoxic T lymphocytes. Cancer Research, 1995 Jan; 55:124-28.

96. Rivoltini L, Kawakami Y, Sakaguchi K, Southwood S, Sette A, Robbins PF, Marincola FM, Salgaller ML, Yanelli JR, et al. Induction of tumor-reactive CTL from peripheral blood and tumor-infiltrating lymphocytes of melanoma patients by in vitro stimulation with a immunodominant peptide of the human melanoma antigen MART-1. J Immunol, 1995 Mar 1; 154(5):2257-65.

97. Nanda NK, Sercarz EE, Hsu DH, Kronenberg M. A unique pattern of lymphokine synthesis is a characteristic of certain antigen specific suppressor T cell clones. Int J Immunol 1994; 6(5):731-7.

98. Tahara H, Zeh HJ, Storkus WJ, et al. Fibroblasts genetically engineered to secrete interleukin 12 can suppress tumor growth and induce anti-tumor immunity to a murine melanoma in vivo. Cancer Res 1994; 54(1):182-9.

99. Fishman MA, Perelson AS. Th1/Th2 cross regulation. J Theor Biol 1994; 170(1):25-56.

100. Wang L, Goillot E, Tepper RI. IL-10 inhibits alloreactive cytotoxic T lymphocyte generation in vivo. Cell Immunol 1994; 159(2):152-69.

101. McAdam AJ, Pulaski BA, Harkins SS, Hutter EK, Lord EM, Frelinger JG. Synergistic effects of co-expression of the TH1 cytokines IL-2 and IFN-gamma on generation of murine tumor reactive cytotoxic cells. Int J Cancer 1995; 61(5):628-34.

102. Benvenisty N, Reshef L, Direct introduction of genes into rats and expression of the genes PNAS 1986; 83: 9551-555.

103. Ulmer JB, Donnelly JJ, Parker SE, et al. Heterologous protection against influenza by injection of DNA encoding a viral protein. Science March 1993; 259:1745-49.

104. Robinson HL, Hunt LA, Webster RG. Protection against a lethal influenza virus challenge by immunization with a hemagglutinin-expressing plasmid DNA. Vaccine 1993; 11(9):957-60.

105. Yankauckas MA, Morrow JE, Parker SE, et al. Long term anti-nucleoprotein cellular and humoral immunity is induced by intramuscular injection of plasmid DNA containing NP gene. 1993 DNA and Cell Biology 12(9):771-76.

106. Cox GJM, Zamb TJ, Babiuk LA. Bovine Herpesvirus 1: Immune responses in mice and cattle injected with plasmid DNA. J Virol Sept. 1993; 67(9):5664-67.

107. Wang B, Ugen KE, Srikantan V, et al. Gene inoculation generates immune responses against human immunodeficiency virus type 1. Proc Natl Acad Sci USA. May 1993; 90:4156-60.

108. Wang B, Boyer J, Srikantan V, et al. DNA inoculation induces neutralizing immune responses against uman immunodeficiency virus type 1 in mice and nonhuman primates. DNA and Cell Biology 1993; 12(9):799-805.

109. Davis HL, Michel ML, Whalen RG. DNA-based immunization induces continuous secretion of hepatitis B surface antigen and high levels of circulating antibody. Human Mol Genetics 1993; 2(11):1847-51.

110. Fynan EF. Webster RG, Fuller DH, Haynes JR, Santoro JC, Robinson HL, DNA vaccines: Protective immunization by parenteral, mucosal, and gene gun inoculations. Proc Natl Acad Sci USA. December 1993; 90: 11478-82.

111. Xiang ZQ, Spitalnik S, Tran M, Wunner WH, Cheng J, Ertl HC. Vaccination with a plasmid vector carrying the rabies virus glycoprotein gene induces protective immunity against rabies virus. Virology 1994; 199:132-40.

112. Conry RM, LoBuglio AF. Kantor J, et. al. Immune response to a carcinoembryonic antigen polynucleotide vaccine. Cancer Res March 1994; 54:1164-68.

113. Sedegah M, Hedstrom R, Hobart P, Hoffman SL. Protection against Malaria by immunization with plasmid DNA encoding circumsporozoite proteinProc Natl Acad Sci USA. October 1994; 91:9866-70.

114. Conry RM, LoBuglio AF. Kantor J, et. al. Immune response to a carcinoembryonic antigen polynucleotide vaccine. Cancer Res March 1994; 54:1164-68.

115. Whalen RG, Davis HL. DNA-mediated immunization and the energetic immune response to hepatitis B surface antigen. Clinic Immunol Immunopath April 1995; 75(1):1-12.

116. Michele ML, Davis HL, Schleef M, Mancini M, Tiollais P, Whalen RG. DNA-mediated immunization to the hepatitis B surface antigen in mice: Aspects of the humoral response mimic hepatitis B viral infection in humans. Proc Natl Acad Sci USA. June 1995; 92:5307-11.

117. Pardoll DM, Beckerleg AM. Exposing the Immunology of Naked DNA Vaccines. 1995 Immunity 3:165-69.

118. Davis HL, Demeneix BA, Quantin B, Coulombe J, Whalen RG. Plasmid DNA is superior to viral vectors for direct gene transfer into adult mouse skeletal muscle. Human Gen therapy 1993; 4:733-40.

119. Fazio VM, Fazio S, Rinaldi M et al. Accumulation of human apoprotein-E in rat plasma after in vivo intramuscular injection of naked DNA. Biochem Biophys Res Comm April 1994; 200(1):298-305.

120. Bourne N Stanberry LR, Bernstein Dl, Lew D. DNA immunization against experimental genital herpes simplex virus infection. J Infect Dis 173(4):800-7 1996.

121. Hildegund E. DNA vaccines to Rabies. Personal communication at NY Acad of Sci sponsored DNA Vaccine Meeting April 1995

122. Yankaukas MA, Morrow JE, Parker SE, et al. Long term anti-nucleoprotein cellular and hum,oral immunity is induced by intramuscular injection of plasmid DNA containing NP gene. DNA Cell Biol 12(9):771-76 1993.

123. Montgomery DL, Shiver JW, Leander K, et al. Heterologous and homologous protection against influenza A by DNA vaccination: optimization of DNA vectors. DNA Cell Biol 12(9):777-83 1993.

124. Lagging LM, Meyer K, Hoft D, et al. Immune responses to plasmid DNA encoding the hepatitis C viral core protein. J Virol 69(9):5859-5863 1995.

125. Major ME, Vitvitski L, Mink MA, et al. DNA-based immunization with chimericvectors for the induction of immune responses against the hepatitis C virus nucleocapsid. J Virol 69(9):5798-5805 1995.

126. Shriver JW, Perry HC, Davies ME, Freed DC, Liu MA.. Cytotoxic T lymphocyte and helper T cell responses following HIV polynucleotide vaccination. Ann NY Acad Sci 772:198-208 1995

127. RobinsonHL, Lu s, Mustafa F, et al. Simian immunodeficiency virus DNA vaccine trial in macaques. Ann NY Acad Sci 772:209-211 1995

128. Jiao S, Williams P, Berg RK, et al. Direct gene transfer into non-human primate myofibers in vivo. Human Gene Ther 3:21-33 1992.

129. Katsumi A, Emi N, Abe A, Hasagawa Y, Ito H, Saitto H. Humoral and cellular immunity to an encoded protein induced by direct DNA injection. Human Gene Ther 5:13335-39 1994

130. Tang d, DeVit M, Johnston SA. Genetic immunization is a simple method for eliciting an immune response. Nature 356:152-54 1992.

131. Ascadi G, Dickson G, Love DR, et al. Human dystrophin expression in mdx mice after intramuscular injection of DNA constructs. Nature 352:81518 1991.

132a. Thomason DB, Booth FW. Stable incorporation of a bacterial gene into adult rat skeletal muscle in vivo. Am J Physiol Mar 1990; 258(3 ptl):c578-81.

132b. Coulie PG, Brichard V, Van Pel A, Wolfel T, Schneider J, Traversari C, et al. A new gene coding for a differentiation antigen recognized by autologous cytolytic T lymphocytes on HLA-A2 melanomas. J Exp Med. 1994 Jul; 180:35-42.

133a. Wolff JA, Williams P, Acsadi G, Jiao S, Jani A, Chong W. Conditions affecting direct gene transfer into rodent muscle in vivo. Biotechniques 1991; 11(4):474-485.

133b. Durrant LG, Buckley TJ, Denton GW, Hardcastle JD, Sewell HF, Robins RA. Enhanced cell mediated tumor killing in patients immunized with human monoclonal anti-idiotypic antibody 105AD7. Cancer Research 1994 Sep 15; 54(18):4827-40.

134a. Wolff JA, Ludtke JL, Acsadi G, Williams P, Jani A. Long-term persistence of plasmid DNA and foreign gene expression in mouse muscle. Human Mol Genet 1992; 1(6):363-69.

134b. Celis E, Fikes J, Wentworth P, Sidney J, Southwood S, Maewal, A, Del Guerrico MF, Sette A, Livingston B. Identification of potential CTL epitopes of tumor-associated antigen MAGE-1 for five common HLA-A alleles. Molecular Immunology, 1994 Dec; 31(18):1423-30.

135a. Manthorpe M, Cornefert-Jensen F, Hartikka J, et al. Gene therapy by intramuscular injection of plasmid DNA: Studies on firefly luciferase gene expression in mice. Human Gene Therpy 1993; 4:419-31.

135b. van der Bruggen P, Bastin J, Gajewski T, Coulie PG, Boel P, De Smet C, Traversari C, Townsend A, Boon T. A peptide encoded by human gene MAGE-3 and presented by HLA-A2 induces cytolytic T lymphocytes that recognize tumor cells expressing MAGE-3. European J Immunol, 1994 Dec; 24(12):3038-43.

136. Morioka N, Kikumoto Y, Hoon DS, Morton DL, Irie RF. A decapeptide (Gln-Asp-Leu-Thr-Met-Lys-Tyr-Gln-Ile-Phe) from human melanoma is recognized by CTL in melanoma patients. J Immunol, 1994 Dec 15; 153(12):5650-8.

137. Frassanito MA, Mayordomo JI, DeLeo RM, Storkus WJ, Lotze MT, DeLeo AB. Identification of meth A sarcoma-derived Class I major histocompatibility complex-associated peptides recognized by a specific CD8 cytotoxic T lymphocytes. Cancer Research, 1995 Jan; 55:124-28.

138. Rivoltini L, Kawakami Y, Sakaguchi K, Southwood S, Sette A, Robbins PF, Marincola FM, Salgaller ML, Yanelli JR, et al. Induction of tumor-reactive CTL from peripheral blood and tumor-infiltrating lymphocytes of melanoma patients by in vitro stimulation with a immunodominant peptide of the human melanoma antigen MART-1. J Immunol, 1995 Mar 1; 154(5):2257-65.

139. Rubin H. The significance of biological heterogeneity. Cancer Metastasis Rev 9:1-20. 1990.

140. Shackney SE, Shankey TV. Genetic and phenotypic heterogeneity of human malignancies: finding order in chaos. Cytometry 21(1):2-5 1995.

141. Fleuren GJ, Gorter A, Kuppen PJ. Tumor heterogeneity and immunotherapy of cancer. Immunological Rev 145:91-122. 1995

142. Oka A, Nomura N, Morita M, Sugisaki H, Sugimoto K, Takanami M. Nucleotide sequence of small ColE1 derivatives: structure of the regions essential for autonomous replication and the colicin El immunity. Molec gen Genet 1979; 172:151-59.

143. Brown EL, Belagaje R, Ryan MJ, Khorana HG. Chemical synthesis and cloning of a tyrosine tRNA gene. Method Enzymology 1979; 68:109-51.

144. DeNoto FM, Moore DD, Goodman HM. Human growth hormone DNA sequence and mRNA structure: possible alternative splicing. Nuc Acids Res. 1981; 9(15):371930.

145. Kozak M The scanning model for translation: an update. J Cell Bio 108:229-41 1989

146. Nelson PJ, Kim HT, Manning WC, Goralski TJ, Krensky AM. Genomic organization and transcriptional regulation of the RANTES cytokine gene. J Immunol 1993; 151-2601-33.

147. Ortiz BD, Krensky AM, Nelson PJ, Kinetics of transcription factors regulating the RANTES chemokine gene reveal a developmental switch in nuclear events during T-lymphocyte maturation. Mol Cell Biol 16(1):202-10 1996.

148. Sallusto F, Lanzavecchia A. Efficient presentation of soluble antigen by cultured human dendritic cells is maintained by granulocyte/macrophage colony stimulating factor plus interleukin 4 and downregulated by tumor necrosis factor alpha. J Exp Med. 179:1109-1118 1994.

149. Mizoguchi H, O'Shea JJ Longo DL, Loeffler CM, McVicar DW, Ochoa AC. Alterations in signal transduction molecules in T lymphocytes from tumor bearing mice. Science 258(5089):1795-8 1992

150. Downward J, Yarden Y, Mayes E, et al. Close similarity of epidermal growth factor receptor and v-erb B oncogene protein sequences. Nature 1984; 307:521-27.

151. Bargmann CI, Hung MC, Weinberg RA. The neu oncogene encodes an epidermal growth factor receptor-related protein. Nature 1986; 319:226-230.

152. Yamamoto T, Ikawa S, Akiyama T, et al. Similarity of protein encoded by the humanc-erb-B2 gene to epidermal growth factor receptor. Nature 1986; 3139:230-34.

153. Schechter AL, Hung MC, Vaidyanathan L, et al. The neu gene: An erbB-homologous gene distinct from and unlinked to the gene encoding the EGF receptor. Science 1985; 229:976-78.

154. Gullick WJ. The role of the epidermal growth factor receptor and the c-erbB-2 protein in breast cancer. Int J Cancer 1990 ; 5:55-61.

155. Slamon DJ, Godolphin W, Johns LA, et al. Studies of the HER-2/neu proto-oncogene in human breast and ovarian cancer. Science 1989; 244:707-12.

156. van de vijver MJ, Mooi WJ, Wisman O Oeterse JL, Nusse R. Immunohistochemical detection of the neu protein in tissue sections of human breast tumors with amplified neu DNA. Oncogene 1988; 2:175-78.

157. Molina R, Filelia X Segui MA, et al. Oncogenic proteins and prognostic correlations in breast cancer. In Vivo 1993; 7:585-90.

158. Tal M, Wetzler M, Josefberg Z, et al. Sporadic amplification of HER-2/neu proto-oncogen in adenocarcinomas of various tissues. Cancer Res 1988; 48:1517-20.

159. Paik S, Hazan Z, Fisher ER, et al. Pathological findings from the NSABP (protocol B-06): prognostic significance of erbB-2 protein overexpression in primary breast cancer. J Clin Oncology 1990; 8:103-12.

160. Thor AD, Schwartz LH, Koerner FC, et al. Analysis of c-erbB-2 expression in breast carcinomas with clinical follow-up. Cancer Res 1989; 49:7147-52.

161. Tandon AK, Clark GM, Chamness,GC, Ulrich A, McGuire WL. HER-2/neu oncogene protein and prognosis in breast cancer. J Clin Oncology 1989; 7:1120-28.

162. Barnes DM, Lammie GA, Millis RR, Gullick WL, Allen DS, Altman DG. An immunohistochemical evaluation of c-erbB-2 expression in human breast carcinoma. Br J Cancer 1988; 58:488-52.

163. Wright C, Angus B, Nicholson SJ, et al. Expression of c-erbB-2 oncoprotein: a prognostic indicator in human breast cancer. Cancer Res 1989; 40:2087-90.

164. Zhou DJ, Ahauja H, Cline MJ. Proto-oncogene abnormalities in human breast cancer: c-erbB-2 amplification does not correlate with recurrence of disease. Oncogene 1989; 4:105-8.

165. Tsuda H, Hirohashi S, Shimosato Y, et al. Correlation between long term survival in breast cancer patients and amplification of two putative oncogene-coamplification units: hst-1/int-2 and c-erbB-2/ear-1. Cancer Res 1989; 49(11):3104-8.

166. Potter CR, van Doele S, vande Vijver MJ, et al The expression of the neu oncogene product in breast lesions and in normal and adult human tissues. Histopathology 1989; 15:351-62

167. Bacus SS, Stancovski I, Heberman E, et al. Tumor inhibitory monoclonal antibodies to the HER-2/neu receptor induce differentiation of human breast cancer cells. Cancer Res 1992; 52:2580-89.

168. Hancock MC, Langton BC, Chan T, et al. A monoclonal antibody against c-erbB-2 protein enhances the cytotoxicity of cis-diamminedichloroplatininum against human breast and ovarian tumor cell lines. Cancer Res 1991; 51:4575-80.

169. Sarup JC, Johnson RM, King KL, et al Characterization of an anti-p85 Her2 monoclonal antibody that stimulates receptor function and inhibits tumor growth. Growth Regulation 1991; 1:72-82.

170. Shepard HM, Lewis GD, Sarup JC, et al. Monoclonal antibody therapy of human cancer: taking the HER-2 proto-oncogen to the clinic. J Clin Immunol 1991; 11:117-27.

171. Gullick WJ, Berger MS, Bennett PLP, Rothbard JB, Waterfield MD. Expression of the c-erbB-2 protein in normal and transformed cells. Int J Cancer 1987; 40:246-54.

172. Ghosh P, Komschlies KL, Cippitelli M, et. Al. Gradual loss of T-helper 1 populations in spleen of mice during progressive tumor growth. J Natl Cancer Inst 87(19):1478-83 1995

173. Stampfer MR, Yaswen P. Culture systems for study of human mammary epithelial cell proliferation, differentiation and transformation. Cancer Surveys 1993; 18:7-33.

174. Shay JW, Tomlinson G, Piatyszek MA, Gollahon LS. Spontaneous in vitro immortalization of breast epithelial cells from a patient with Li-Fraumeni syndrome. Mol Cell Biol 1995; 15(1):425-32.

175. Ethier SP, Dilts CM, Pierce LJ, Kokeny K., Mahacek M. Identification of altered growth phenotypes in human breast cancer cells using cell culture methods that support growth of normal and neoplastic mammary epithelial cells. 1993 Proc AACR Abstract #251.

176. Transfection of DNA into eukaryotic cells. Section I, Unit 9.1. In Current Protocols in Molecular Biology Ausuberl FM, Brent R, Kingston RE, et al. Editors 1995 J. Wiley and Sons Inc. NY, NY.

177. Measurement of basic immunologic characteristics of human mononuclear cells. Section II, Unit 7.10, In Current Protocols in Immunology. Coligan JE, Kruisbeck AM, Margulies DH, Shevack EM, and Strober W. Editors 1995 Wiley and Sons Inc. NY, NY.

178. Nelson EL, Xioping L, Hsu FW, et al. Tumor specific, cytotoxic T lymphocyte response after "Idiotype Vaccination" for B cell, non-Hodgkin's lymphoma. Blood, Vol. 88, No. 2 (July 15) 1996, pp. 580-589.

SEQUENCE LISTING

(1) GENERAL INFORMATION:

(i) APPLICANT: (A) NAME:THE GOVERNMENT OF THE UNITED STATES OF AMERICA AS REPRESENTED BY THE SECRETARY, DEPARTMENT OF HEALTH AND HUMAN SERVICES ET AL.

(B) STREET: SUITE 325, 6011 EXECUTIVE BOULEVARD
(C) CITY: ROCKVILLE
(D) STATE: MD

(E) COUNTRY: USA
(F) ZIP: 20852

(ii) TITLE OF INVENTION: NOVEL VECTOR FOR POLYNUCLEOTIDE VACCINES

(iii) NUMBER OF SEQUENCES: 29

(v) COMPUTER READABLE FORM:

(A) MEDIUM TYPE: DISKETTE, 3.5 INCH, 1.44 MB
(B) COMPUTER: IBM PC COMPATIBLE
(C) OPERATING SYSTEM: PC-DOS/MS-DOS
(D) SOFTWARE: WORDPERFECT 5.1

(vi) CURRENT APPLICATION DATA:

(A) APPLICATION NUMBER: PCT/US97/14306
(B) FILING DATE: 14-AUG-1997

(vii) PRIOR APPLICATION DATA:

(A) APPLICATION NUMBER: US60/023931
(B) FILING DATE: 14-AUG-1996

(2) INFORMATION FOR SEQ ID NO:1:

(i) SEQUENCE CHARACTERISTICS:

(A) LENGTH: 453 base pairs
(B) TYPE: nucleic acid
(C) STRANDEDNESS: single
(D) TOPOLOGY: unknown

(ii) MOLECULE TYPE: Cdna

(iii) HYPOTHETICAL: No

(iv) ANTI-SENSE: No

(xi) SEQUENCE DESCRIPTION:SEQ ID NO:1:

```
GGCCGCGTTG CTGGCGTTTT TCCATAGGCT CCGCCCCCCT     40
GACGAGCATC ACAAAAATCG ACGCTCAAGT CAGAGGTGGC     80
GAAACCCGAC AGGACTATAA AGATACCAGG CGTTTCCCCC    120
TGGAAGCTCC CTCGTGCGCT CTCCTGTTCC GACCCTGCCG    160
CTTACCGGAT ACCTCTCCGC CTTTCTCCCT TCGGGAAGCG    200
TGGCGCTTTC TCAATGCTCA CGCTGTAGGT ATCTCAGTTC    240
GGTGTAGGTC GTTCGCTCCA AGCTGGGCTG TGTGCACGAA    280
CCCCCCGTTC AGCCCGACCG CTGCGCCTTA TCCGGTAACT    320
ATCGTCTTGA GTCCAACCCG GTAAGACACG ACTTATCGCC    360
ACTGGCAGCA GCCACTGGTA ACAGGATTAG CAGAGCGAGG    400
TATGTAGGCG GTGCTACAGA GTTCTTGAAG TGGTGGCCTA    440
ACTACGGCTA CAC                                 453
```

(2) INFORMATION FOR SEQ ID NO:2:

(i) SEQUENCE CHARACTERISTICS:

(A) LENGTH: 453 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:2:

```
GTGTAGCCGT AGTTAGGCCA CCACTTCAAG AACTCTGTAG     40
CACCGCCTAC ATACCTCGCT CTGCTAATCC TGTTACCAGT     80
GGCTGCTGCC AGTGGCGATA AGTCGTGTCT TACCGGGTTG    120
GACTCAAGAC GATAGTTACC GGATAAGGCG CAGCGGTCGG    160
GCTGAACGGG GGGTTCGTGC ACACAGCCCA GCTTGGAGCG    200
AACGACCTAC ACCGAACTGA GATACCTACA CCGTGAGCAT    240
TGAGAAAGCG CCACGCTTCC CGAAGGGAGA AAGGCGGACA    280
GGTATCCGGT AAGCGGCAGG GTCGGAACAG GAGAGCGCAC    320
GAGGGAGCTT CCAGGGGGAA ACGCCTGGTA TCTTTATAGT    360
CCTGTCGGGT TTCGCCACCT CTGACTTGAG CGTCGATTTT    400
TGTGATGCTC GTCAGGGGGG CGGAGCCTAT GGAAAAACGC    440
CAGCAACGCG GCC                                 453
```

(2) INFORMATION FOR SEQ ID NO:3:

(i) SEQUENCE CHARACTERISTICS:

(A) LENGTH: 209 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:3:

```
GAATTCTTTC GGACTTTTGA AAGTGATGGT GGTGGCCGAA     40
GGATTCGAAC CTTCGAAGTC GATGACGGCA GATTTAGAGT     80
CTGCTCCCTT TGGCCGCTCG GGAACCCCAC CACGGGTAAT    120
GCTTTTACTG GCCTGCTCCC TTATCGGGAA GCGGGGCGCA    160
TCATATCAAA TGACGCGCCG CTGTAAAGTG TTACGTTGAG    200
AAAGAATTC                                      209
```

(2) INFORMATION FOR SEQ ID NO:4:

(i) SEQUENCE CHARACTERISTICS:

(A) LENGTH: 209 base pairs
(B) TYPE: nucleic acid

(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:4:

```
GAATTCTTTC TCAACGTAAC ACTTTACAGC GGCGCGTCAT     40
TTGATATGAT GCGCCCCGCT TCCCGATAAG GGAGCAGGCC     80
AGTAAAAGCA TTACCCGTGG TGGGGTTCCC GAGCGGCCAA    120
AGGGAGCAGA CTCTAAATCT GCCGTCATCG ACTTCGAAGG    160
TTCGAATCCT TCCCCCACCA CCATCACTTT CAAAAGTCCG    200
AAAGAATTC                                      209
```

(2) INFORMATION FOR SEQ ID NO:5:

(i) SEQUENCE CHARACTERISTICS:

(A) LENGTH: 6 base pairs
(B) TYPE: nucleic acid
(C) STRANDEDNESS: single
(D) TOPOLOGY: unknown

(ii) MOLECULE TYPE: cDNA
(iii) HYPOTHETICAL: No
(xi) SEQUENCE DESCRIPTION:SEQ ID NO:5:

```
AATAAA    6
```

(2) INFORMATION FOR SEQ ID NO:6:

(i) SEQUENCE CHARACTERISTICS:

(A) LENGTH: 6 base pairs
(B) TYPE: nucleic acid
(C) STRANDEDNESS: single
(D) TOPOLOGY: unknown

(ii) MOLECULE TYPE: cDNA
(iii) HYPOTHETICAL: No
(iv) ANTI-SENSE: No
(xi) SEQUENCE DESCRIPTION:SEQ ID NO:6:

```
ATTAAA    6
```

(2) INFORMATION FOR SEQ ID NO:7:

(i) SEQUENCE CHARACTERISTICS:

(A) LENGTH: 6 base pairs

(B) TYPE: nucleic acid
(C) STRANDEDNESS: single
(D) TOPOLOGY: unknown

(ii) MOLECULE TYPE: cDNA
(iii) HYPOTHETICAL: No
(xi) SEQUENCE DESCRIFTION:SEQ ID NO:7:

AGTAAA 6

(2) INFORMATION FOR SEQ ID NO:8:

(i) SEQUENCE CHARACTERISTICS:

(A) LENGTH: 6 base/pairs
(B) TYPE: nucleic acid
(C) STRANDEDNESS: single
(D) TOPOLOGY: unknown

(ii) MOLECULE TYPE: cDNA

(iii) HYPOTHETICAL: No

(xi) SEQUENCE DESCRIPTION:SEQ ID NO:8:

AAGAAC 6

(2). INFORMATION FOR SEQ ID NO:9:

(i) SEQUENCE CHARACTERISTICS:

(A) LENGTH: 6 base pairs
(B) TYPE: 'nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:9:

AATACA 6

(2) INFORMATION FOR SEQ ID NO:10:

(i) SEQUENCE CHARACTERISTICS:

(A) LENGTH: 227 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:10:

```
GCCTTAAGGG CCATATGGTG AGTGGATCCC TTGACCCCAG    40
GCGGGGATGG GGAGACCTGT AGTCAGAGCC CCCGGGCAGC    80
ACAGGCCAAT GCCCGTCCTT CCCCTGCAGG ATGAGTAGTG   120
AGTGCCTCTC CTGGCCCTGG AAGTTGCCAC TCCAGTGCCC   160
ACCAGCCTTG TCCTAATAAA ATTAAGTTGC ATCATTTTGT   200
CTGACTAGGT GTCCTCTATA ATATTAT                 227
```

(2) INFORMATION FOR SEQ ID NO:11:

(i) SEQUENCE CHARACTERISTICS:

(A) LENGTH: 227 base pairs
(B) TYPE: nucleic acid
(C) STRAMDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:11:

```
ATAATATTAT AGAGGACACC TAGTCAGAAC AAATGATGCA    40
ACTTAATTTT ATTAGGACAA GGCTGGTGGG CACTGGAGTG    80
GCAACTTCCA GGGCCAGGAG AGGCACTCAC TACTCATCCT   120
GCAGGGGAAG GACGGGCATT GGCCTGTGCT GCCCGGGGGC   160
TCTGACTACA GGTCTCCCCC ATCCCCGCCT GGGGTCAAGG   200
CATCCACTCA CCATATGGCC CTTAAGG                 227
```

(2) INFORMATION FOR SEQ ID NO:12:

(i) SEQUENCE CHARACTERISTICS:

(A) LENGTH: 252 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:12:

```
CCTCGGTACC TGCCATGGCG CGGATTCTTT ATCACTGATA    40
AGTTGGTGGA CATATTATGT TTATCAGTGA TAAAGTGTCA    80
AGCATGACAA AGTTGCAGCC GAATACAGTG ATCCGTGCCG   120
GCCCTGGACT GTTGAACGAG GTCGGCGTAG ACGGTCTGAC   160
GACACGCAAA CTGGCGGAAC GGTTGGGGGT GCAGCAGCCG   200
GCGCTTTACT GGCACTTCAG GAACAAGCGG GCGCCTTAAG   240
GGCCATATGC CG                                  252
```

(2) INFORMATION FOR SEQ ID NO:13:

(i) SEQUENCE CHARACTERISTICS:

(A) LENGTH: 35 base pairs
(B) TYPE: nucleic acid
(C) STRANDEDNESS: single
(D) TOPOLOGY: unknown

(ii) MOLECULE TYPE: cDNA
(iii) HYPOTHETICAL: No
(iv) ANTI-SENSE: No
(xi) SEQUENCE DESCRIPTION:SEQ ID NO:13:

```
CCTCGGTACC TGCCACCATG GCGCGGATTC TTTAT    35
```

(2) INFORMATION FOR SEQ ID NO:14:

(i) SEQUENCE CHARACTERISTICS:

(A) LENGTH: 38 base pairs
(B) TYPE: nucleic acid
(C) STRANDEDNESS: single
(D) TOPOLOGY: unknown

(ii) MOLECULE TYPE: cDNA
(iii) HYPOTHETICAL: No
(iv) ANTI-SENSE: No
(xi) SEQUENCE DESCRIPTION:SEQ ID NO:14:

```
CGGCATATGG CCTTAAGGCG CCCGCTTGTT CCTGAAGT    38
```

(2) INFORMATION FOR SEQ ID NO:15:

(i) SEQUENCE CHARACTERISTICS:

(A) LENGTH: 228 base pairs
(B) TYPE: nucleic acid
(C) STRANDEDNESS: single
(D) TOPOLOGY: unknown

(ii) MOLECULE TYPE: cDNA
(iii) HYPOTHETICAL: No
(iv) ANTI-SENSE: No
(xi) SEQUENCE DESCRIPTION:SEQ ID NO:15:

```
GCCTTAAGGG CCATATGGTG AGTGGATGCC TTGACCCCAG    40
GCGGGGATGG GGGAGACCTG TAGTCAGAGC CCCCGGGCAG    80

CACAGGCCAA TGCCCGTCCT TCCCCTGCAG GATGAGTAGT   120
GAGTGCCTCT CCTGGCCCTG GAAGTTGCCA CTCCAGTGCC   160
CACCAGCCTT GTCCTAATAA AATTAAGTTG CATCATTTTG   200
TCTGACTAGG TGTCCTCTAT AATATTAT                228
```

(2) INFORMATION FOR SEQ ID NO:16:

(i) SEQUENCE CHARACTERISTICS:

(A) LENGTH: 1425 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:16:

```
TGCCATGGCG CGGATTCTTT ATCACTGATA AGTTGGTGGA     40
CATATTATGT TTATCAGTGA TAAAGTGTCA AGCATGACAA     80
AGTTGCAGCC GAATACAGTG ATCCGTGCCG GCCCTGGACT    120
GTTGAACGAG GTCGGCGTAG ACGGTCTGAC GACACGCAAA    160
CTGGCGGAAC GGTTGGGGGT GCAGCAGCCG GCGCTTTACT    200
GGCACTTCAG GAACAAGCGG GCGCCTTAAG GGCCATATGG    240
TGAGTGGATG CCTTGACCCC AGGCGGGGAT GGGGGAGACC    280
TGTAGTCAGA GCCCCCGGGC AGCACAGGCC AATGCCCGTC    320
CTTCCCCTGC AGTGAGTAGT GACTGCCCGG GTGGGATCCC    360
TGTGACCCCT CCCCAGTGCC TCTCCTGGCC CTGGAAGTTG    400
CCACTCCAGT GCCCACCAGC CTTGTCCTAA TAAAATTAAG    440
TTGCATCATT TTGTCTGACT AGGTGTCCTC TATAATATTA    480
TAAGCTTGAT ATCGAATTCT TTCTCAACGT AACACTTTAC    520
AGCGGCGCGT CATTTGATAT GATGCGCCCC GCTTCCCGAT    560
AAGGGAGCAG GCCAGTAAAA GCATTACCCG TGGTGGGGTT    600
CCCGAGCGGC CAAAGGGAGC AGACTCTAAA TCTGCCGTCA    640
TCGACTTCGA AGGTTCGAAT CCTTCCCCCA CCACCATCAC    680
TTTCAAAAGT CCGAAAGAAT TCCTGCAGCC CGTGTAGCCG    720
TAGTTAGGCC ACCACTTCAA GAACTCTGTA GCACCGCCTA    760
CATACCTCGC TCTGCTAATC CTGTTACCAG TGGCTGCTGC    800
CAGTGGCGAT AAGTCGTGTC TTACCGGGTT GGACTCAAGA    840
CGATAGTTAC CGGATAAGGC GCAGCGGTCG GGCTGAACGG    880
GGGGTTCGTG CACACAGCCC AGCTTGGAGC GAACGACCTA    920
CACCGAACTG AGATACCTAC AGCGTGAGCA TTGAGAAAGC    960
GCCACGCTTC CCGAAGGGAG AAAGGCGGAC AGGTATCCGG   1000
TAAGCGGCAG GGTCGGAACA GGAGAGCGCA CGAGGGAGCT   1040
TCCAGGGGGA AACGCCTGGT ATCTTTATAG TCCTGTCGGG   1080
TTTCGCCACC TCTGACTTGA GCGTCGATTT TTGTGATGCT   1120
CGTCAGGGGG GCGGAGCCTA TGGAAAAACG CCAGCAACGC   1160
GGCCGGGGGA TCCGGAGAGC TCACTCTAGA TGAGAGAGCA   1200
GTGAGGGAGA GACAGAGACT CGAATTTCCG GAGCTATTTC   1240
AGTTTTCTTT TCCGTTTTGT GCAATTTCAC TTATGATACC   1280

GGCCAATGCT TGGTTGCTAT TTTGGAAACT CCCCTTAGGG   1320
GATGCCCCTC AACTGGCCCT ATAAAGGGCC AGCCTGAGCT   1360
GCAGAGGATT CCTGCAGAGG ATCAAGACAG CACGTGGACC   1400
TCGCACAGCC TCTCCCACAG GTACC                   1425
```

(2) INFORMATION FOR SEQ ID NO:17:

(i) SEQUENCE CHARACTERISTICS:

(A) LENGTH: 719 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:17:

```
ATGAGCAAGG GCGAGGAACT GTTCACTGGC GTGGTCCCAA    40
TTCTCGTGGA ACTGGATGGC GATGTGAATG GGCACAAATT    80
TTCTGTCAGC GGAGAGGGTG AAGGTGATGC CACATACGGA   120
AAGCTCACCC TGAAATTCAT CTGCACCACT GGAAAGCTCC   160
CTGTGCCATG GCCAACACTG GTCACTACCT TCACCTATGG   200
CGTGCAGTGC TTTTCCAGAT ACCCAGACCA TATGAACGAG   240
CATGACTTTT TCAAGAGCGC CATGCCCGAG GGCTATGTGC   280
AGGAGAGAAC CATCTTTTTC AAAGATGACG GGAACTACAA   320
GACCCGCGCT GAAGTCAAGT TCGAAGGTGA CACCCTGGTG   360
AATAGAATCG AGTTGAAGGG CATTGACTTT AAGGAAGATG   400
GAAACATTCT CGGCCACAAG CTGGAATACA ACTATAACTC   440
CCACAATGTG TACATCATGG CCGACAAGCA AAAGAATGGC   480
ATCAAGGTCA ACTTCAAGAT CAGACACAAC ATTGAGGATG   520
GATCCGTGCA GCTGGCCGAC CATTATCAAC AGAACACTCC   560
AATCGGCGAC CGCCCTGTGC TCCTCCCAGA CAACAATTAC   600
CTGTCCACCC AGTCTGCCCT GTCTAAAGAT CCCAACGAAA   640
AGAGAGACCA CATGGTCCTG CTGGAGTTTG TGACCGCTGC   680
TGGGATCACA CATGGCATGG ACGAGCTGTA CAAGTGAGC    719
```

(2) INFORMATION FOR SEQ ID NO:18:

(i) SEQUENCE CHARACTERISTICS:

(A) LENGTH: 1911 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:18:

```
TATGAGCAAG GGCGAGGAAC TGTTCACTGG CGTGGTCCCA   40
ATTCTCGTGG AACTGGATGG CGATGTGAAT GGGCACAAAT   80
TTTCTGTCAG CGGAGAGGGT GAAGGTGATG CCACATACGG  120
AAAGCTCACC CTGAAATTCA TCTGCACCAC TGGAAAGCTC  160
CCTGTGCCAT GGCCAACACT GGTCACTACC TTCACCTATG  200
GCGTGCAGTG CTTTTCCAGA TACCCAGACC ATATGAAGCA  240
GCATGACTTT TTCAAGAGCG CCATGCCCGA GGGCTATGTG  280
CAGGAGAGAA CCATCTTTTT CAAAGATGAC GGGAACTACA  320
AGACCCGCGC TGAAGTCAAG TTCGAAGGTG ACACCCTGGT  360
GAATAGAATC GAGTTGAAGG GCATTGACTT TAAGGAAGAT  400
GGAAACATTC TCGGCCACAA GCTGGAATAC AACTATAACT  440
CCCACAATGT GTACATCATG GCCGACAAGC AAAAGAATGG  480
CATCAAGGTC AACTTCAAGA TCAGACACAA CATTGAGGAT  520
GGATCCGTGC AGCTGGCCGA CCATTATCAA CAGAACACTC  560
CAATCGGCGA CGGCCCTGTG CTCCTCCCAG ACAACCATTA  600
CCTGTCCACC CAGTCTGCCC GTCTAAAGAT CCCAACGAAA  640
AGAGAGACCA CATGGTCCTG CTGGAGTTTG TGACCGCTGC  680
TGGGATCACA CATGGCATGG ACGAGCTGTA CAAGTGAGCC  720
ATATGGTGAG TGGATGCCTT GACCCCAGGC GGGGATGGGG  760
GAGACCTGTA GTCAGAGCCC CCGGGCAGCA CAGGCCAATG  800
CCCGTCCTTC CCCTGCAGTG AGTAGTGACT GCCCGGGTGG  840
GATCCCTGTG ACCCCTCCCC AGTGCCTCTC CTGGCCCTGG  880
AAGTTGCCAC TCCAGTGCCC ACCAGCCTTG TCCTAATAAA  920
ATTAAGTTGC ATCATTTTGT CTGACTAGGT GTCCTCTATA  960
ATATTATAAG CTTGATATCG AATTCTTTCT CAACGTAACA 1000
CTTTACAGCG GCGCGTCATT TGATATGATG CGCCCCGCTT 1040
CCCGATAAGG GAGCAGGCCA GTAAAAGCAT TACCCGTGGT 1080
GGGGTTCCCG AGCGGCCAAA GGGAGCAGAC TCTAAATCTG 1120
CCGTCATCGA CTTCGAAGGT TCGAATCCTT CCCCCACCAC 1160
CATCACTTTC AAAAGTCCGA AAGAATTCCT GCAGCCCGTG 1200
TAGCCGTAGT TAGGCCACCA CTTCAAGAAC TCTGTAGCAC 1240
CGCCTACATA CCTCGCTCTG CTAATCCTGT TACCAGTGGC 1280
TGCTGCCAGT GGCGATAAGT CGTGTCTTAC CGGGTTGGAC 1320
TCAAGACGAT AGTTACCGGA TAAGGCGCAG CGGTCGGGCT 1360
GAACGGGGGG TTCGTGCACA CAGCCCAGCT TGGAGCGAAC 1400
GACCTACACC GAACTGAGAT ACCTACAGCG TGAGCATTGA 1440
GAAAGCGCCA CGCTTCCCGA AGGGAGAAAG GCGGACAGGT 1480
ATCCGGTAAG CGGCAGGGTC GGAACAGGAG AGCGCACGAG 1520
GGAGCTTCCA GGGGGAAACG CCTGGTATCT TTATAGTCCT 1560
GTCGGGTTTC GCCACCTCTG ACTTGAGCGT CGATTTTTGT 1600
GATGCTCGTC AGGGGGGCGG AGCCTATGGA AAAACGCCAG 1640
CAACGCGGCC GGGGGATCCG GAGAGCTCAC TCTAGATGAG 1680
AGAGCAGTGA GGGAGAGACA GAGACTCGAA TTTCCGGAGC 1720
TATTTCAGTT TTCTTTTCCG TTTTGTGCAA TTTCACTTAT 1760
GATACCGGCC AATGCTTGGT TGCTATTTTG GAAACTCCCC 1800
TTAGGGGATG CCCCTCAACT GGCCCTATAA AGGGCCAGCC 1840
TGAGCTGCAG AGGATTCCTG CAGAGGATCA AGACAGCACG 1880
TGGACCTCGC ACAGCCTCTC CCACAGGTAC C          1911
```

(2) INFORMATION FOR SEQ ID NO:19:

(i) SEQUENCE CHARACTERISTICS:

(A) LENGTH: 69 amino acids
(B) TYPE: amino acid
(D) TOPOLOGY: unknown

(ii) MOLECULE TYPE: peptide

(xi) SEQUENCE DESCRIPTION:SEQ ID NO:19:

Pro Asp Leu Ser Tyr Met Pro Ile Trp Lys Phe Pro
1 5 10
Asp Glu Glu Gly Ala Cys Gln Pro Cys Pro Ile Asn
15 20
Cys Thr His Ser Cys Val Asp Leu Asp Asp Lys Gly
25 30 35
Cys Pro Ala Glu Gln Arg Ala Ser Pro Leu Thr Ser
40 45
Ile Ile Ser Ala Val Val Gly Ile Leu Leu Val Val
50 55 60
Val Leu Gly Val Val Phe Gly Ile Leu
65

(2) INFORMATION FOR SEQ ID NO:20:

(i) SEQUENCE CHARACTERISTICS:

(A) LENGTH: 287 amino acids
(B) TYPE: amino acid
(D) TOPOLOGY: unknown

(ii) MOLECULE TYPE: protein

(xi) SEQUENCE DESCRIPTION:SEQ ID NO:20:

Pro Ala Pro Gly Ala Gly Gly Met Val His His Arg
1 5 10
His Arg Ser Ser Ser Thr Arg Ser Gly Gly Gly Asp
15 20
Leu Thr Leu Gly Leu Glu Pro Ser Glu Glu Glu Ala
25 30 35
Pro Arg Ser Pro Leu Ala Pro Ser Glu Gly Ala Gly
40 45
Ser Asp Val Phe Asp Gly Asp Leu Gly Met Gly Ala
50 55 60
Ala Lys Gly Leu Ser Leu Pro Thr His Asp Pro Ser
65 70
Pro Leu Gln Arg Tyr Ser Glu Asp Pro Thr Val Pro
75 80
Leu Pro Ser Glu Thr Asp Gly Tyr Val Ala Pro Leu
85 90 95
Thr Cys Ser Pro Gln Pro Glu Tyr Val Asn Gln Pro
100 105
Asp Val Arg Pro Pro Pro Ser Pro Arg Glu Gly Pro
110 115 120
Leu Pro Ala Ala Arg Pro Ala Gly Ala Thr Leu Glu
125 130
Arg Pro Lys Thr Leu Ser Pro Gly Lys Asn Gly Val
135 140

```
Val Lys Asp Val Phe Ala Phe Gly Gly Ala Val Glu
145                 150                 155
Asn Pro Glu Tyr Leu Thr Pro Gln Gly Thr Cys Ser
            160                 165
Pro Gln Pro Glu Tyr Val Asn Gln Pro Asp Val Arg
    170                 175                 180
Pro Gln Pro Pro Ser Pro Arg Glu Gly Pro Leu Pro
                185                 190
Ala Ala Arg Pro Ala Gly Ala Thr Leu Glu Arg Pro
        195                 200
Lys Leu Ser Pro Gly Lys Asn Gly Val Val Lys Asp
205                 210                 215
Val Phe Ala Phe Gly Gly Ala Val Glu Asn Pro Glu
            220                 225
Tyr Leu Thr Pro Gln Gly Gly Ala Ala Pro Gln Pro
    230                 235                 240
His Pro Pro Pro Ala Phe Ser Pro Ala Phe Asp Asn
                245                 250
Leu Tyr Tyr Trp Asp Asp Pro Pro Glu Arg Gly Ala
        255                 260
Pro Pro Ser Thr Phe Lys Gly Thr Pro Thr Ala Glu
260                 270                 275
Asn Pro Glu Tyr Leu Gly Leu Asp Val Pro Val
            280                 285
```

(2) INFORMATION FOR SEQ ID NO:21:

(i) SEQUENCE CHARACTERISTICS:

(A) LENGTH: 22 amino acids
(B) TYPE: amino acid
(D) TOPOLOGY: unknown

(ii) MOLECULE TYPE: peptide

(xi) SEQUENCE DESCRIPTION:SEQ ID NO:21:

```
Ile Ile Ser Ala Val Val Gly Ile Leu Leu Val Val
 1              5                   10

Val Leu Gly Val Val Phe Gly Ile Leu Ile
        15                  20
```

(2) INFORMATION FOR SEQ ID NO:22:

(i) SEQUENCE CHARACTERISTICS:

(A) LENGTH: 2125 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:22:

```
GCCACCATGG CCCCTGACCT CTCCTACATG CCCATCTGGA      40
AGTTTCCAGA TGAGGAGGGC GCATGCCAGC CTTGCCCCAT      80
CAACTGCACC CACTCCTGTG TGGACCTGGA TGACAAGGGC     120
TGCCCCGCCG AGCAGAGAGC CAGCCCTCTG ACGTCCATCA     160
TCTCTGCGGT GGTTGGCATT CTGCTGGTCG TGGTCTTGGG     200
GGTGGTCTTT GGGATCCTCA TCAAGCGACG GCAGCAGAAG     240
ATCACATGTC CAGACCCTGC CCCGGGCGCT GGGGGCATGG     280
TCCACCACAG GCACCGCAGC TCATCTACCA GGAGTGGCGG     320
TGGGGACCTG ACACTAGGGC TGGAGCCCTC TGAAGAGGAG     360
GCCCCCAGGT CTCCACTGGC ACCCTCCGAA GGGGCTGGCT     400
CCGATGTATT TGATGGTGAC CTGGGAATGG GGGCAGCCAA     440
GGGGCTGCAA AGCCTCCCCA CACATGACCC CAGCCCTCTA     480
CAGCGGTACA GTGAGGACCC CACAGTACCC CTGCCCTCTG     520
AGACTGATGG CTACGTTGCC CCCCTGACCT GCAGCCCCCA     560
GCCTGAATAT GTGAACCAGC CAGATGTTCG GCCCCAGCCC     600
CCTTCGCCCC GAGAGGGCCC TCTGCCTGCT GCCCGACCTG     640
CTGGTGCCAC TCTGGAAAGG CCCAAGACTC TCTCCCCAGG     680
GAAGAATGGG GTCGTCAAAG ACGTTTTTGC CTTTGGGGGT     720
GCCGTGGAGA ACCCCGAGAC TTGACACCCC AGGGAGGAGC     760
TGCCCCTCAG CCCCACCCTC CTCCTGCCTT CAGCCCAGCC     800
TTCGACAACC TCTATTACTG GGACCAGGAC CCACCAGAGC     840
GGGGGGCTCC ACCCAGCACC TTCAAAGGGA CACCTACGGC     880
AGAGAACCCA GAGTACCTGG GTCTGGACGT GCCAGTGTGA     920
AGCCTTAAGG GCCATATGGT GAGTGGATGC CTTGACCCCA     960
GGCGGGGATG GGGGAGACCT GTAGTCAGAG CCCCCGGGCA    1000
GCACAGGCCA ATGCCCGTCC TTCCCCTGCA GTGAGTAGTG    1040
ACTGCCCGGG TGGGATCCCT GTGACCCCTC CCCAGTGCCT    1080
CTCCTGGCCC TGGAAGTTGC CACTCCAGTG CCCACCAGCC    1120
TTGTCCTAAT AAAATTAAGT TGCATCATTT TGTCTGACTA    1160
GGTGTCCTCT ATAATATTAT AAGCTTGATA TCGAATTCTT    1200
TCTCAACGTA ACACTTTACA GCGGCGCGTC ATTTGATATG    1240
ATGCGCCCCG CTTCCCGATA AGGGAGCAGG CCAGTAAAAG    1280
CATTACCCGT GGTGGGGTTC CCGAGCGGCC AAAGGGAGCA    1320
GACTCTAAAT CTGCCGTCAT CGACTTCGAA GGTTCGAATC    1360
CTTCCCCCAC CACCATCACT TTCAAAAGTC CGAAAGAATT    1400
CCTGCAGCCC GTGTAGCCGT AGTTAGGCCA CCACTTCAAG    1440
AACTCTGTAG CACCGCCTAC ATACCTCGCT CTGCTAATCC    1480
TGTTACCAGT GGCTGCTGCC AGTGGCGATA AGTCGTGTCT    1520
TACCGGGTTG GACTCAAGAC GATAGTTACC GGATAAGGCG    1560
CAGCGGTCGG GCTGAACGGG GGGTTCGTGC ACACAGCCCA    1600
GCTTGGAGCG AACGACCTAC ACCGAACTGA GATACCTACA    1640
GCGTGAGCAT TGAGAAAGCG CCACGCTTCC CGAAGGGAGA    1680
AAGGCGGACA GGTATCCGGT AAGCGGCAGG GTCGGAACAG    1720
GAGAGCGCAC GAGGGAGCTT CCAGGGGGAA ACGCCTGGTA    1760
TCTTTATAGT CCTGTCGGGT TTCGCCACCT CTGACTTGAG    1800
CGTCGATTTT TGTGATGCTC GTCAGGGGGG CGGAGCCTAT    1840
GGAAAAACGC CAGCAACGCG GCCGGGGGAT CCGGAGAGCT    1880
CACTCTAGAT GAGAGAGCAG TGAGGGAGAG ACAGAGACTC    1920
GAATTTCCGG AGCTATTTCA GTTTTCTTTT CCGTTTTGTG    1960

CAATTTCACT TATGATACCG GCCAATGCTT GGTTGCTATT    2000
TTGGAAACTC CCCTTAGGGG ATGCCCCTCA ACTGGCCCTA    2040
TAAAGGGCCA GCCTGAGCTG CAGAGGATTC CTGCAGAGGA    2080
TCAAGACAGC ACGTGGACCT CGCACAGCCT CTCCCACAGG    2120
TACCT                                          2125
```

(2) INFORMATION FOR SEQ ID NO:23:

(i) SEQUENCE CHARACTERISTICS:

(A) LENGTH: 27 base pairs
(B) TYPE: nucleic acid
(C) STRANDEDNESS: single
(D) TOPOLOGY: unknown

(ii) MOLECULE TYPE: oligonucleotide

(iii) HYPOTHETICAL: No

(iv) ANTI-SENSE: No

(xi) SEQUENCE, DESCRIPTION:SEQ ID NO:23:

GTCTGCCACC ATGGCCTACT CCCCTGC 27

(2) INFORMATION FOR SEQ ID NO:24:

(i) SEQUENCE CHARACTERISTICS:

(A) LENGTH: 36 base pairs
(B) TYPE: nucleic acid
(C) STRANDEDNESS: single
(D) TOPOLOGY: linear

(ii) MOLECULE TYPE: oligonucleotide

(iii) HYPOTHETICAL: No

(iv) ANTI-SENSE: No

(xi) SEQUENCE DESCRIPTION:SEQ ID NO:24:

TTCTTTGGTG ACCTACCTCT TCGGAATTGC CGAGTC 36

(2) INFORMATION FOR SEQ ID NO:25:

(i) SEQUENCE CHARACTERISTICS:

(A) LENGTH: 1242 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:25:

ATGGAGGAGC CGCAGTCAGA TCCTAGCGTC GAGCCCCCTC 40
TGAGTCAGGA AACATTTTCA GACCTATGGA AACTACTTCC 80
TGAAAACAAC GTTCTGTCCC CCTTGCCGTC CCAAGCAATG 120
GATGATTTGA TGCTGTCCCC GGACGATATT GAACAATGGT 160
TCACTGAAGA CCCAGGTCCA GATGAAGCTC CCAGAATGCC 200
AGAGGCTGCT CCCCGCGTGG CCCCTGCACC AGCAGCTCCT 240
ACACCGGCGG CCCCTGCACC AGCCCCCTCC TGGCCCCTGT 280
CATCTTCTGT CCCTTCCCAG AAAACCTACC AGGGCAGCTA 320
CGGTTTCCGT CTGGGCTTCT TGCATTCTGG GACAGCCAAG 360
TCTGCCACCA TGGCCTACTC CCCTGCGTCT GTGACTTGCA 400
CGTACTCCCC TGCCCTCAAC AAGATGTTTT GCCAACTGGC 440
CAAGACCTGC CCTGTGCAGC TGTGGGTTGA TTCCACACCC 480
CCGCCCGGCA CCCGCGTCCG CGCCATGGCC ATCTACAAGC 520
AGTCACAGCA CATGACGGAG GTTGTGAGGC GCTGCCCCCA 560
CCATGAGCGC TGCTCAGATA GCGATGGTCT GGCCCCTCCT 600
CAGCGTCTTA TCCGAGTGGA AGGAAATTTG CGTGTGGAGT 640
ATTTGGATGA CAGAAACACT TTTCGACATA GTGTGGTGGT 680
GCCCTATGAG CCGCCTGAGG TTGGCTCTGA CTGTACCACC 720
ATCCACTACA ACTACATGTG TAACAGTTCC TGCATGGGCG 760
GCATGAACCG GAGGCCCATC CTCACCATCA TCACACTGGA 800
AGACTCCAGT GGTAATCTAC TGGGACGGAA CAGCTTTGAG 840
GTGCGTGTTT GTGCCTGTCC TGGGAGAGAC CGGCGCACAG 880
AGGAAGAGAA TCTCCGCAAG AAAGGGGAGC CTCACCACGA 920
GCTGCCCCCA GGGAGCACTA AGCGAGCACT GCCCAACAAC 960
ACCAGCTCCT CTCCCCAGCC AAAGAAGAAA CCACTGGATG 1000
GAGAATATTT CACCCTTCAG ATCCGTGGGC GTGAGCGCTT 1040
CGAGATGTTC TTTGGTGACC TACCTCTTCG GAATTGCCGA 1080
GTCTTCCGAG AGCTGAATGA GGCCTTGGAA CTCAAGGATG 1120
CCCAGGCTGG GAAGGAGCCA GGGGGGAGCA GGGCTCACTC 1160
CAGCCACCTG AAGTCCAAAA AGGGTCAGTC TACCTCCCGC 1200
CATAAAAAAC TCATGTTCAA GACAGAAGGG CCTGACTCAG 1240
AC 1242

(2) INFORMATION FOR SEQ ID NO:26:

(i) SEQUENCE CHARACTERISTICS:

(A) LENGTH: 608 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:26:

```
CTCGGGCCGC GTTGCTGGCG TTTTTCCATA GGCTCCGCCC    40
CCCTGACGAG CATCACAAAA ATCGACGCTC AAGTCAGAGG    80
TGGCGAAACC CGACAGGACT ATAAAGATAC CAGGCGTTTC   120
CCCCTGGAAG CTCCCTCGTG CGCTCTCCTG TTCCGACCCT   160
GCCGCTTACC GGATACCTGT CCGCCTTTCT CCCTTCGGGA   200
AGCGTGGCGC TTTCTCAATG CTCACGCTGT AGGTATCTCA   240
GTTCGGTGTA GGTCGTTCGC TCCAAGCTGG GCTGTGTGCA   280
CGAACCCCCC GTTCAGCCCG ACCGCTGCGC CTTATCCGGT   320
AACTATCGTC TTGAGTCCAA CCCGGTAAGA CACGACTTAT   360
CGCCACTGGC AGCAGCCACT GGTAACAGGA TTAGCAGAGC   400
GAGGTATGTA GGCGGTGCTA CAGAGTTCTT GAAGTGGTGG   440
CCTAACTACG GCTACACTAG AAGGACAGTA TTTGGTATCT   480
GCGCTCTGCT GAAGCCAGTT ACCTTCGGAA AAAGAGTTGG   520
TAGCTCTTGA TCCGGCAAAC AAACCACCGC TGGTAGCGGT   560
GGTTTTTTTG TTTGCAAGCA GCAGATTACG CGCAGAAAAA   600
AAGGATCT                                      608
```

(2) INFORMATION FOR SEQ ID NO:27:

(i) SEQUENCE CHARACTERISTICS:

(A) LENGTH: 1547 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:27:

```
GGTACCTGCC ACCATGGCGC GGATTCTTTA TCACTGATAA    40
GTTGGTGGAC ATATTATGTT TATCAGTGAT AAAGTGTCAA    80
GCATGACAAA GTTGCAGCCG AATACAGTGA TCCGTGCCGG   120
CCCTGGACTG TTGAACGAGG TCGGCGTAGA CGGTCTGACG   160
ACACGCAAAC TGGCGGAACG GTTGGGGGTG CAGCAGCCGG   200
CGCTTTACTG GCACTTCAGG AACAAGCGGG CGCCTTAAGG   240
GCCATATGGT GAGTGGATGC CTTGACCCCA GGCGGGGATG   280
GGGGAGACCT GTAGTCAGAG CCCCCGGGCA GCACAGGCCA   320
ATGCCCGTCC TTCCCCTGCA GGATGAGTAG TGAGTGCCTC   360
TCCTGGCCCT GGAAGTTGCC ACTCCAGTGC CCACCAGCCT   400
TGTCCTAATA AAATTAAGTT GCATCATTTT GTCTGACTAG   440
GTGTCCTCTA TAATATTATA AGCTTGATAT CGAATTCTTT   480
CGGACTTTTG AAAGTGATGG TGGTGGGGGA AGGATTCGAA   520
CCTTCGAAGT CGATGACGGC AGATTTAGAG TCTGCTCCCT   560
TTGGCCGCTC GGGAACCCCA CCACGGGTAA TGCTTTTACT   600
GGCCTGCTCC CTTATCGGGA AGCGGGGCGC ATCATATCAA   640
ATGACGCGCC GCTGTAAAGT GTTACGTTGA GAAAGAATTC   680
CTGCAGCCCG CCGCGTTGCT GGCGTTTTTC CATAGGCTCC   720
```

```
GCCCCCCTGA CGAGCATCAC AAAAATCGAC GCTCAAGTCA     760
GAGGTGGCGA AACCCGACAG GACTATAAAG ATACCAGGCG     800
TTTCCCCCTG GAAGCTCCCT CGTGCGCTCT CCTGTTCCGA     840
CCCTGCCGCT TACCGGATAC CTGTCCGCCT TTCTCCCTTC     880
GGGAAGCGTG GCGCTTTCTC AATGCTCACG CTGTAGGTAT     920
CTCAGTTCGG TGTAGGTCGT TCGCTCCAAG CTGGGCTGTG     960
TGCACGAACC CCCCGTTCAG CCCGACCGCT GCGCCTTATC    1000
CGGTAACTAT CGTCTTGAGT CCAACCCGGT AAGACACGAC    1040
TTATCGCCAC TGGCAGCAGC CACTGGTAAC AGGATTAGCA    1080
GAGCGAGGTA TGTAGGCGGT GCTACAGAGT TCTTGAAGTG    1120
GTGGCCTAAC TACGGCTACA CTAGAAGGAC AGTATTTGGT    1160
ATCTGCGCTC TGCTGAAGCC AGTTACCTTC GGAAAAAGAG    1200
TTGGTAGCTC TTGATCCGGC AAACAAACCA CCGCTGGTAG    1240
CGGTGGTTTT TTTGTTTGCA AGCAGCAGAT TACGCGCAGA    1280
AAAAAAGGAT CTGGGGGATC CGGAGAGCTC ACTCTAGATG    1320
AGAGAGCAGT GAGGGAGAGA CAGAGACTCG AATTTCCGGA    1360
GCTATTTCAG TTTTCTTTTC CGTTTTGTGC AATTTCACTT    1400
ATGATACCGG CCAATGCTTG GTTGCTATTT TGGAAACTCC    1440
CCTTAGGGGA TGCCCCTCAA CTGGCCCTAT AAAGGGCCAG    1480
CCTGAGCTGC AGAGGATTCC TGCAGAGGAT CAAGACAGCA    1520
CGTGGACCTC GCACAGCCTC TCCCACA                  1547
```

(2) INFORMATION FOR SEQ ID NO:28:

(i) SEQUENCE CHARACTERISTICS:

(A) LENGTH: 1807 base pairs
(B) TYPE: nucleic acid
(C) STRANDEDNESS: single
(D) TOPOLOGY: unknown

(ii) MOLECULE TYPE: cDNA

(iii) HYPOTHETICAL: No

(iv) ANTI-SENSE: No

(xi) SEQUENCE DESCRIPTION:SEQ ID NO:28:

```
GGTACCTGCC ACCATGGCGC GGATTCTTTA TCACTGATAA      40
GTTGGTGGAC ATATTATGTT TATCAGTGAT AAAGTGTCAA      80
GCATGACAAA GTTGCAGCCG AATACAGTGA TCCGTGCCGG     120
CCCTGGACTG TTGAACGAGG TCGGCGTAGA CGGTCTGACG     160
ACACGCAAAC TGGCGGAACG GTTGGGGGTG CAGCAGCCGG     200
CGCTTTACTG GCACTTCAGG AACAAGCGGG CGCCTTAAGG     240
GCCATATGGT GAGTGGATGC CTTGACCCCA GGCGGGGATG     280
GGGGAGACCT GTAGTCAGAG CCCCCGGGCA GCACAGGCCA     320
ATGCCCGTCC TTCCCCTGCA GGATGAGTAG TGAGTGCCTC     360
TCCTGGCCCT GGAAGTTGCC ACTCCAGTGC CCACCAGCCT     400
TGTCCTAATA AAATTAAGTT GCATCATTTT GTCTGACTAG     440
GTGTCCTCTA TAATATTATA AGCTTGATAT CGAATTCTTT     480
CGGACTTTTG AAAGTGATGG TGGTGGGGGA AGGATTCGAA     520
CCTTCGAAGT CGATGACGGC AGATTTAGAG TCTGCTCCCT     560
TTGGCCGCTC GGGAACCCCA CCACGGGTAA TGCTTTTACT     600
GGCCTGCTCC CTTATCGGGA AGCGGGGCGC ATCATATCAA     640
ATGACGCGCC GCTGTAAAGT GTTACGTTGA GAAAGAATTC     680
```

```
CTGCAGCCCG CCGCGTTGCT GGCGTTTTTC CATAGGCTCC     720
GCCCCCCTGA CGAGCATCAC AAAAATCGAC GCTCAAGTCA     760
GAGGTGGCGA AACCCGACAG GACTATAAAG ATACCAGGCG     800
TTTCCCCCTG GAAGCTCCCT CGTGCGCTCT CCTGTTCCGA     840
CCCTGCCGCT TACCGGATAC CTGTCCGCCT TTCTCCCTTC     880
GGGAAGCGTG GCGCTTTCTC AATGCTCACG CTGTAGGTAT     920
CTCAGTTCGG TGTAGGTCGT TCGCTCCAAG CTGGGCTGTG     960
TGCACGAACC CCCCGTTCAG CCCGACCGCT GCGCCTTATC    1000
CGGTAACTAT CGTCTTGAGT CCAACCCGGT AAGACACGAC    1040
TTATCGCCAC TGGCAGCAGC CACTGGTAAC AGGATTAGCA    1080
GAGCGAGGTA TGTAGGCGGT GCTACAGAGT TCTTGAAGTG    1120
GTGGCCTAAC TACGGCTACA CTAGAAGGAC AGTATTTGGT    1160
ATCTGCGCTC TGCTGAAGCC AGTTACCTTC GGAAAAAGAG    1200
TTGGTAGCTC TTGATCCGGC AAACAAACCA CCGCTGGTAG    1240
CGGTGGTTTT TTTGTTTGCA AGCAGCAGAT TACGCGCAGA    1280
AAAAAAGGAT CTGGGGGATC CGGAGAGCTC CCAACGCGTT    1320
GGATGCATGG ATGAGGGAAA GGAGGTAAGA TCTGTAATGA    1360
ATAAGCAGGA ACTTTGAAGA CTCAGTGACT CAGTGAGTAA    1400
TAAAGACTCA GTGACTTCTG ATCCTGTCCT AACTGCCACT    1440
CCTTGTTGTC CCAAGAAAGC GGCTTCCTGC TCTCTGAGGA    1480
GGACCCCTTC CCTGGAAGGT AAAACTAAGG ATGTCAGCAG    1520
AGAAATTTTT CCACCATTGG TGCTTGGTCA AAGAGGAAAC    1560
TGATGAGCTC ACTCTAGATG AGAGAGCAGT GAGGGAGAGA    1600
CAGAGACTCG AATTTCCGGA GCTATTTCAG TTTTCTTTTC    1640
CGTTTTGTGC AATTTCACTT ATGATACCGG CCAATGCTTG    1680
GTTGCTATTT TGGAAACTCC CCTTAGGGGA TGCCCCTCAA    1720
CTGGCCCTAT AAAGGGCCAG CCTGAGCTGC AGAGGATTCC    1760
TGCAGAGGAT CAAGACAGCA CGTGGACCTC GCACAGCCTC    1800
TCCCACA                                       1807
```

(2) INFORMATION FOR SEQ ID NO:29:

(i) SEQUENCE CHARACTERISTICS:

(A) LENGTH: 2308 base pairs
(B) TYPE: nucleic acid
(C) STRANDEDNESS: single
(D) TOPOLOGY: unknown

(ii) MOLECULE TYPE: CDNA

(iii) HYPOTHETICAL: No

(iv) ANTI-SENSE: No

(xi) SEQUENCE DESCRIPTION:SEQ ID NO:29:

```
GGTACCTGCC ACCATGGCGA AGGGCGAGGA ACTGTTCACT      40
GGCGTGGTCC CAATTCTCGT GGAACTGGAT GGCGATGTGA      80
ATGGGCACAA ATTTTCTGTC AGCGGAGAGG GTGAAGGTGA     120
TGCCACATAC GGAAAGCTCA CCCTGAAATT CATCTGCACC     160
ACTGGAAAGC TCCCTGTGCC ATGGCCAACA CTGGTCACTA     200
CCTTCACCTA TGGCGTGCAG TGCTTTTCCA GATACCCAGA     240
CCATATGAAG CAGCATGACT TTTTCAAGAG CGCCATGCCC     280
```

```
GAGGGCTATG TGCAGGAGAG AACCATCTTT TTCAAAGATG   320
ACGGGAACTA CAAGACCCGC GCTGAAGTCA AGTTCGAAGG   360
TGACACCCTG GTGAATAGAA TCGAGTTGAA GGGCATTGAC   400
TTTAAGGAAG ATGGAAACAT TCTCGGCCAC AAGCTGGAAT   440
ACAACTATAA CTCCCACAAT GTGTACATCA TGGCCGACAA   480
GCAAAAGAAT GGCATCAAGG TCAACTTCAA GATCAGACAC   520
AACATTGAGG ATGGATCCGT GCAGCTGGCC GACCATTATC   560
AACAGAACAC TCCAATCGGC GACGGCCCTG TGCTCCTCCC   600
AGACAACCAT TACCTGTCCA CCCAGTCTGC CCTGTCTAAA   640
GATCCCAACG AAAAGAGAGA CCACATGGTC CTGCTGGAGT   680
TTGTGACCGC TGCTGGGATC ACACATGGCA TGGACGAGCT   720
GTACAAGTGA GCGCCTTAAG GGCCATATGG TGAGTGGATG   760
CCTTGACCCC AGGCGGGGAT GGGGGAGACC TGTAGTCAGA   800
GCCCCCGGGC AGCACAGGCC AATGCCCGTC CTTCCCCTGC   840
AGGATGAGTA GTGAGTGCCT CTCCTGGCCC TGGAAGTTGC   880
CACTCCAGTG CCCACCAGCC TTGTCCTAAT AAAATTAAGT   920
TGCATCATTT TGTCTGACTA GGTGTCCTCT ATAATATTAT   960
AAGCTTGATA TCGAATTCTT TCGGACTTTT GAAAGTGATG  1000
GTGGTGGGGG AAGGATTCGA ACCTTCGAAG TCGATGACGG  1040
CAGATTTAGA GTCTGCTCCC TTTGGCCGCT CGGGAACCCC  1080
ACCACGGGTA ATGCTTTTAC TGGCCTGCTC CCTTATCGGG  1120
AAGCGGGGCG CATCATATCA AATGACGCGC CGCTGTAAAG  1160
TGTTACGTTG AGAAAGAATT CCTGCAGCCC GCCGCGTTGC  1200
TGGCGTTTTT CCATAGGCTC CGCCCCCCTG ACGAGCATCA  1240
CAAAAATCGA CGCTCAAGTC AGAGGTGGCG AAACCCGACA  1280
GGACTATAAA GATACCAGGC GTTTCCCCCT GGAAGCTCCC  1320
TCGTGCGCTC TCCTGTTCCG ACCCTGCCGC TTACCGGATA  1360
CCTGTCCGCC TTTCTCCCTT CGGGAAGCGT GGCGCTTTCT  1400
CAATGCTCAC GCTGTAGGTA TCTCAGTTCG GTGTAGGTCG  1440
TTCGCTCCAA GCTGGGCTGT GTGCACGAAC CCCCCGTTCA  1480
GCCCGACCGC TGCGCCTTAT CCGGTAACTA TCGTCTTGAG  1520
TCCAACCCGG TAAGACACGA CTTATCGCCA CTGGCAGCAG  1560
CCACTGGTAA CAGGATTAGC AGAGCGAGGT ATGTAGGCGG  1600
TGCTACAGAG TTCTTGAAGT GGTGGCCTAA CTACGGCTAC  1640
ACTAGAAGGA CAGTATTTGG TATCTGCGCT CTGCTGAAGC  1680
CAGTTACCTT CGGAAAAAGA GTTGGTAGCT CTTGATCCGG  1720
CAAACAAACC ACCGCTGGTA GCGGTGGTTT TTTTGTTTGC  1760
AAGCAGCAGA TTACGCGCAG AAAAAAAGGA TCTGGGGGAT  1800
CCGGAGAGCT CCCAACGCGT TGGATGCATG GATGAGGGAA  1840
AGGAGGTAAG ATCTGTAATG AATAAGCAGG AACTTTGAAG  1880
ACTCAGTGAC TCAGTGAGTA ATAAAGACTC AGTGACTTCT  1920
GATCCTGTCC TAACTGCCAC TCCTTGTTGT CCCAAGAAAG  1960
CGGCTTCCTG CTCTCTGAGG AGGACCCCTT CCCTGGAAGG  2000
TAAAACTAAG GATGTCAGCA GAGAAATTTT TCCACCATTG  2040
GTGCTTGGTC AAAGAGGAAA CTGATGAGCT CACTCTAGAT  2080
GAGAGAGCAG TGAGGGAGAG ACAGAGACTC GAATTTCCGG  2120
AGCTATTTCA GTTTTCTTTT CCGTTTTGTG CAATTTCACT  2160
TATGATACCG GCCAATGCTT GGTTGCTATT TTGGAAACTC  2200
CCCTTAGGGG ATGCCCCTCA ACTGGCCCTA TAAAGGGCCA  2240
GCCTGAGCTG CAGAGGATTC CTGCAGAGGA TCAAGACAGC  2280
ACGTGGACCT CGCACAGCCT CTCCCACA               2308
```

## Claims

**1.** A humanized polynucleotide vector comprising:

a human derived promotor or mammalian homolog thereof which is functional in a target tissue or target cells, said promotor operably linked to a sequence acceptance site which directionally accepts cDNA target products from rtPCR cloning via unique sites within an interrupted palindrome recognition sequence for a restriction endonuclease, wherein said vector lacks an antibiotic resistance encoding nucleic acid sequence, and comprises minimal non-human components necessary for production of the vector, wherein said minimal non-human components are an origin of replication which allows replication and growth of the vector in bacteria or yeast and a nucleic acid sequence which allows for selection of recombinant plasmids in bacteria or yeast.

**2.** The humanized polynucleotide vector according to claim 1 wherein the target cells are selected from the group consisting of myocytes and professional antigen presenting cells.

**3.** The humanized polynucleotide vector according to claim 1 or 2 wherein the target cells or target tissue are human.

**4.** The humanized polynucleotide vector according to claims 1 to 3 wherein the human derived promotor is a RANTES promotor or portion thereof.

**5.** The humanized polynucleotide vector according to claim 4 wherein the promotor has approximately 440 base pairs.

**6.** The humanized polynucleotide vector according to claims 4 or 5 wherein the portion corresponds to a region spanning the NCO site through the KpnI site of the genomic RANTES promotor.

**7.** The humanized polynucleotide vector according to claim 6 wherein the origin for replication is colE1 or functional portion thereof.

**8.** The humanized polynucleotide vector according to claim 6 wherein the origin for replication comprises a 635 base pair region of the colE1 origin of replication.

**9.** The humanized polynucleotide vector according to claims 1 to 8 further comprising a human-derived 3' splice sequence and a human-derived poly A sequence, both sequences located downstream of the sequence acceptance site.

**10.** The humanized polynucleotide vector according to claim 9 wherein the human derived 3' splice and poly A sequence are derived from human growth hormone.

**11.** A polynucleotide vector according to claims 1 to 10 wherein a 5' sequence acceptance site reads on the positive strand as GCCACCATGGCC or wherein a 3' sequence acceptance site reads on the positive strand as GCCTTAAGGGC.

**12.** A polynucleotide vector comprising SEQ ID NO:16, SEQ ID NO:27 or SEQ ID NO:28.

**13.** A polynucleotide vector contained within a host cell deposited with the ATCC under the ATCC designation 98400 or ATCC designation 98401.

**14.** A polynucleotide vector according to claims 1 to 13 further comprising cDNA target products, and an optional internal ribosomal entry site, said cDNA target products integrated into said sequence acceptance site, said cDNA target products comprising a nucleotide sequence encoding at least one target antigen or antigenic epitope thereof alone or in combination with a nucleotide sequence encoding a cytokine or chemokine.

**15.** The humanized polynucleotide vector according to claims 1 to 11 wherein the sequence acceptance site comprises the nucleotide sequence as depicted in Figure 2.

**16.** The humanized polynucleotide vector according to claims 1 to 14 wherein the recognition sequence is recognized by BglI restriction endonuclease.

**17.** The humanized polynucleotide vector according to claim 6 wherein the nucleic acid sequence which allows for selection is a suppressor tRNA gene, a synthetic SupF complementation tRNA gene, or functional derivatives thereof.

**18.** The humanized polynucleotide vector according to claim 17, wherein the nucleic acid sequence is selected from the group consisting of SupE, SupP, SupD, SupU, SupF, SupZ, glyT, glyU, SerP, $psu_1^+$, $psu_2^+$-C34, $psu_3^+$AM and $psu_3^+$OC.

**19.** A polynucleotide vector vaccine according to claim 1 further comprising an optional internal ribosomal entry site, and cDNA target products, said cDNA target products integrated into said sequence acceptance site, said cDNA target products comprising a nucleotide sequence encoding at least one target antigen or antigenic epitope thereof.

**20.** A polynucleotide vector vaccine according to claim 19 wherein the target antigen is a product of a tumor associated genetic derangement.

**21.** The polynucleotide vector vaccine of claim 19 wherein the target antigen is a tumor antigen, bacterial antigen, viral antigen, or parasitic antigen.

**22.** The polynucleotide vector vaccine of claim 19 wherein the tumor antigen is p53, RB, ras, int-2, Hst, Tre 17, BRCA-1, BRCA-2, MUC-1, HER-2/neu, truncated EGFRvIII, CEA, MyC, Myb, OB-1, OB-2, BCR/ABL, GIP, GSP, RET, ROS, FIS, SRC, TRC, WT1, DCC, NF1, FAB, MEN-1, ERB-B1 or combinations thereof.

**23.** A polynucleotide vector vaccine according to claim 19, 20, 21 or 22 further comprising an additional cDNA target product comprising a nucleic acid sequence encoding a cytokine or chemokine.

**24.** A polynucleotide vector vaccine according to claim 23 wherein the cytokine is selected from the group consisting of interleukin 2, interleukin 3, interleukin 4, interleukin 7, interleukin 8, interleukin 12, interleukin 15, GM-CSF, tumor necrosis factor, and interferon.

**25.** A polynucleotide vector vaccine according to claim 23 wherein the chemokine is selected from the group consisting of RANTES, MCP, MIP-1$^{\alpha}$, MIP-1$^{\beta}$, defensins and combinations thereof.

**26.** A pharmaceutical composition comprising at least one polynucleotide vector according to claims 1 to 14 and a pharmaceutically acceptable carrier.

**27.** A pharmaceutical composition comprising the polynucleotide vector vaccine according to claims 19-20 or 21-25 and a pharmaceutically acceptable carrier.

**28.** A kit comprising the polynucleotide vector according to claims 1 to 14.

**29.** A kit comprising the polynucleotide vector vaccine according to claims 19-20 or 21-25.

**30.** A kit according to claim 29, further comprising an expression enhancing agent.

**31.** The kit according to claim 30 wherein the expression enhancing agent is a mycotoxic agent.

**32.** The kit according to claim 31 wherein the mycotoxic agent is bupivacaine-HCl and dextrose.

**33.** A host cell comprising:

the polynucleotide vector of claims 19-20 or 21-25, wherein the host cell is capable of expressing the target antigen or antigenic epitope.

**34.** The host cell according to claim 33 wherein the host cell is a myocyte or professional antigen presenting cell.

**35.** An in vitro or ex vivo method for expressing at least one target antigen or antigenic epitope thereof in cells comprising:

introducing a humanized polynucleotide vector according to claim 1 or 15 into said cells, under conditions for expression of the target antigen or antigenic epitope thereof.

**36.** The method of claim 35 wherein the cells are selected from the group consisting of myocytes and professional antigen presenting cells.

**37.** A method of making a humanized polynucleotide vector according claim 1 or 4 comprising:

operably linking a human derived promotor or mammalian homolog thereof which is functional in a target tissue or target cells to a sequence acceptance site, said site directionally accepts cDNA target products from rtPCR cloning via unique sites within an interrupted palindrome recognition sequence for a restriction endonuclease.

**38.** The method of claim 35 wherein the target antigen is a tumor antigen, bacterial antigen, viral antigen, or parasitic antigen.

**39.** The method of claim 38 wherein the tumor antigen is p53, RB, ras, int-2, Hst, Tre 17, BRCA-1, BRCA-2, MUC-1, HER-2/neu, truncated EGFRvIII, CEA, MyC, Myb, OB-1, OB-2, BCR/ABL, GIP, GSP, RET, ROS, FIS, SRC, TRC, WT1, DCC, NF1, FAB, MEN-1, ERB-B1, or combinations thereof.

**40.** The use of a sequence acceptance site having a 5' acceptance site and a 3' acceptance site comprising nucleic acid sequences for accepting cDNA target products from rtPCR cloning wherein the sequence acceptance site directionally accepts target sequence specific products from rtPCR cloning via unique sites within an interrupted palindrome recognition sequence for a restriction enconuclease, wherein the 5'acceptance site comprises an initiation codon and Kozak consensus sequence and the 3' acceptance site comprises a termination codon.

**41.** The use according to claim 40 wherein the restriction endonuclease is BglI.

**42.** The use according to claims 40 or 41 wherein a 5' acceptance site reads on the positive strand as GCCACCATGGCC.

**43.** The use according to claim 42 wherein a 3' acceptance site reads on the positive strand as GCCTTAAGGGC.

**44.** The use according to claim 40 wherein the site comprises the nucleotide sequence as depicted in Figure 2.

**45.** A use of a polynucleotide vector vaccine according to claims 19-20 or 21-25 in the manufacture of a medicament for use in a method of stimulating a specific immune response to at least one target antigen or antigenic epitope thereof in a mammal.

**46.** A use according to claim 45, wherein the medicament can be administered to the muscle or skin.

**47.** A use according to claim 45 or 46 wherein the medicament further comprises an expression enhancing agent.

**48.** The use according to claim 47, wherein the expression enhancing agent is a mycotoxic agent.

**49.** The use according to claim 48, wherein the mycotocix agent is bupivacaine-HCl or dextrose.

**50.** The use according to claims 45 to 48 or 49 wherein the target antigen is a tumor antigen, bacterial antigen, viral antigen or parasitic antigen.

**51.** The use according to claim 50, wherein the tumor antigen is selected for the group consisting of p53, RB, ras, int-2, Hst, Tre 17, BRCA-1, BRCA-2, MUC-1, HER-2/neu, truncated EGFRvIII, CEA, MyC, Myb, OB-1, OB-2, BCR/ABL, GIP, GSP, RET, ROS, FIS, SRC, TRC, WT1, DCC, NF1, FAB, MEN-1, ERB-B1 and combinations thereof.

**52.** The use according to claim 51, wherein the method generates antigen specific cytotoxic lymphocytes to the tumor antigen or antigenic epitopes thereof.

**Patentansprüche**

**1.** Ein humanisierter Polynukleotidvektor, umfassend:

einen human-abgeleiteten Promotor oder ein Säuger-Homolog davon, der in einem Zielgewebe oder Zielzellen funktionell ist, dieser Promotor ist funktionell mit einer Sequenz-Akzeptanz-Stelle verbunden, die gerichtet cDNA Zielprodukte aus rtPCR Klonierungen über einzigartige Stellen in einer unterbrochenen Palindrom-Erkennungssequenz für eine Restriktionsendonuklease akzeptiert, wobei diesem Vektor eine Antibiotikaresistenz kodierende Nukleinsäuresequenz fehlt und er minimale nicht-humane Komponenten, die zur Herstellung des Vektors notwendig sind umfasst, wobei diese minimalen nicht-humanen Komponenten ein Replikationsursprung, der Replikation und Wachstum des Vektors in Bakterien oder Hefe ermöglicht und eine Nukleinsäuresequenz, die die Selektion von rekombinanten Plasmiden in Bakterien oder Hefe ermöglicht, sind.

2. Humanisierter Polynukleotidvektor gemäß Anspruch 1, wobei die Zielzellen ausgewählt sind, aus der Gruppe bestehend aus, Monozyten und professionellen Antigenpräsentierenden Zellen.

3. Humanisierter Polynukleotidvektor gemäß Anspruch 1 oder 2, wobei die Zielzellen oder das Zielgewebe human sind (ist).

4. Humanisierter Polynukleotidvektor gemäß den Ansprüchen 1 bis 3, wobei der human-abgeleitete Promotor ein RANTES Promotor oder ein Teil davon ist

5. Humanisierter Polynukleotidvektor gemäß Anspruch 4, wobei der Promotor etwa 440 Basenpaare besitzt

6. Humanisierter Polynukleotidvektor gemäß den Ansprüchen 4 oder 5, wobei der Teil einer Region entspricht, die die NCO Stelle bis zur KpnI Stelle des genomischen RANTES Promotors überbrückt

7. Humanisierter Polynukleotidvektor gemäß Anspruch 6, wobei der Replikationsursprung colE1 oder ein funktioneller Teil davon ist.

8. Humanisierter Polynukleotidvektor gemäß Anspruch 6, wobei der Replikationsursprung eine 635 Basenpaarregion des colE1 Replikationsursprungs umfasst.

9. Humanisierter Polynukleotidvektor gemäß den Ansprüchen 1 bis 8, weiterhin umfassend, eine human-abgeleitete 3'-Spleiß-Sequenz und eine human-abgeleitete Poly-A-Sequenz, wobei beide Sequenzen stromabwärts der Sequenz-Akzeptanz-Stelle lokalisiert sind.

10. Humanisierter Polynukleotidvektor gemäß Anspruch 9, wobei die human-abgeleiteten 3'-Spleiß- und Poly-A-Sequenzen von humanem Wachstumshormon abgeleitet sind.

11. Polynukleotidvektor gemäß den Ansprüchen 1 bis 10, wobei eine 5'-Sequenz-Akzeptanz-Stelle sich auf dem positiven Strang als GCCACCATGGCC liest oder wobei sich eine 3'-Sequenz-Akzeptanz-Stelle auf dem positiven Strang als GCCTTAAGGGC liest.

12. Polynukleotidvektor umfassend SEQ ID NO:16, SEQ ID NO:27 oder SEQ ID NO:28.

13. Polynukleotidvektor enthalten in einer Wirtszelle, hinterlegt bei der ATCC unter der ATCC Kennzeichnung 98400 oder ATCC Kennzeichnung 98401.

14. Polynukleotidvektor gemäß den Ansprüche 1 bis 13, weiterhin umfassend cDNA Zielprodukte und eine optionale interne ribosomale Eintrittsstelle, wobei diese cDNA Zielprodukte in die Sequenz-Akzeptanz-Stelle integriert sind, diese cDNA Zielprodukte umfassen eine Nukleotidsequenz, die wenigstens ein Ziel-Antigen oder ein antigenes Epitop davon kodiert, allein oder in Kombination mit einer Nukleotidsequenz, die ein Cytokin oder Chemokin kodiert

15. Humanisierter Polynukleotidvektor gemäß den Ansprüchen 1 bis 11, wobei die Sequenz-Akzeptanz-Stelle die Nukleotidsequenz wie in Figur 2 dargestellt umfasst.

16. Humanisierter Polynukleotidvektor gemäß den Ansprüchen 1 bis 14, wobei die Erkennungssequenz von der BglI Restriktionsendonuklease erkannt wird.

17. Humanisierter Polynukleotidvektor gemäß Anspruch 6, wobei die Nukleinsäuresequenz, die Selektion ermöglicht, ein Suppressor tRNA-Gen, ein synthetisches SupF Komplementations tRNA-Gen oder funktionelle Derivate davon ist (sind).

18. Humanisier Polynukleotidvektor gemäß Anspruch 17, wobei der Nukleinsäure Sequenz ausgewählt ist, aus der Gruppe bestehend aus, SupE, SupP, SupD, SupU, SupF, SupZ, glyT, glyU, SerP, $psu_1^+$, $psu_2^+$-C34, $psu_3^+$AM und $psu_3^+$OC.

**19.** Polynukleotidvektor Vakzin gemäß Anspruch 1, weiterhin umfassend eine optionale ribosomale Eintrittsstelle und ein cDNA Zielprodukt, wobei die cDNA Zielprodukte in die Sequenz-Akzeptanz-Stelle integriert sind, wobei die cDNA Zielprodukte eine Nukleotidsequenz umfassen, die wenigstens ein Zielantigen oder antigenes Epitop davon kodiert.

**20.** Polynukleotidvektor Vakzin gemäß Anspruch 19, wobei das Zielantigen ein Produkt einer Tumor-assoziierten genetischen Unordnung ist.

**21.** Polynukleotidvektor Vakzin gemäß Anspruch 19, wobei das Zielantigen ein Tumorantigen, bakterielles Antigen, virales Antigen oder parasitisches Antigen ist.

**22.** Polynukleotidvektor Vakzin gemäß Anspruch 19, wobei das Tumorantigen p53, RB, ras, int-2, Hst, Tre 17, BRCA-1, BRCA-2, MUC-1, HER-2/neu, trunkiertes EGFRvIII, CEA, MyC, Myb, OB-1, OB-2, BCR/ABL, GIP, GSP, RET, ROS, FIS, SRC, TRC, WT1, DCC, NF1, FAB, MEN-1, ERB-B1 ist oder Kombinationen davon.

**23.** Polynukleotidvektor Vakzin gemäß den Ansprüchen 19, 20, 21 oder 22, weiterhin umfassend ein zusätzliches cDNA Zielprodukt, umfassend eine Nukleinsäuresequenz, die ein Cytokin oder Chemokin kodiert.

**24.** Polynukleotidvektor Vakzin gemäß Anspruch 23, wobei das Cytokin ausgewählt ist, aus der Gruppe bestehend aus, Interleukin 2, Interleukin 3, Interleukin 4, Interleukin 7, Interleukin 8, Interleukin 12, Interleukin 15, GM-CSF, Tumor-Nekrosefaktor und Interferon.

**25.** Polynukleotidvektor Vakzin gemäß Anspruch 23, wobei das Chemokin ausgewählt ist aus der Gruppe, bestehend aus RANTES, MCP, MIP-$1^{\alpha}$, MIP-$1^{\beta}$, Defensinen und Kombinationen davon.

**26.** Pharmazeutische Zusammensetzung, umfassend wenigstens einen Polynukleotidvektor gemäß den Ansprüchen 1 bis 14 und einen pharmazeutisch verträglicher Träger.

**27.** Pharmazeutische Zusammensetzung, umfassend das Polynukleotidvektor Vakzin gemäß den Ansprüchen 19-20 oder 21-25 und einen pharmazeutisch verträglichen Träger.

**28.** Kit umfassend den Polynukleotidvektor gemäß den Ansprüchen 1 bis 14.

**29.** Kit umfassend das Polynukleotidvektor Vakzin gemäß den Ansprüchen 19-20 oder 21-25.

**30.** Kit gemäß Anspruch 29, weiterhin umfassend ein Expressions-verstärkendes Mittel.

**31.** Kit gemäß Anspruch 30, wobei das Expressions-verstärkende Mittel ein mykotoxisches Mittel ist.

**32.** Kit gemäß Anspruch 31, wobei das mykotoxische Mittel Bupivacain-HCL und Dextrose ist

**33.** Wirtszelle umfassend:

den Polynukleotidvektor der Ansprüche 19-20 oder 21-25, wobei die Wirtszelle geeignet ist, das Zielantigen oder das antigene Epitop zu exprimieren.

**34.** Wirtszelle gemäß Anspruch 33, wobei die Wirtszelle eine Myozyte oder eine professionelle Antigen-präsentierende Zelle ist

**35.** In vitro oder ex vivo Verfahren zur Expression wenigstens eines Zielantigens oder antigenen Epitop davon in Zellen, umfassend:

Einbringen eines humanisierten Polynukleotidvektors gemäß Anspruch 1 oder 15 in die Zellen, unter Bedingungen zur Expression des Zielantigens oder antigenen Epitops davon.

**36.** Verfahren nach Anspruch 35, wobei die Zellen ausgewählt sind aus der Gruppe bestehend aus, Myozyten und professionellen antigen-präsentierenden Zellen.

**37.** Verfahren zur Herstellung eines humanisierten Polynukleotidvektors gemäß Anspruch 1 oder 4, umfassend:

funktionelles Verbinden eines human-abgeleiteten Promotors oder Säuger-Homolog davon, der funktionell in einem Zielgewebe oder in Zielzellen zu einer Sequenz-Akzeptanz-Stelle ist, diese Stelle akzeptiert gerichtet cDNA Zielprodukte aus rtPCR Klonierung über einzigartige Stellen in einer unterbrochenen Palindrom-Erkennungssequenz für eine Restriktionsendonuklease.

**38.** Verfahren nach Anspruch 35, wobei das Zielantigen ein Tumorantigen, bakterielles Antigen, virales Antigen oder parasitisches Antigen ist.

**39.** Verfahren nach Anspruch 38, wobei das Tumorantigen p53, RB, ras, int-2, Hst, Tre 17, BRCA-1, BRCA-2, MUC-1, HER-2/neu, trunkiertes EGFRvIII, CEA, MyC, Myb, OB-1, OB-2, BCR/ABL, GIP, GSP, RET, ROS, FIS, SRC, TRC, WT1, DCC, NF1, FAB, MEN-1, ERB-B1 ist oder Kombinationen davon.

**40.** Verwendung einer Sequenz-Akzeptanz-Stelle mit einer 5' Akzeptanz-Stelle und einer 3'-Akzeptanz-Stelle, umfassend Nukleinsäuresequenzen zur Akzeptanz von cDNA Zielprodukten aus rtPCR Klonierung, wobei die Sequenz-Akzeptanz-Stelle gerichtet Zielsequenz-spezifische Produkte aus rtPCR Klonierung über einzigartige Stellen in einer unterbrochenen Palindrom-Erkennungssequenz für eine Restriktionsendonuklease akzeptiert, wobei die 5'-Akzeptanzstelle ein Initiationskodon und Kozak Konsensussequenz umfasst und die 3'-Akzeptanzstelle ein Terminationskodon umfasst.

**41.** Verwendung gemäß Anspruch 40, wobei die Restriktionsendonuklease BglI ist

**42.** Verwendung gemäß den Ansprüchen 40 oder 41, wobei eine 5'-Akzeptanzstelle sich auf dem positiven Strang als GCCACCATGGCC ließt

**43.** Verwendung gemäß Anspruch 42, wobei eine 3'-Akzeptanzstelle sich auf dem positiven Strang als GCCTTAAGGGC ließt

**44.** Verwendung gemäß Anspruch 40, wobei die Stelle eine Nukleotidsequenz wie in Figur 2 dargestellt umfasst.

**45.** Verwendung eines Polynukleotidvektor Vakzins gemäß den Ansprüchen 19-20 oder 21-25 zur Herstellung eines Medikaments zur Verwendung in einem Verfahren zur Stimulierung einer spezifischen Immunantwort auf wenigstens ein Zielantigen oder antigenes Epitop davon in einem Säuger.

**46.** Verwendung gemäß Anspruch 45, wobei das Medikament an den Muskel oder die Haut verabreicht werden kann.

**47.** Verwendung gemäß den Ansprüchen 45 oder 46, wobei das Medikament weiterhin ein Expressions-verstärkendes Mittel umfasst

**48.** Verwendung gemäß Anspruch 47, wobei das Expressions-verstärkende Mittel ein mykotoxisches Mittel ist

**49.** Verwendung gemäß Anspruch 48, wobei das mykotoxische Mittel Bupivacain-HCL und Dextrose ist

**50.** Verwendung gemäß den Ansprüchen 45 bis 48 oder 49, wobei das Zielantigen ein Tumorantigen, bakterielles Antigen, virales Antigen oder parasitisches Antigen ist

**51.** Verwendung gemäß Anspruch 50, wobei das Tumorantigen ausgewählt ist, aus der Gruppe bestehend aus, p53, RB, ras, int-2, Hst, Tre 17, BRCA-1, BRCA-2, MUC-1, HER-2/neu, trunkiertes EGFRvIII, CEA, MyC, Myb, OB-1, OB-2, BCR/ABL, GIP, GSP, RET, ROS, FIS, SRC, TRC, WT1, DCC, NF1, FAB, MEN-1, ERB-B1 und Kombinationen davon.

**52.** Verwendung gemäß Anspruch 51, wobei das Verfahren antigen-spezifische zytotoxische Lymphozyten gegen das Tumorantigen oder antigene Epitope davon erzeugt.

**Revendications**

1. Vecteur polynucléotidique humanisé comprenant :

   un promoteur dérivé de l'humain ou un homologue mammifère de celui-ci, fonctionnel dans un tissu-cible ou des cellules-cibles, ledit promoteur étant lié de façon fonctionnelle à un site accepteur de séquence qui accepte de façon dirigée des produits-cibles d'ADNc à partir d'un clonage de PCR par transcriptase inverse via des sites uniques dans une séquence interrompue de reconnaissance palindromique pour une endonucléase de restriction, ledit vecteur étant dépourvu d'une séquence en acide nucléique codant une résistance à un antibiotique, et
   comprenant des composants minimaux non humains nécessaires pour la production du vecteur, dans lequel lesdits composants minimaux non humains sont une origine de réplication qui permet la réplication et la croissance du vecteur dans des bactéries ou des levures et une séquence d'acides nucléiques qui permet la sélection des plasmides recombinants dans les bactéries et les levures.

2. Vecteur polynucléotidique humanisé selon la revendication 1, dans lequel les cellules-cibles sont choisies dans le groupe formé par les myocytes et les cellules professionnelles présentatrices d'antigène.

3. Vecteur polynucléotidique humanisé selon la revendication 1 ou 2, dans lequel les cellules-cibles ou le tissu-cible sont d'origine humaine.

4. Vecteur polynucléotidique humanisé selon l'une quelconque des revendications 1 à 3, dans lequel le promoteur d'origine humaine est un promoteur RANTES ou une portion de celui-ci.

5. Vecteur polynucléotidique humanisé selon la revendication 4, dans lequel le promoteur a approximativement 440 paires de bases.

6. Vecteur polynucléotidique humanisé selon la revendication 4 ou 5, dans lequel la portion correspond à une région s'étendant du site NCO jusqu'au site Kpn1 du promoteur génomique RANTES.

7. Vecteur polynucléotidique humanisé selon la revendication 6, dans lequel l'origine de réplication est colE1 ou une portion fonctionnelle de celui-ci.

8. Vecteur polynucléotidique humanisé selon la revendication 6, dans lequel l'origine de réplication comprend une région de 635 paires de bases de l'origine de réplication colE1.

9. Vecteur polynucléotidique humanisé selon l'une quelconque des revendications 1 à 8, comprenant de plus une séquence d'épissage en 3' d'origine humaine et une séquence poly A d'origine humaine, les deux séquences étant situées en aval de la séquence du site accepteur.

10. Vecteur polynucléotidique humanisé selon la revendication 9, dans lequel les séquences humaines d'épissage en 3' et poly A sont dérivées de l'hormone de croissance humaine.

11. Vecteur polynucléotidique selon l'une quelconque des revendications 1 à 10, dans lequel une séquence en 5' de site accepteur se lit sur le brin positif comme GCCACCATGGCC ou dans lequel une séquence en 3' de site accepteur se lit sur le brin positif comme GCCTTAAGGGC.

12. Vecteur polynucléotidique comprenant les séquences SEQ ID NO : 16, SEQ ID NO : 27 ou SEQ ID NO : 28.

13. Vecteur polynucléotidique contenu dans une cellule-hôte déposée à l'ATCC sous le numéro ATCC 98400 ou le numéro ATCC 98 401.

14. Vecteur polynucléotidique selon l'une quelconque des revendications 1 à 13, comprenant de plus des produits-cibles d'ADNc et optionnellement un site d'entrée interne ribosomique, lesdits produits-cibles d'ADNc étant intégrés dans ladite séquence du site accepteur, lesdits produits-cibles d'ADNc comprenant une séquence nucléotidique codant au moins un antigène-cible ou un épitope antigénique de celui-ci seul ou en combinaison avec une séquence nucléotidique codant une cytokine ou une chimiokine.

**15.** Vecteur polynucléotidique humanisé selon l'une quelconque des revendications 1 à 11, dans lequel la séquence du site accepteur comprend la séquence nucléotidique telle que montrée dans la figure 2.

**16.** Vecteur polynucléotidique humanisé selon l'une quelconque des revendications 1 à 14, dans lequel la séquence de reconnaissance est reconnue par une endonucléase de restriction BglI.

**17.** Vecteur polynucléotidique humanisé selon la revendication 6, dans lequel la séquence en acides nucléiques qui permet la sélection est un gène suppresseur d'ARNt, un gène synthétique SupF de complémentation de l'ARNt ou des dérivés fonctionnels de ceux-ci.

**18.** Vecteur polynucléotidique humanisé selon la revendication 17, dans lequel la séquence en acides nucléiques est choisie dans le groupe constitué de SupE, SupP, SupD, SupU, SupF, SupZ, glyT, glyU, SerP, $psu_1^+$, $psu_2^+$-C34, $psu_3^+$AM et $psu_3^+$OC.

**19.** Vecteur vaccin polynucléotidique selon la revendication 1 comprenant de plus un site d'entrée optionnel interne ribosomique, et des produits-cibles ADNc, lesdits produits-cibles d'ADNc étant intégrés dans ladite séquence du site accepteur, lesdits produits-cibles d'ADNc comprenant une séquence nucléotidique codant au moins un antigène-cible ou un épitope antigénique de celui-ci.

**20.** Vecteur vaccin polynucléotidique selon la revendication 19, dans lequel l'antigène-cible est un produit d'une tumeur associé à un dérangement génétique.

**21.** Vecteur vaccin polynucléotidique selon la revendication 19, dans lequel l'antigène-cible est un antigène tumoral, un antigène bactérien, un antigène viral, ou un antigène de parasite.

**22.** Vecteur vaccin polynucléotidique selon la revendication 19, dans lequel l'antigène tumoral est p53, RB, ras, int-2, Hst, Tre 17, BRCA-1, BRCA-2, MUC-1, HER-2/neu, EGFRvIII tronqué, CEA, MyC, Myb, OB-1, OB-2, BCR/ABL, GIP, GSP, RET, ROS, FIS, SRC, TRC, WTI, DCC, NF1, FAB, MEN-1, ERB-B1 ou des combinaisons de ceux-ci.

**23.** Vecteur vaccin polynucléotidique selon la revendication 19, 20, 21 ou 22 comprenant de plus un produit-cible d'ADNc comprenant une séquence d'acides nucléiques codant une cytokine ou une chimiokine.

**24.** Vecteur vaccin polynucléotidique selon la revendication 23, dans lequel la cytokine est choisie dans le groupe constitué de l'interleukine 2, l'interleukine 3, l'interleukine 4, l'interleukine 7, l'interleukine 8, l'interleukine 12, l'interleukine 15, le GM-CSF, le facteur de nécrose tumorale et l'interféron.

**25.** Vecteur vaccin polynucléotidique selon la revendication 23, dans lequel la chimiokine est choisie dans le groupe constitué de RANTES, MCP, MIP-$1^{\alpha}$, MIP-$1^{\beta}$, les défensines et des combinaisons de celles-ci.

**26.** Composition pharmaceutique comprenant au moins un vecteur polynucléotidique selon l'une quelconque des revendications 1 à 14, et un véhicule pharmaceutiquement acceptable.

**27.** Composition pharmaceutique comprenant le vecteur vaccin polynucléotidique selon l'une quelconque des revendications 19, 20 ou 21 à 25, et un véhicule pharmaceutiquement acceptable.

**28.** Kit comprenant le vecteur polynucléotidique selon l'une quelconque des revendications 1 à 14.

**29.** Kit comprenant le vecteur vaccin polynucléotidique selon l'une quelconque des revendications 19, 20 ou 21 à 25.

**30.** Kit selon la revendication 29, comprenant de plus un agent augmentant l'expression.

**31.** Kit selon la revendication 30, dans lequel l'agent augmentant l'expression est un agent mycotoxique.

**32.** Kit selon la revendication 31, dans lequel l'agent mycotoxique est la bupivacaïne-HCl et le dextrose.

**33.** Cellule-hôte comprenant :

le vecteur polynucléotidique selon l'une quelconque des revendications 19, 20 ou 21 à 25 dans lequel la cellule-hôte est capable d'exprimer l'antigène-cible ou l'épitope antigénique.

**34.** Cellule-hôte selon la revendication 33, dans laquelle la cellule-hôte est un myocyte ou une cellule professionnelle présentatrice d'antigène.

**35.** Procédé *in vitro* ou *ex vivo* afin d'exprimer au moins un antigène-cible ou un épitope antigénique de celui-ci dans des cellules comprenant :

le fait d'introduire un vecteur polynucléotidique humanisé selon l'une quelconque de revendications 1 à 15 dans lesdites cellules, dans des conditions pour l'expression de l'antigène-cible ou de l'épitope antigénique de celui-ci.

**36.** Procédé selon la revendication 35, dans lequel les cellules sont choisies dans le groupe constitué des myocytes et des cellules professionnelles présentatrices d'antigène.

**37.** Procédé de fabrication d'un vecteur polynucléotidique humanisé selon les revendications 1 ou 4 comprenant :

le fait de lier de façon fonctionnelle un promoteur d'origine humaine ou un homologue mammifère de celui-ci fonctionnel dans un tissu-cible ou des cellules-cibles à une séquence de site accepteur, ledit site acceptant de façon directionnelle les produits-cibles d'ADNc à partir d'un clonage en PCR par transcriptase inverse via des sites uniques à l'intérieur d'une séquence interrompue de reconnaissance palindromique pour une endonucléase de restriction.

**38.** Procédé selon la revendication 35, dans lequel l'antigène-cible est un antigène de tumeur, un antigène bactérien, un antigène viral ou un antigène de parasite.

**39.** Procédé selon la revendication 38, dans lequel l'antigène de tumeur est p53, RB, ras, int-2, Hst, Tre 17, BRCA-1, BRCA-2, MUC-1, HER-2/neu, EGFRvIII tronqué, CEA, MyC, Myb, OB-1, OB-2, BCR/ABL, GIP, GSP, RET, ROS, FIS, SRC, TRC, WTI, DCC, NF1, FAB, MEN-1, ERB-B1, ou des combinaisons de ceux-ci.

**40.** Utilisation d'une séquence de site accepteur ayant un site accepteur en 5' et un site accepteur en 3' comprenant des séquences d'acide nucléique pour accepter des produits-cibles d'ADNc à partir d'un clonage en PCR par transcriptase inverse dans lequel la séquence du site accepteur accepte de façon directionnelle des produits spécifiques cibles de séquence à partir d'un clonage en PCR par transcriptase inverse via des sites uniques à l'intérieur d'une séquence interrompue de reconnaissance palindromique pour une endonucléase de restriction, dans lequel le site accepteur 5' comprend un codon de démarrage et une séquence consensus de Kozak et le site accepteur en 3' comprend un codon de terminaison.

**41.** Utilisation selon la revendication 40, dans laquelle l'endonucléase de restriction est BglI.

**42.** Utilisation selon les revendications 40 ou 41 dans lesquelles le site accepteur 5' se lit sur le brin positif comme GCCACCATGGCC.

**43.** Utilisation selon la revendication 42 dans laquelle le site accepteur en 3' se lit sur le brin positif comme GCCTTAAGGGC.

**44.** Utilisation selon la revendication 40, dans laquelle le site comprend la séquence nucléotidique telle que décrite dans la figure 2.

**45.** Utilisation d'un vecteur vaccin polynucléotidique selon l'une quelconque des revendications 19, 20 ou 21 à 25 dans la fabrication d'un médicament pour l'utilisation dans un procédé de stimulation d'une réponse immunitaire spécifique à au moins un antigène-cible ou un épitope antigénique de celui-ci chez un mammifère.

**46.** Utilisation selon la revendication 45, dans laquelle le médicament peut être administré au niveau du muscle ou de la peau.

**47.** Utilisation selon la revendication 45 ou 46, dans laquelle le médicament comprend en outre un agent augmentant l'expression.

**48.** Utilisation selon la revendication 47, dans laquelle l'agent augmentant l'expression est un agent mycotoxique.

**49.** Utilisation selon la revendication 48, dans laquelle l'agent mycotoxique est le bupivacaïne-HCl ou la dextrose.

**50.** Utilisation selon les revendications 45 à 48 ou 49 dans laquelle l'antigène-cible est un antigène de tumeur, un antigène bactérien, un antigène viral ou un antigène de parasite.

**51.** Utilisation selon la revendication 50, dans laquelle l'antigène de tumeur est choisi dans le groupe constitué de p53, RB, ras, int-2, Hst, Tre 17, BRCA-1, BRCA-2, MUC-1, HER-2/neu, EGFRvIII tronqué, CEA, MyC, Myb, OB-1, OB-2, BCR/ABL, GIP, GSP, RET, ROS, FIS, SRC, TRC, WTI, DCC, NF1, FAB, RIEN-1, ERB-B1, et des combinaisons de ceux-ci.

**52.** Utilisation selon la revendication 51, dans laquelle le procédé génère des lymphocytes cytotoxiques spécifiques de l'antigène tumoral ou des épitopes antigéniques de celui-ci.

FIG. 1

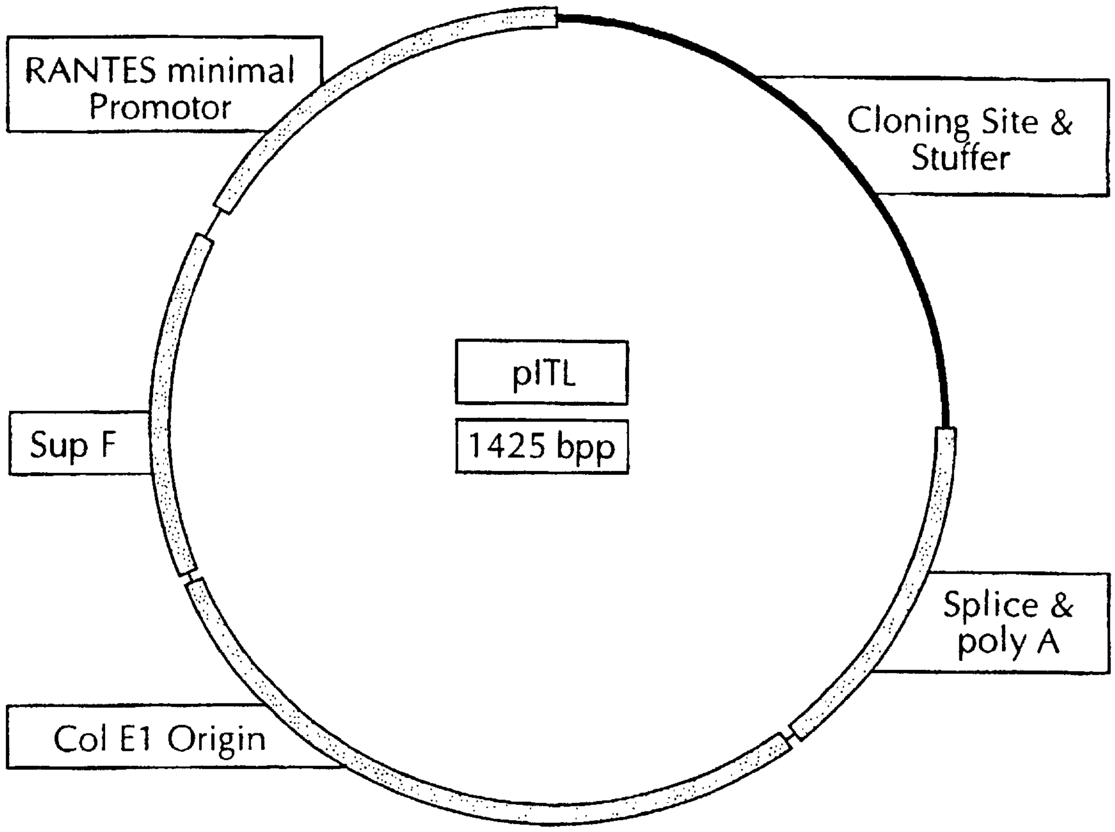
RANTES minimal Promotor
Cloning Site & Stuffer
pITL
1425 bpp
Sup F
Splice & poly A
Col E1 Origin

FIG. 2

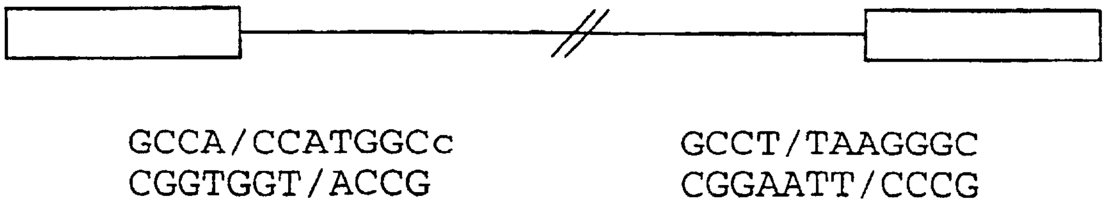
GCCA/CCATGGCc
CGGTGGT/ACCG
GCCT/TAAGGGC
CGGAATT/CCCG

# FIG. 3A

```
                                                          60
MRPSGTAGAALLALLAALCPASRALEEKKVCQGTSNKLTQLGTFEDHFLSLQRMFNNCEVVLGNLEITYVQRNYD
M---ELAALCRWGLLLALLP-PGA-AST-VCTGTDMKLRLPASPETHLDMLRHLYQGCQVVQGNLELTYLPTNAS
                                                          57

                                                 125                      140
LSFLKTIQEVAGYVLIALNTVERIPLENLQIIRGNMYYENSYALAVLSNYD--AN---KT-----GLKELPMRNL
LSFLQDIQEVQGYVLIAHNQVRQVPLQRLRIVRGTQLFEDNYALAVLDNGDPLNNTTPVTGASPGGLRELQLRSL
                                                 122                      147

                *                175             *  *   *     *       *
QEILHGAVRFSNNPALCNVESIQWRDIVSSDFLSNMSMDFQNHLGSCQKCDPSCPNGS-CWGAGEENCQKLTKII
TEILKGGVLIQRNPQLCYQDTILWKDIFHKNNQLALTLIDTNRSRACHPCSPMCK-GSRCWGESSEDCQSLTRTV
                          175

*   *   *      **   *                                                     289
CAQQCSGRCRGKSPSDCCHNQCAAGCTGPRESDCLVCRKFRDEATCKDTCPPLMLYNPTTYQMDVNPEGKYSFGA
CAGGCA-RCKGPLPTDCCHEQCAAGCTGPKHSDCLACLHFNHSGICELHCPALVTYNTDTFESMPNPEGRYTFGA
                                                                          295

       *   *               *  *   *   *                    350  *
YVVTDHGSCVRACGADSYEME-EDGVRKCKKCEGPCRKVCNGIGIGEFKDSLSINATNIKHFKNCTSISGDLHIL
YLSTDVGSCTLVCPLHNQEVTAEDGTQRCEKCSKPCARVCYGLGMEHLREVRAVTSANIQEFAGCKKIFGSLAFL
                                                              352

                                                                          438
PVAFRGDSFTHTPPLDPQELDILKTVKEITGFLLIQAWPENRTDLHAFENLEIIRGRTKQPGQFSLAVVSLNITS
PESFDGDPASNTAPLQPEQLQVFETLEEITGYLYISAWPDSLPDLSVFQNLQVIRGRILHNGAYSLTLQGLGISW
                                                                          445
```

# FIG. 3B

```
                             475
LGLRSLKEISDGDVIISGNKNLCYANTINWKKLFGTSGQKTKIISNRGENSCKATGQVCHALCSPEGCWGPEPRD
LGLRSLRELGSGLALIHHNTHLCFVHTVPWDQLFRNPHQALLHTANRPEDECVGEGLACHQLCARGHCWGPGPTQ
                                                      500

                                                                         583
CVSCRNVSRGRECVDKCKLLEGEPREFVENSECIQCHPECLPQAMNITCTGRGPDNCIQCAHYIDGPHCVKTCPA
CVNCSQFLRGQECVEECRVLQGLPREYVNARHCLPCHPECQPQNGSVTCFGPEADQCVACAHYKDPPFCVARCPS
                                                 531                     595

                                                  631
GVMGENNTLV-WKYADAGHVCHLCHPNCTYGCTGPGLEGCPTNGPKIPSIATGMVGALLLLLVVAL---GIGLF
GVKPDLSYMPIWKFPDEEGACQPCPINCTHSCVDLDDKGCPAEQRASPLTSIISAVVGILLVVVLGVVFGI-LI
                                                  644

                           684                                            727
MRRRHIVRKRTLRRLLQERELVEPLTPSGEAPNQALLRILKETEFKKIKVLGSGAFGTVYKGLWIPEGEKVKIP
KRRQQKIRKYTMRRLLQETELVEPLTPSGAMPNQAQMRILKETELRKVKVLGSGAFGTVYKGIWIPDGENVKIP
                           700                                            742

                                                  775
VAIKELREATSPKANKEILDEAYVMASVDNPHVCRLLGICLTSTVQLITQLMPFGCLLDYVREHKDNIGSQYLLN
VAIKVLRENTSPKANKEILDEAYVMAGVGSPYVSRLLGICLTSTVQLVTQLMPYGCLLDHVTENRGRLGSQDLLN
                                              790

                                                  850                       877
WCVQIAKGMNYLEDRRLVHRDLAARNVLVKTPQHVKITDFGLAKLLGAEEKEYHAEGGKVPIKWMALESILHRIY
WCMQIAKGMSYLEDVRLVHRDLAARNVLVKSPNHVKITDFGLARLLDIDETEYHADGGKVPIKWMALESILRRRF
                          850                                             892
```

# FIG. 3C

```
                                                 929
THQSDVWSYGVTVWELMTFGSKPYDGIPASEISSILEKGERLPQPPICTIDVYMIMVKCWMIDADSRPKFRELII
THQSDVWSYGVTVWELMTFGAKPYDGIPAREIPDLLEKGERLPQPPICTIDVYMIMVKCWMIDSECRPRFRELVS
                                                 940

                                     990                     1010
EFSKMARDPQRYLVIQGDERMHLPSPTDSNFYRALMDEE-DMDDVVDADEYLIPQQGFF----------------
EFSRMARDPQRFVVIQN-EDLGPASPLDSTFYRSL-LEDDDMGDLVDAEEYLVPQQGFFCPDPAPGAGGMVHHRH
                           1000                                         1040

------------------SSPSTSRTPLLSSLSATSNNSTVACIDRN--GLQSCPIKEDSFLQRYSSDPTGAL-T
RSSSTRSGGGDLTLGLEPSEEEAPRSPLAPSEGAGSDVFDGDLGMGAAKGLQSLPTHDPSPLQRYSEDPTVPLPS

    1070                                                                1137
E-D--SIDDTFLPVPEYINQ-SVPKRPAGSVQNPVYHNQPLNPAPSRDPHYQDPHSTAVGNPEYLNTVQPTCVNS
ETDGYVAPLTCSPQPEYVNQPDVRPQPPSPREGPLPAARPAGATLERPKTLSPGKNGVVKDVFAFGGAVENPEYL
 1120                                                                   1290

       1150
TFDSTFLPVPEYINQ-SVPKRPAGSVQNPVYHNQPLNPAPSRDPHYQDPHSTAVGNPEYLNTVQPTCVNSTFDSP
TPQGTCSPQPEYVNQPDVRPQPPSPREGPLPAARPAGATLERPKTLSPGKNGVVKDVFAFGGAVENPEYLTPQGG
    1300

                                                            1210
AHWAQKGSHQ-ISL--DNPDYQ-QDFFPKEAKPNGIFKGST--AENAEYLR-VAPQSSEFIGA
AAP-QPHPPPAFSPAFDNLYYWDQDPPERGAPPS-TFKG-TPTAENPEYLGLDVPV
                                                    1255
```

# FIG. 4A

```
                                                           57
  MELAALCRWGLLLALLPPGAASTQVCTGTDMKLRLPASPETHLDMLRHLYQGCQVVQGNLELTYLPTNASLS
MIIMELAAWCRWGFLLALLPPGIAGTQVCTGTDMKLRLPASPETHLDMLRHLYQGCQVVQGNLELTYVPANASLS
                                                           60

                                             117
FLQDIQEVQGYVLIAHNQVRQVPLQRLRIVRGTQLFEDNYALAVLDNGDPLNNTTPVT-GASPGGLRELQLRSLT
FLQDIQEVQGYMLIAHNQVKRVPLQRLRIVRGTQLFEDKYALAVLDNRDPQDNVAASTPGRTPEGLRELQLRSLT
                                             120

                         176
EILKGGVLIQRNPQLCYQDTILWKDIFHKNNQLALTLIDTNRSRACHPCSPMCKGSRCWGESSEDCQSLTRTVCA
EILKGGVLIRGNPQLCYQDMVLWKDVFRKNNQLAPVDIDTNRSRACPPCAPACKDNHCWGESPEDCQILTGTICT
                         180

         236                                                            296
GGCARCKGPLPTDCCHEQCAAGCTGPKHSDCLACLHFNHSGICELHCPALVTYNTDTFESMPNPEGRYTFGASCV
SGCARCKGRLPTDCCHEQCAAGCTGPKHSDCLACLHFNHSGICELHCPALVTYNTDTFESMHNPEGRYTFGASCV
         240                                                            300

                                                           356
TACPYNYLSTDVGSCTLVCPLHNQEVTAEDGTQRCEKCSKPCARVCYGLGMEHLREVRAVTSANIQEFAGCKKIF
TTCPYNYLSTEVGSCTLVCPPNNQEVTAEDGTQRCEKCSKPCARVCYGLGMEHLRGARAITSDNVQEFDGCKKIF
                                                           360

                                             416
GSLAFLPESFDGDPASNTAPLQPEQLQVFETLEEITGYLYISAWPDSLPDLSVFQNLQVIRGRILHNGAYSLTLQ
GSLAFLPESFDGDPSSGIAPLRPEQLQVFETLEEITGYLYISAWPDSLRDLSVFQNLRIIRGRILHDGAYSLTLQ
                                             420
```

# FIG. 4B

```
476
GLGISWLGLRSLRELGSGLALIHHNTHLCFVHTVPWDQLFRNPHQALLHTANRPEDE-CVGEGLACHQLCARGHC
GLGIHSLGLRSLRELGSGLALIHRNAHLCFVHTVPWDQLFRNPHQALLHSGNRPEEDLCVSSGLVCNSLCAHGHC
480

535                                                         595
WGPGPTQCVNCSQFLRGQECVEECRVLQGLPREYVNARHCLPCHPECQPQNGSVTCFGPEADQCVACAHYKDPPF
WGPGPTQCVNCSHFLRGQECVEECRVWKGLPREYVSDKRCLPCHPECQPQNSSETCFGSEADQCAACAHYKDSSS
540                                                         600

                                                         655
CVARCPSGVKPDLSYMPIWKFPDEEGACQPCPINCTHSCVDLDDKGCPAEQRASPLTSIISAVVGILLVVVLGVV
CVARCPSGVKPDLSYMPIWKYPDEEGICQPCPINCTHSCVDLDERGCPAEQRASPVTFIIATVEGVLLFLILVVV
                                                         660

                              710
FGILI KRR-QQKIRKYTMRRLLQETELVEPLTPSGAMPNQAQMRILKETELRKVKVLGSGAFGTVYKGIWIPD
VGILI KRRRQ-KIRKYTMRRLLQETELVEPLTPSGAMPNQAQMRILKETELRKVKVLGSGAFGTVYKGIWIPD
                              715

                   770
GENVKIPVAIKVLRENTSPKANKEILDEAYVMAGVGSPYVSRLLGICLTSTVQLVTQLMPYGCLLDHVRENRGRL
GENVKIPVAIKVLRENTSPKANKEILDEAYVMAGVGSPYVSRLLGICLTSTVQLVTQIMPYGCLLDHVREHRGRL
                   775

830                                                                      870
GSQDLLNWCMQIAKGMSYLEDVRLVHRDLAARNVLVKSPNHVKITDFGLARLLDIDETEYHADGGKVPIKWMALE
GSQDLLNWCVQIAKGMSYLEDVRLVHRDLAARNVLVKSPNHVKITDFGLARLLDIDETEYHADGGKVPIKWMALE
835                                                                      896
```

# FIG. 4C

```
                                                                950
SILRRRFTHQSDVWSYGVTVWELMTFGAKPYDGIPAREIPDLLEKGERLPQPPICTIDVYMIMVKCWMIDSECRP
SILRRRFTHQSDVWSYGVTVWEIMTFGAKPYDGIPAREIPDLLEKGERLPQPPICTIDVYMIMVKCWMIDSECRP
                                                                955

                                         1010
RFRELVSEFSRMARDPQRFVVIQNEDLGPASPLDSTFYRSLLEDDDMGDLVDAEEYLVPQQGFFCPDPAPGAGGM
RFRELVSEFSRMARDPQRFVVIQNEDLGPSSPMDSTFYRSLLEDDDMGDLVDAEEYLVPQQGFFSPDPTPGTGST
                                         1015

                        1070
VHHRHRSSSTRSGGGDLTLGLEPSEEEAPRSPLAPSEGAGSDVFDGDLGMGAAKGLQSLPTHDPSPLQRYSEDPT
AHRRHRSSSTRSGGGELTLGLEPSEEGPPRSPLAPSEGAGSDVFDGDLAMGVTKGLQSLSPHDLSPLQRYSEDPT
                        1075

        1130                                                             1193
VPLPSETDGYVAPLTCSPQPEYVNQPDVRPQPPSPREGPLPAARPAGATLERPKTLSPGKNGVVKDVFAFGGAVE
LPLPPETDGYVAPLACSPQPEYVNQSEVQPQPPLTPEGPLPPVRPAGATLERPKTLSPGKNGVVKDVFAFGGAVE
        1135                                                             1198

                                                               1255
NPEYLTPQGGAAPQPHPPPAFSPAFDNLYYWDQDPPERGAPPSTFKGTPTAENPEYLGLDVPV
NPEYLVPREGTASPPHPSPAFSPAFDNLYYWDQNSSEQGPPPSNFEGTPTAENPEYLGLDVPV
                                                               1263
```

# FIG. 5

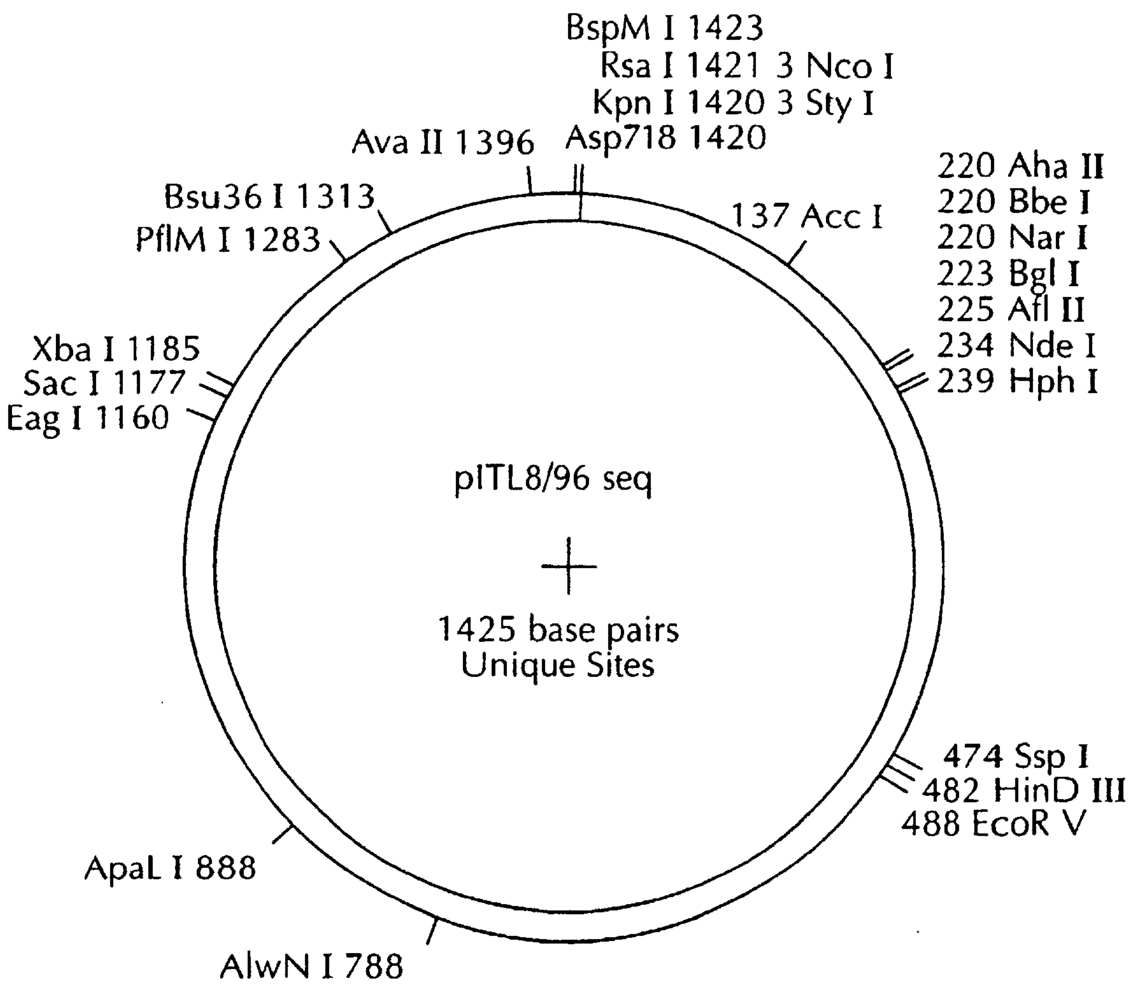
BspM I 1423
Rsa I 1421 3 Nco I
Kpn I 1420 3 Sty I
Asp718 1420
Ava II 1396
Bsu36 I 1313
PflM I 1283
137 Acc I
220 Aha II
220 Bbe I
220 Nar I
223 Bgl I
225 Afl II
234 Nde I
239 Hph I
Xba I 1185
Sac I 1177
Eag I 1160
pITL8/96 seq
1425 base pairs
Unique Sites
474 Ssp I
482 HinD III
488 EcoR V
ApaL I 888
AlwN I 788